# EUROPEAN PATENT APPLICATION

(11) **EP 4 813 975 A1**
(43) Date of publication of application: **30.09.2026**
(21) Application number: 26164130.2
(22) Date of filing: 11.03.2026
(51) Int. Cl.: C07D 307/91, C07D 333/76, C09K 11/06, H10K 50/00, H10K 85/60

(54) **LIGHT EMITTING ELEMENT, AMINE COMPOUND FOR THE LIGHT EMITTING ELEMENT, AND ELECTRONIC DEVICE INCLUDING THE LIGHT EMITTING ELEMENT**

(30) Priority: 24.03.2025 JP 2025048134
(71) Applicant: Samsung Display Co., Ltd., Yongin-si, Gyeonggi-do (KR)
(72) Inventor: JIN, XiuLan, Yokohama-shi, 220-0011 (JP); TAKADA, Ichinori, Yokohama-shi, 220-0011 (JP); SAKAMOTO, Naoya, Yokohama-shi, 220-0011 (JP)
(74) Representative: Marks & Clerk LLP

(57) **Abstract**

Provided is a light emitting element including a first electrode, a second electrode on the first electrode, an emission layer between the first electrode and the second electrode, and a hole transport region between the first electrode and the emission layer, wherein the hole transport region includes an amine compound represented by Formula 1 below. Accordingly, the light emitting element may exhibit long lifespan characteristics.

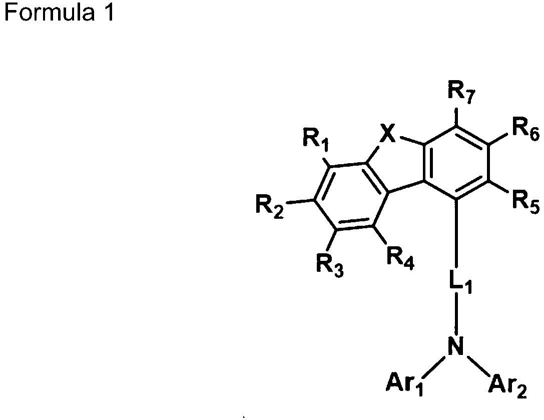

## Description

### BACKGROUND

Embodiments of the present disclosure herein relate to a light emitting element, an amine compound used therein, and an electronic device including the light emitting element.

Organic electroluminescence display devices, which include organic electroluminescence elements, are used as display devices included in electronic devices. The organic electroluminescence display devices are display devices including so-called self-luminescent light emitting elements in which holes and electrons are injected into an emission layer from a first electrode and a second electrode, and thus a light emitting material in the emission layer emits light to accomplish display.

For application of light emitting elements to display devices, there is a demand for greater light efficiency and service life, and development of materials, for light emitting elements, capable of stably attaining such characteristics is being researched.

### SUMMARY

Embodiments of the present disclosure provide a light emitting element operable at low voltage and having improved luminous efficiency and lifespan.

Embodiments of the present disclosure also provide an amine compound that enables a light emitting element to operate at low voltage and to have improved luminous efficiency and lifespan characteristics.

Embodiments of the present disclosure also provide an electronic device having excellent display quality by including a light emitting element device operable at low voltage and having improved luminous efficiency and lifespan.

An embodiment of the present disclosure provides a light emitting element including a first electrode, a second electrode on the first electrode, an emission layer between the first electrode and the second electrode, and a hole transport region between the first electrode and the emission layer and including an amine compound represented by Formula 1 below.

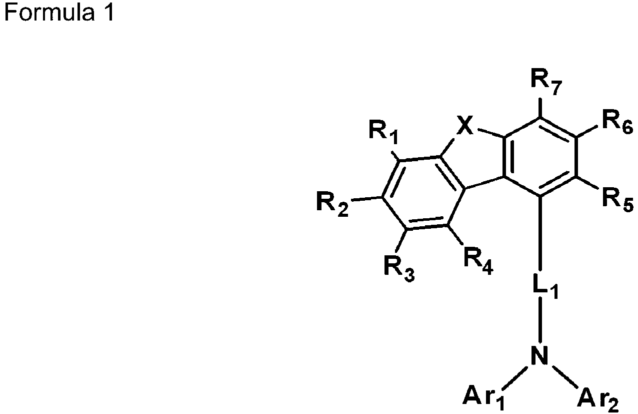

In Formula 1 above,
L₁ is a direct linkage, a substituted or unsubstituted arylene group having 6 to 30 ring-forming carbon atoms, or a substituted or unsubstituted heteroarylene group having 2 to 30 ring-forming carbon atoms.
X is O or S.
R₁ to R₇ are each independently hydrogen atom, a deuterium atom, a halogen atom, a substituted or unsubstituted alkyl group having 1 to 10 carbon atoms, a substituted or unsubstituted aryl group having 6 to 30 ring-forming carbon atoms, or a substituted or unsubstituted heteroaryl group having 5 to 30 ring-forming carbon atoms.
Ar₁ and Ar₂ are different from each other.

Each of Ar₁ and Ar₂ does not include a 4-(8-phenylnaphthylen-1-yl)phenyl group, a 4-(2-phenylnaphthylen-1-yl)phenyl group, and a 1,1'-binaphthyl group.

Ar₁ and Ar₂ are each independently represented by Formula 2 below.

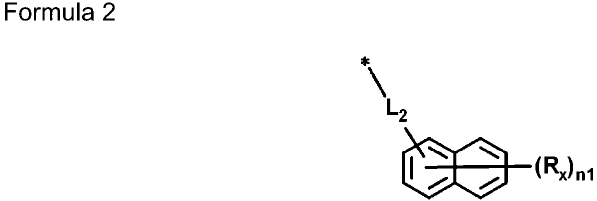

In Formula 2 above, L₂ is a substituted or unsubstituted arylene group having 6 to 30 ring-forming carbon atoms, or a substituted or unsubstituted heteroarylene group having 5 to 30 ring-forming carbon atoms.

Rₓ is a hydrogen atom, a deuterium atom, a halogen atom, a substituted or unsubstituted alkyl group having 1 to 10 carbon atoms, a substituted or unsubstituted aryl group having 6 to 30 ring-forming carbon atoms, or a substituted or unsubstituted heteroaryl group having 5 to 30 ring-forming carbon atoms wherein a bonding position of the heteroaryl group is not a heteroatom.

n1 is an integer of 0 to 7.

If (e.g., when) Rₓ is a fused ring in which rings having three or more rings are fused, a case in which the fused ring Rₓ is bonded to a carbon adjacent to a carbon bonded to L₂ is excluded.

At least one selected from R₁ to R₇, and Rₓ of Ar₁ is a substituted or unsubstituted aryl group having 6 to 30 ring-forming carbon atoms, or a substituted or unsubstituted heteroaryl group having 5 to 30 ring-forming carbon atoms wherein a heteroatom is not a bonding position.

At least one selected from Rₓ's of Ar₂ is a substituted or unsubstituted aryl group having 6 to 30 ring-forming carbon atoms, or a substituted or unsubstituted heteroaryl group having 5 to 30 ring-forming carbon atoms wherein a heteroatom is not a bonding position.

In an embodiment, the hole transport region may include at least one selected from a hole injection layer, a hole transport layer, an electron blocking layer, and an auxiliary emission layer, and at least one selected from the hole injection layer, the hole transport layer, the electron blocking layer, and the auxiliary emission layer may include the amine compound.

In an embodiment, the hole transport region may include a hole injection layer on the first electrode, and a hole transport layer on the hole injection layer, and the hole transport layer may include the amine compound.

In an embodiment, the amine compound (the compound represented by Formula 1) may be represented by Formula 1-1 below.

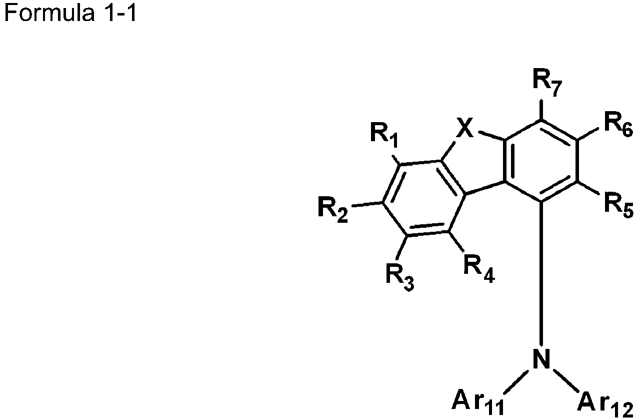

In Formula 1-1 above, R₁ to R₇, and X are the same as defined in Formula 1 above.

Ar₁₁ and Ar₁₂ are each represented by any one selected from Ar-a to Ar-k below, and Ar₁₁ and Ar₁₂ are different from each other.

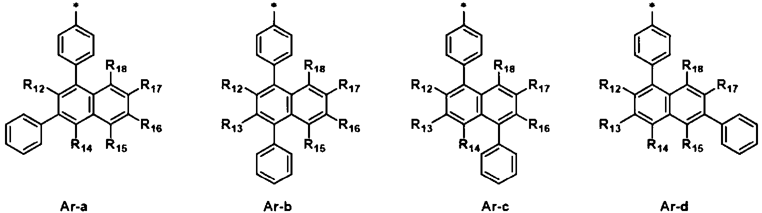

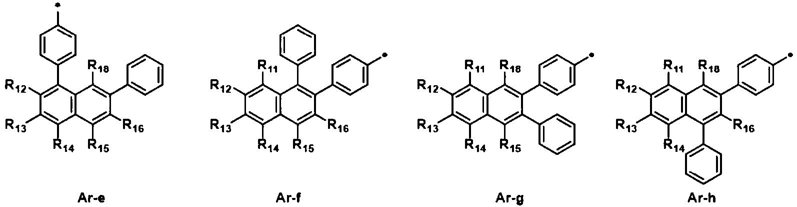

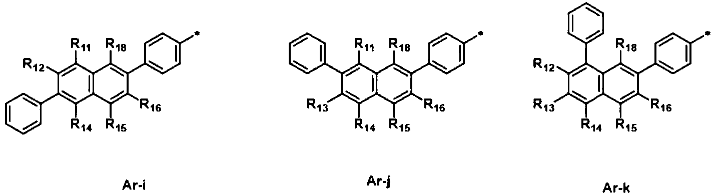

In Ar-a to Ar-k above, R₁₁ to R₁₈ are each independently a hydrogen atom, a deuterium atom, a halogen atom, or a substituted or unsubstituted alkyl group having 1 to 10 carbon atoms.

In an embodiment, the amine compound (the compound represented by Formula 1) may be represented by any one selected from Formulas 1-a to 1-c below.

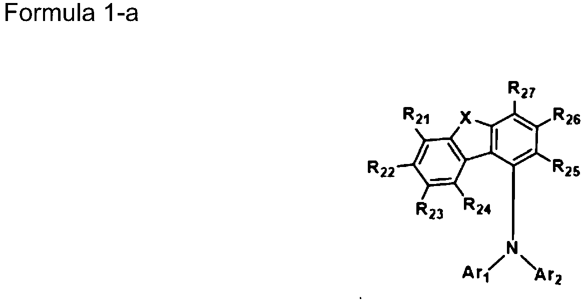

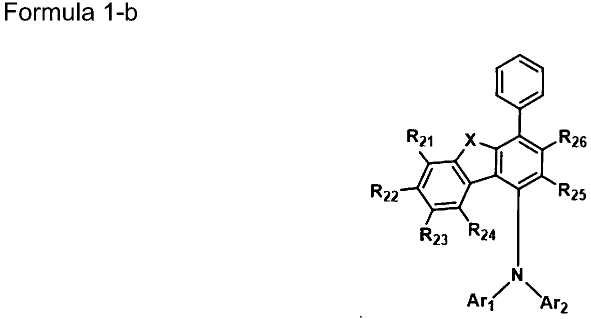

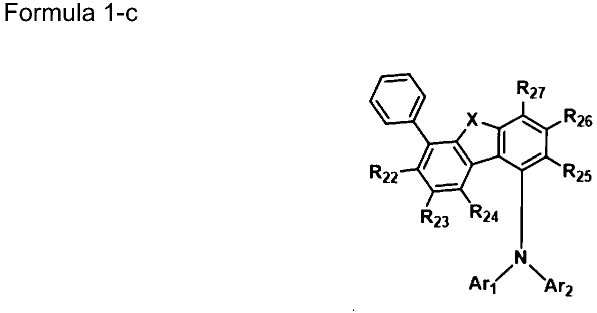

In Formulas 1-a to 1-c above, R₂₁ to R₂₇ are each independently a hydrogen atom, a deuterium atom, a halogen atom, or a substituted or unsubstituted alkyl group having 1 to 10 carbon atoms, and X, Ar₁, and Ar₂ are the same as defined in Formula 1 above.

In an embodiment, at least one hydrogen atom of the amine compound may be substituted with a deuterium atom.

In an embodiment, the amine compound may be a monoamine compound that does not include an amino group as a substituent.

In an embodiment, the emission layer may include a compound represented by Formula E-1 below.

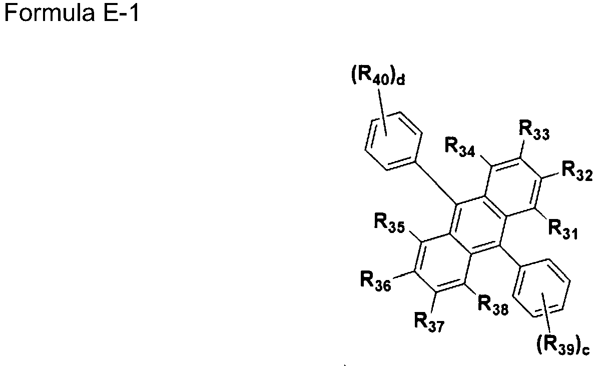

In Formula E-1,
c and d are each independently an integer of 0 to 5.
R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring.

In an embodiment, the amine compound may be represented by any one selected from compounds from Compound Group 1, which will be further described herein.

In an embodiment of the present disclosure, provided is an amine compound represented by Formula 1 above.

In an embodiment of the present disclosure, an electronic device includes a display module including a plurality of light emitting elements,

wherein at least one selected from the plurality of light emitting elements includes a first electrode, a second electrode on the first electrode, an emission layer between the first electrode and the second electrode, and a hole transport region between the first electrode and the emission layer and including an amine compound represented by Formula 1 above.

At least some of the above and other features of the invention are set out in the claims.

### BRIEF DESCRIPTION OF THE DRAWINGS

The accompanying drawings are included to provide a further understanding of the subject matter of the present disclosure, and are incorporated in and constitute a part of this specification. The drawings illustrate embodiments of the present disclosure and, together with the description, serve to explain principles of the present disclosure. In the drawings:
FIG. 1 is a block diagram of an electronic device according to an embodiment;
FIG. 2 shows schematic views of electronic devices according to an embodiment;
FIG. 3 is a plan view of a display module according to an embodiment;
FIG. 4 is a cross-sectional view showing a portion corresponding to line I-I' of FIG. 3;
FIG. 5 is a cross-sectional view schematically showing a light emitting element according to an embodiment;
FIG. 6 is a cross-sectional view schematically showing a light emitting element according to an embodiment;
FIG. 7 is a cross-sectional view schematically showing a light emitting element according to an embodiment;
FIG. 8 is a cross-sectional view schematically showing a light emitting element according to an embodiment;
FIG. 9 is a cross-sectional view schematically showing a light emitting element according to an embodiment;
FIG. 10 is a cross-sectional view showing a display module according to an embodiment;
FIG. 11 is a cross-sectional view showing a display module according to an embodiment;
FIG. 12 is a cross-sectional view showing a display module according to an embodiment;
FIG. 13 is a cross-sectional view showing a display module according to an embodiment;
FIG. 14 is a perspective view of an electronic device according to an embodiment;
FIG. 15 is a perspective view of an electronic device according to an embodiment; and
FIG. 16 is a view showing an inside of a vehicle in which an electronic device according to an embodiment is provided.

### DETAILED DESCRIPTION

In this specification, it will be understood that if (e.g., when) an element (or a region, a layer, a portion, or the like) is referred to as being "on", "connected to" or "coupled to" another element, it may be directly on, connected to, or coupled to the other element, or other elements may be therebetween.

Like reference numerals or symbols refer to like elements throughout. In the drawings, the thickness, ratio, and size of the elements may be exaggerated for effectively describing the technical contents. The term "and/or" includes any and all combinations of one or more of the associated listed elements.

It will be understood that, although the terms "first", "second", etc. may be used herein to describe various elements, the elements are not to be limited by these terms. These terms are only used to distinguish one element, component, region, layer or section from another element, component, region, layer or section. For instance, a first element, component, region, layer or section discussed below could be termed a second element, component, region, layer or section without departing from the scope of the present disclosure. Similarly, a second element, component, region, layer or section could be termed a first element, component, region, layer or section. The singular expressions "a", "an" and "the" are intended to include the plural forms as well, unless the context clearly indicates otherwise.

In embodiments, the terms "below", "under", "on the lower side", "above", "over", "on the upper side", or the like may be used to describe the relationships between the elements illustrated in the drawings. These terms are relative concepts and are described on the basis of the directions indicated in the drawings.

It will be further understood that the terms "comprises, includes, has" and/or "comprising, including, having", if (e.g., when) used in this specification, specify the presence of stated features, numbers, steps, operations, elements, components or combinations thereof, but do not preclude the possibility of the presence or addition of one or more other features, numbers, steps, operations, elements, components, and/or combinations thereof.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

In the present description, the term "substituted or unsubstituted" may indicate that one is substituted or unsubstituted with at least one substituent selected from the group consisting of a deuterium atom, a halogen atom, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. In embodiments, each of the substituents presented as an example above may be substituted or unsubstituted. For example, a biphenyl group may be interpreted as an aryl group or as a phenyl group substituted with a phenyl group.

Herein, the term "bonded to an adjacent group to form a ring" may indicate that one is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocyclic or polycyclic. In embodiments, the rings formed by being bonded to each other may be linked to another ring to form a spiro structure.

Herein, the term "adjacent group" may indicate a substituent substituted for an atom which is directly linked to an atom substituted with a corresponding substituent, another substituent substituted for an atom which is substituted with a corresponding substituent, or a substituent sterically closest to a corresponding substituent. For example, two methyl groups in 1,2-dimethylbenzene may be interpreted as mutually "adjacent groups" and two ethyl groups in 1,1-diethylcyclopentane may be interpreted as mutually "adjacent groups". In embodiments, two methyl groups in 4,5-dimethylphenanthrene may be interpreted as mutually "adjacent groups".

Herein, examples of a halogen atom may include a fluorine atom, a chlorine atom, a bromine atom, or an iodine atom.

Herein, an alkyl group may be linear or branched. The number of carbon atoms in the alkyl group is 1 to 60, 1 to 30, 1 to 20, 1 to 15, 1 to 10, or 1 to 6. Examples of the alkyl group may include a methyl group, an ethyl group, an n-propyl group, an isopropyl group, an n-butyl group, a s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-icosyl group, a 2-ethylicosyl group, a 2-butylicosyl group, a 2-hexylicosyl group, a 2-octylicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, and the like, but are not limited thereto.

As used herein, the alkyl group may include a cycloalkyl group (cyclic alkyl group). The number of carbon atoms in the cycloalkyl group is 3 to 60, 3 to 30, 3 to 20, or 3 to 10. Examples of the cycloalkyl group include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, and the like, but are not limited thereto.

In the present description, an alkenyl group refers to a hydrocarbon group including at least one carbon double bond at a main chain (e.g., in the middle) or a terminal end of an alkyl group having 2 or more carbon atoms. The alkenyl group may be linear or branched. The number of carbon atoms is not particularly limited, but is 2 to 60, 2 to 30, 2 to 20, or 2 to 10. Examples of the alkenyl group include a vinyl group, a 1-butenyl group, a 1-pentenyl group, a 1,3-butadienyl aryl group, a styrenyl group, a styryl vinyl group, and the like, but are not limited thereto.

In the present description, an alkynyl group refers to a hydrocarbon group including at least one carbon triple bond at a main chain (e.g., in the middle) or a terminal end of an alkyl group having 2 or more carbon atoms. The alkynyl group may be linear or branched. The number of carbon atoms is not particularly limited, but is 2 to 30, 2 to 20, or 2 to 10. Examples of the alkynyl group may include an ethynyl group, a propynyl group, and the like, but are not limited thereto.

Herein, a hydrocarbon ring group indicates any suitable functional group or substituent derived from an aliphatic hydrocarbon ring. The hydrocarbon ring group may be a saturated hydrocarbon ring group having 5 to 20 ring-forming carbon atoms.

Herein, an aryl group indicates any suitable functional group or substituent derived from an aromatic hydrocarbon ring. The aryl group may be a monocyclic aryl group or a polycyclic aryl group. The number of ring-forming carbon atoms in the aryl group may be 6 to 60, 6 to 30, 6 to 20, or 6 to 15. Examples of the aryl group may include a phenyl group, a naphthyl group, a fluorenyl group, an anthracenyl group, a phenanthrenyl group, a biphenylyl group, a terphenylyl group, a quaterphenylyl group, a quinquephenylyl group, a sexiphenylyl group, a triphenylenyl group, a pyrenyl group, a benzofluoranthenyl group, a chrysenyl group, and the like, but are not limited thereto.

Herein, a fluorenyl group may be substituted, and two substituents may be bonded to each other to form a spiro structure. An example that the fluorenyl group is substituted is as follows. However, an embodiment is not limited thereto.

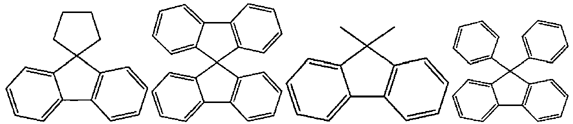

In the present description, a heterocyclic group refers to any suitable functional group or substituent derived from a ring containing at least one selected from B, O, N, P, S, Si, and Se as a hetero atom. The heterocyclic group includes an aliphatic heterocyclic group and an aromatic heterocyclic group. The aromatic heterocyclic group may be a heteroaryl group. The aliphatic heterocycle and the aromatic heterocycle may be monocyclic or polycyclic.

As used herein, the heterocyclic group may contain at least one selected from B, O, N, P, S, Si, and S as a hetero atom. If (e.g., when) the heterocyclic group contains two or more hetero atoms, the two or more hetero atoms may be the same as or different from each other. The heterocyclic group may be a monocyclic heterocyclic group or a polycyclic heterocyclic group, and include a heteroaryl group. The number of ring-forming carbon atoms in the heterocyclic group may be 2 to 60, 2 to 30, 2 to 20, or 2 to 10.

As used herein, the aliphatic heterocyclic group may contain at least one selected from B, O, N, P, S, Si, and Se as a hetero atom. The number of ring-forming carbon atoms in the aliphatic heterocyclic group may be 2 to 30, 2 to 20, or 2 to 10. Examples of the aliphatic heterocyclic group include an oxirane group, a thiirane group, a pyrrolidine group, a piperidine group, a tetrahydrofuran group, a tetrahydrothiophene group, a thiane group, a tetrahydropyran group, a 1,4-dioxane group, and the like, but are not limited to thereto.

As used herein, the heteroaryl group may contain at least one selected from B, O, N, P, S, Si, and S as a hetero atom. If (e.g., when) the heteroaryl group contains two or more hetero atoms, the two or more hetero atoms may be the same as or different from each other. The heteroaryl group may be a monocyclic heteroaryl group or a polycyclic heteroaryl group. The number of ring-forming carbon atoms in the heteroaryl group may be 2 to 60, 2 to 30, 2 to 20, or 2 to 10. Examples of the heteroaryl group include a thienyl group, a furyl group, a pyrrolyl group, an imidazolyl group, a pyridyl group, a bipyridinyl group, a pyrimidinyl group, a triazinyl group, a triazolyl group, an acridinyl group, a pyridazinyl group, a pyrazinyl group, a quinolyl group, a quinazolinyl group, a quinoxalinyl group, a phenoxazinyl group, a phthalazinyl group, a pyridopyrimidinyl group, a pyridopyrazinyl group, a pyrazinopyrazinyl group, an isoquinolinyl group, an indolyl group, a carbazolyl group, an N-arylcarbazolyl group, an N-heteroarylcarbazolyl group, an N-alkylcarbazolyl group, a benzoxazolyl group, a benzoimidazolyl group, a benzothiazolyl group, a benzocarbazolyl group, a benzothiophenyl group, a dibenzothiophenyl group, a thienothiophenyl group, a benzofuranyl group, a phenanthrolinyl group, a thiazolyl group, an isoxazolyl group, an oxazolyl group, an oxadiazolyl group, a thiadiazolyl group, a phenothiazinyl group, a dibenzosilolyl group, or a dibenzofuranyl group, but are not limited thereto.

Herein, the above description of the aryl group may also apply to an arylene group, except that the arylene group is a divalent group. The above description of the heteroaryl group may also apply to a heteroarylene group, except that the heteroarylene group is a divalent group.

Herein, a silyl group includes an alkyl silyl group and an aryl silyl group. Examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

Herein, the number of carbon atoms in a carbonyl group is not particularly limited, but may be 1 to 40, 1 to 30, or 1 to 20. For example, the carbonyl group may have the following structure, but is not limited thereto.

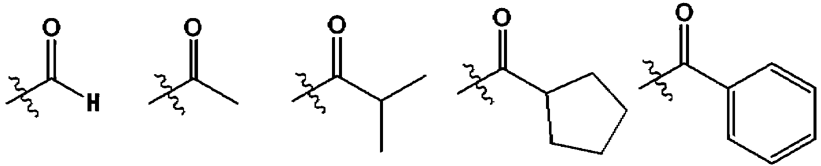

Herein, the number of carbon atoms in a sulfinyl group and a sulfonyl group is not particularly limited, but may be 1 to 30. The sulfinyl group may include an alkyl sulfinyl group and an aryl sulfinyl group. The sulfonyl group may include an alkyl sulfonyl group and an aryl sulfonyl group.

Herein, a thio group may include an alkyl thio group and an aryl thio group. The thio group may indicate the one that a sulfur atom is bonded to the alkyl group or the aryl group as defined above. Examples of the thio group include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, and the like, but are not limited to thereto.

Herein, an oxy group may indicate the one that an oxygen atom is bonded to the alkyl group or the aryl group as defined above. The oxy group may include an alkoxy group and an aryl oxy group. The alkoxy group may be linear, branched, or cyclic. The number of carbon atoms in the alkoxy group is not particularly limited, but may be, for example, 1 to 20, or 1 to 10. Examples of the oxy group include methoxy, ethoxy, n-propoxy, isopropoxy, butoxy, pentyloxy, hexyloxy, octyloxy, nonyloxy, decyloxy, benzyloxy, and the like, but are not limited thereto.

Herein, a boron group may indicate the one that a boron atom is bonded to the alkyl group or the aryl group as defined above. The boron group includes an alkyl boron group and an aryl boron group. Examples of the boron group include a dimethyl boron group, a diethyl boron group, a t-butylmethyl boron group, a diphenyl boron group, a phenyl boron group, and the like, but are not limited thereto.

Herein, the number of carbon atoms in an amine group is not particularly limited, but may be 1 to 30. The amine group may include an alkyl amine group and an aryl amine group. Examples of the amine group include a methylamine group, a dimethylamine group, a phenylamine group, a diphenylamine group, a naphthylamine group, a 9-methyl-anthracenylamine group, and the like, but are not limited thereto.

Herein, the above-described examples of the alkyl group also apply to an alkylthio group, an alkyl sulfoxy group, an alkylaryl group, an alkylamino group, an alkyl boron group, an alkyl silyl group, and an alkyl amine group.

Herein, the above-described examples of the aryl group also apply to an aryloxy group, an arylthio group, an aryl sulfoxy group, an arylamino group, an aryl boron group, an aryl silyl group, and an aryl amine group.

Herein, the phosphine oxide group may be substituted with, for example, at least one of the alkyl groups or aryl groups described above.

Herein, the phosphine sulfide group may be substituted with, for example, at least one of the alkyl groups or aryl groups described above.

Herein, a direct linkage may indicate a single bond (e.g., a single covalent bond).

Herein, "deuterated" indicates that isotopic abundance is greater than natural abundance for deuterium.

In the present description, 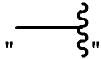 and 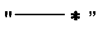 refers to a position to be connected.

Hereinafter, embodiments of the present disclosure will be described with reference to the accompanying drawings.

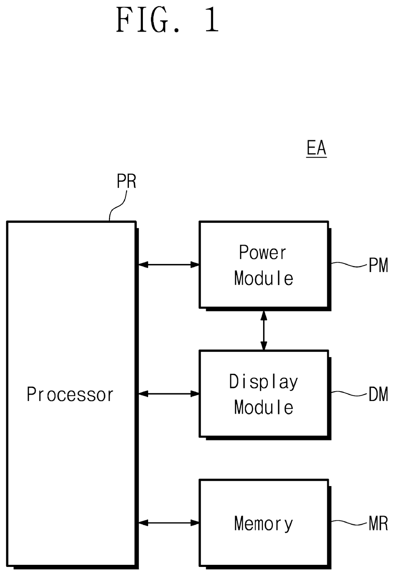

FIG. 1 is a block diagram of an electronic device according to an embodiment; Referring to FIG. 1, an electronic device EA according to an embodiment may include a display module DM, a processor PR, a memory MR, and a power module PM.

The processor PR may include at least one selected from a central processing unit (CPU), an application processor (AP), a graphic processing unit (GPU), a communication processor (CP), an image signal processor (ISP), and a controller.

The memory MR may store data information useful or required for the operation of the processor PR or the display module DM. If (e.g., when) the processor PR executes an application stored in the memory MR, image data signals and/or input control signals are transmitted to the display module DM, and the display module DM may process the received signal and output image information through a display screen. The display module DM may include a display panel that displays an image.

The power module PM may include a power supply module such as a power adapter or a battery device, and a power conversion module that converts power supplied by the power supply module to generate power useful or required for the operation of the electronic device EA.

At least one of the components of the electronic device EA described above may be included in a display module according to embodiment, which will be further described hereinbelow, and a display device according to an embodiment including the same. In embodiments, some of the individual modules functionally included in one module may be included in the display device, and others may be separately provided from the display device. For example, the display device may include a display module DM, and the processor PR, the memory MR, and the power module PM may be provided in the form of other devices within the electronic device EA, rather than the display device.

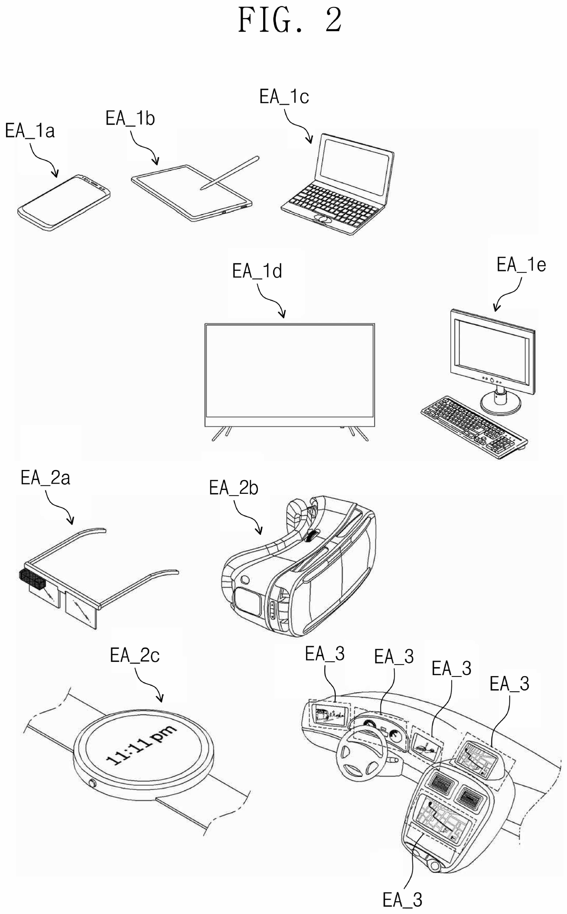

FIG. 2 shows schematic views of embodiments of various electronic devices.

Referring to FIG. 2, various electronic devices including the display module according to an embodiment may include electronic devices for displaying images, such as a smart phone EA_1a, a tablet PC EA_1b, a laptop EA_1c, a TV EA_1d, and a desk monitor EA_1e, wearable electronic devices such as smart glasses EA_2a, a head mounted display EA_2b, and a smart watch EA_2c, and vehicle electronic devices EA_3 such as a center information display (CID) and a room mirror display on an instrument panel, a center fascia, or a dashboard of a vehicle.

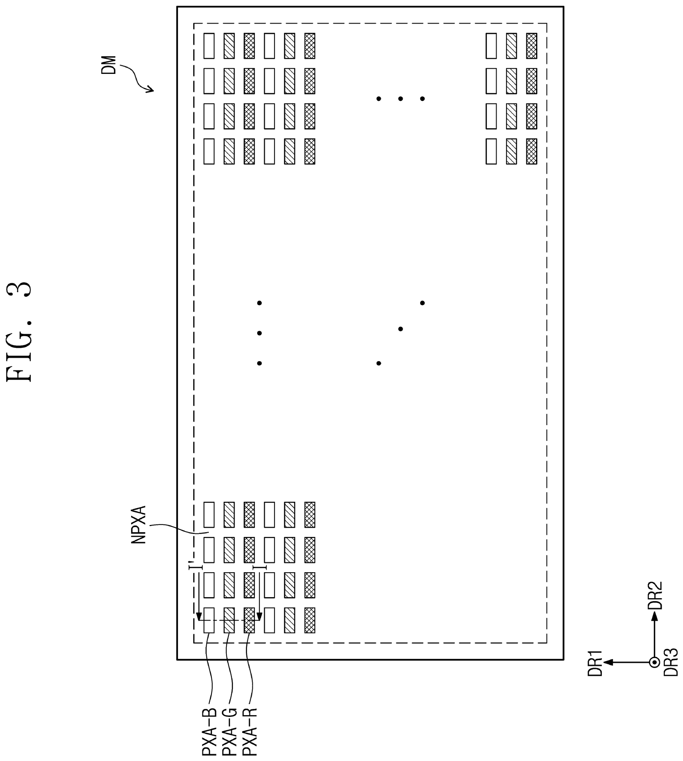

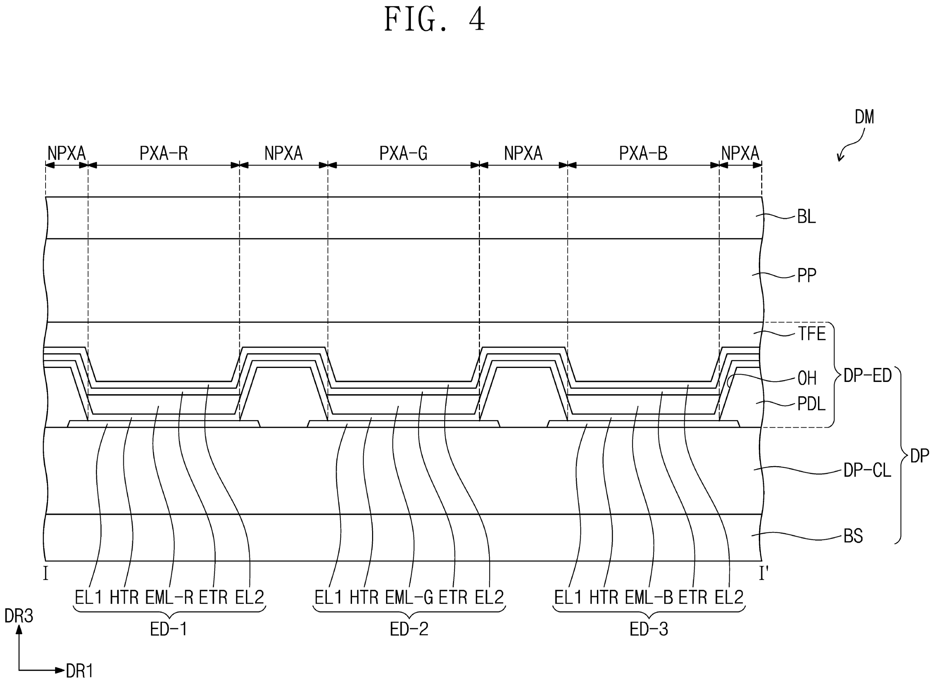

FIG. 3 is a plan view showing an embodiment of a display module DM. FIG. 4 is a cross-sectional view showing a display module DM according to an embodiment. FIG. 4 is a cross-sectional view showing a portion corresponding to line I-I' of FIG. 3;

The display module DM may include a plurality of light emitting elements ED-1, ED-2, and ED-3. In an embodiment, the display module DM may include a display panel DP including a plurality of light emitting elements ED-1, ED-2, and ED-3 and further include an optical layer PP on the display panel DP.

The display panel DP may include a base layer BS, a circuit layer DP-CL provided on the base layer BS, and a display element layer DP-ED. The display element layer DP-ED may include pixel defining films PDL, a plurality of light emitting elements ED-1, ED-2, and ED-3 between the pixel defining films PDL, and an encapsulation layer TFE on the plurality of light emitting elements ED-1, ED-2, and ED-3.

The base layer BS may be a member providing a base surface on which the display element layer DP-ED is provided. The base layer BS may be a glass substrate, a metal substrate, a plastic substrate, and/or the like. However, an embodiment is not limited thereto, and the base layer BS may be an inorganic layer, an organic layer, or a composite material layer.

In an embodiment, the circuit layer DP-CL may be on the base layer BS, and the circuit layer DP-CL may include a plurality of transistors. The transistors may each include a control electrode, an input electrode, and an output electrode. For example, the circuit layer DP-CL may include a switching transistor and a driving transistor for driving the light emitting elements ED-1, ED-2 and ED-3 of the display element layer DP-ED.

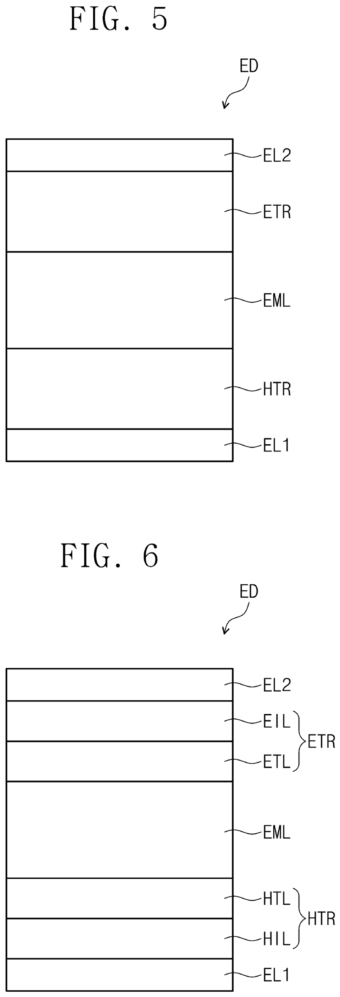

The light emitting elements ED-1, ED-2, and ED-3 may each have a structure of a light emitting element ED according to an embodiment from FIGS. 5 to 9, which will be further described herein. The light emitting elements ED-1, ED-2, and ED-3 may each include a first electrode EL1, a hole transport region HTR, emission layers EML-R, EML-G, and EML-B, an electron transport region ETR, and a second electrode EL2.

The optical layer PP may be on the display panel DP to control reflected light in the display panel DP due to external light. The optical layer PP may include, for example, a polarizing layer and/or a color filter layer. In embodiments, the optical layer PP may not be provided in the display module DM according to an embodiment.

A base substrate BL may be on the optical layer PP. The base substrate BL may be a member providing a base surface on which the optical layer PP is provided. The base substrate BL may be a glass substrate, a metal substrate, a plastic substrate, and/or the like. However, an embodiment is not limited thereto, and the base substrate BL may be an inorganic layer, an organic layer, or a composite material layer. In embodiments, the base substrate BL may not be provided in an embodiment.

The display module DM according to an embodiment may further include a filling layer. The filling layer may be between a display element layer DP-ED and the base substrate BL. The filling layer may be an organic material layer. The filling layer may include at least one selected from an acrylic resin, a silicone-based resin, and an epoxy-based resin.

FIG. 4 shows an embodiment in which the emission layers EML-R, EML-G, and EML-B of the light emitting elements ED-1, ED-2, and ED-3 are provided in openings OH defined in the pixel defining films PDL, and the hole transport region HTR, the electron transport region ETR, and the second electrode EL2 are provided as a common layer throughout the light emitting elements ED-1, ED-2, and ED-3. However, an embodiment is not limited thereto, and in an embodiment, the hole transport region HTR and the electron transport region ETR may be provided to be patterned inside the openings OH defined in the pixel defining films PDL. For example, in an embodiment, the hole transport region HTR, the emission layers EML-R, EML-G, and EML-B, and the electron transport region ETR, and the like of the light emitting elements ED-1, ED-2, and ED-3 may be patterned and provided through an inkjet printing method.

The encapsulation layer TFE may cover the light emitting elements ED-1, ED-2 and ED-3. The encapsulation layer TFE may seal the display element layer DP-ED. The encapsulation layer TFE may be a thin film encapsulation layer. The encapsulation layer TFE may be a single layer or a stack layer of a plurality of layers. The encapsulation layer TFE includes at least one insulating layer (e.g., electrically insulating layer). The encapsulation layer TFE according to an embodiment may include at least one inorganic film (hereinafter, an encapsulation inorganic film). In embodiments, the encapsulation layer TFE according to an embodiment may include at least one organic film (hereinafter, an encapsulation organic film) and at least one encapsulation inorganic film.

The encapsulation inorganic film protects the display element layer DP-ED from moisture/oxygen, and the encapsulation organic film protects the display element layer DP-ED from foreign substances such as dust particles. The encapsulation inorganic film may include silicon nitride, silicon oxy nitride, silicon oxide, titanium oxide, aluminium oxide, and/or the like, but is not particularly limited thereto. The encapsulation organic film may include an acrylic compound, an epoxy-based compound, and/or the like. The encapsulation organic film may include a photopolymerizable organic material, and is not particularly limited.

The encapsulation layer TFE may be on the second electrode EL2, and may be provided to fill the openings OH.

Referring to FIGS. 3 and 4, the display module DM may include a non-light emitting region NPXA and light emitting regions PXA-R, PXA-G, and PXA-B. The light emitting regions PXA-R, PXA-G, and PXA-B may each be a region emitting light generated from each of the light emitting elements ED-1, ED-2, and ED-3. The display module DM may include a first light emitting region PXA-R, a second light emitting region PXA-G, and a third light emitting region PXA-B.

The first to third light emitting regions PXA-R, PXA-G, and PXA-B may each be a region separated by the pixel defining film PDL. The non-light emitting region NPXA may be a region between neighboring light emitting regions PXA-R, PXA-G, and PXA-B, and may correspond to the pixel defining films PDL. Herein, the light emitting regions PXA-R, PXA-G, and PXA-B may each correspond to a pixel. The pixel defining films PDL may separate the light emitting elements ED-1, ED-2 and ED-3. The emission layers EML-R, EML-G, and EML-B of the light emitting elements ED-1, ED-2 and ED-3 may be provided in the openings OH defined by the pixel defining films PDL and thus be separated.

The light emitting regions PXA-R, PXA-G, and PXA-B may be divided into a plurality of groups according to the color of light generated from the light emitting elements ED-1, ED-2, and ED-3. In the display module DM according to an embodiment shown in FIGS. 3 and 4, three light emitting regions PXA-R, PXA-G, and PXA-B which emit red light, green light, and blue light, are shown as an example. For example, the first light emitting region PXA-R may be referred to as a red light emitting region, the second light emitting region PXA-G may be referred to as a green light emitting region, and the third light emitting region PXA-B may be referred to as a blue light emitting region.

In the display module DM according to an embodiment, the plurality of light emitting elements ED-1, ED-2, and ED-3 may emit light having different wavelength ranges. For example, in an embodiment, the display module DM may include a first light emitting element ED-1 emitting red light, a second light emitting element ED-2 emitting green light, and a third light emitting element ED-3 emitting blue light. In embodiments, the red light emitting region PXA-R, the green light emitting region PXA-G, and the blue light emitting region PXA-B of the display module DM may correspond to the first light emitting element ED-1, the second light emitting element ED-2, and the third light emitting element ED-3, respectively.

However, an embodiment is not limited thereto, and the first to third light emitting elements ED-1, ED-2 and ED-3 may emit light in the same wavelength range or emit light in at least one different wavelength range. For example, the first to third light emitting elements ED-1, ED-2, and ED-3 may all emit blue light.

The light emitting regions PXA-R, PXA-G, and PXA-B in the display module DM according to an embodiment may be arranged in the form of a stripe. Referring to FIG. 1, a plurality of first light emitting regions PXA-R, a plurality of second light emitting regions PXA-G, and a plurality of third light emitting regions PXA-B may each be arranged along the second directional axis DR2. In embodiments, the first light emitting region PXA-R, the second light emitting region PXA-G, and the third light emitting region PXA-B may be alternately arranged in that order along the first directional axis DR1.

FIGS. 3 and 4 show that the light emitting regions PXA-R, PXA-G, and PXA-B are all similar in size, but an embodiment is not limited thereto, and the light emitting regions PXA-R, PXA-G, and PXA-B may be different in size from each other according to wavelength range of emitted light. The areas of the light emitting regions PXA-R, PXA-G, and PXA-B may indicate areas if (e.g., when) viewed on a plane defined by the first directional axis DR1 and the second directional axis DR2.

The arrangement of the light emitting regions PXA-R, PXA-G, and PXA-B is not limited to what is shown in FIG. 1, and the order in which the first light emitting region PXA-R, the second light emitting region PXA-G, and the third light emitting region PXA-B are arranged comes with varied combinations according to display quality characteristics suitable or required for the display module DM and a display device DD including the same. For example, the light emitting regions PXA-R, PXA-G, and PXA-B may be arranged in the form of a PENTILE^{®} arrangement structure (e.g., an RGBG matrix, RGBG structure, or RGBG matrix structure) or a DIAMOND PIXEL^{®} arrangement structure, but the present disclosure is not limited thereto. PENTILE^{®} and DIAMOND PIXEL^{®} are duly registered trademarks of Samsung Display Co., Ltd.

In embodiments, areas of each of the light emitting regions PXA-R, PXA-G, and PXA-B may be different in size from one another. For example, in an embodiment, the second pixel region PXA-G corresponding to a green light emitting region may be smaller than the third pixel region PXA-B corresponding to a blue light emitting region in size, but an embodiment is not limited thereto.

Hereinafter, FIGS. 5 to 9 are cross-sectional views schematically showing a light emitting element according to an embodiment. The light emitting element ED according to an embodiment may include a first electrode EL1, a hole transport region HTR, an emission layer EML, an electron transport region ETR, and a second electrode EL2, which are sequentially stacked.

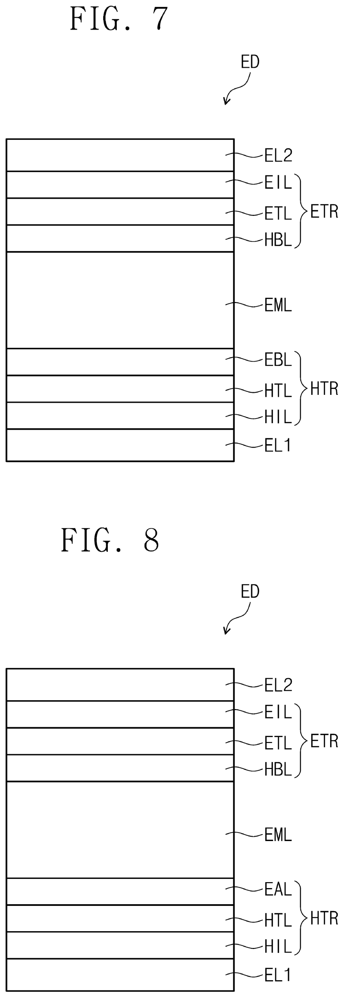

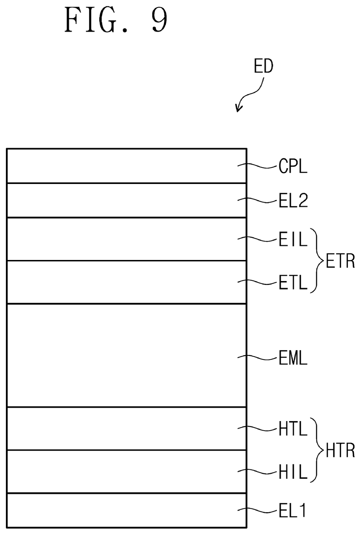

FIG. 6 shows, compared with FIG. 5, a cross-sectional view of a light emitting element ED according to an embodiment in which the hole transport region HTR includes a hole injection layer HIL and a hole transport layer HTL, and the electron transport region ETR includes an electron injection layer EIL and an electron transport layer ETL. In embodiments, FIG. 7 shows, compared with FIG. 5, a cross-sectional view of a light emitting element ED according to an embodiment, in which the hole transport region HTR includes a hole injection layer HIL, a hole transport layer HTL, and an electron blocking layer EBL, and the electron transport region ETR includes an electron injection layer EIL, an electron transport layer ETL, and a hole blocking layer HBL. FIG. 8 shows, compared with FIG. 5, a cross-sectional view of a light emitting element ED according to an embodiment in which the hole transport region HTR includes a hole injection layer HIL, a hole transport layer HTL, and an auxiliary emission layer EAL, and the electron transport region ETR includes an electron injection layer EIL, an electron transport layer ETL, and a hole blocking layer HBL. FIG. 9 shows, compared with FIG. 6, a cross-sectional view of a light emitting element ED according to an embodiment, in which a capping layer CPL on the second electrode EL2 is provided.

The first electrode EL1 has conductivity (e.g., electrical conductivity). The first electrode EL1 may be formed of a metal material, a metal alloy, and/or a conductive compound (e.g., an electrically conductive compound). The first electrode EL1 may be an anode or a cathode. However, an embodiment is not limited thereto. In embodiments, the first electrode EL1 may be a pixel electrode. The first electrode EL1 may be a transmissive electrode, a transflective electrode, or a reflective electrode. The first electrode EL1 may be a transmissive electrode, a transflective electrode, or a reflective electrode. The first electrode EL1 may include at least one selected from Ag, Mg, Cu, Al, Pt, Pd, Au, Ni, Nd, Ir, Cr, Li, Ca, LiF, Mo, Ti, W, In, Sn, and Zn, at least two compounds selected therefrom, two or more mixtures selected therefrom, or an oxide thereof.

If (e.g., when) the first electrode EL1 is a transmissive electrode, the first electrode EL1 may include a transparent metal oxide such as indium tin oxide (ITO), indium zinc oxide (IZO), zinc oxide (ZnO), and indium tin zinc oxide (ITZO). If (e.g., when) the first electrode EL1 is a transflective electrode or a reflective electrode, the first electrode EL1 may include Ag, Mg, Cu, Al, Pt, Pd, Au, Ni, Nd, Ir, Cr, Li, Ca, LiF/Ca (a stack structure of LiF and Ca), LiF/Al (a stack structure of LiF and Al), Mo, Ti, W, or a compound thereof or a mixture thereof (e.g., a mixture of Ag and Mg). In embodiments, the first electrode EL1 may have a multilayer structure including a reflective film or a transflective film formed of the above-described materials, and a transparent conductive film (e.g., a transparent electrically conductive film) formed of indium tin oxide (ITO), indium zinc oxide (IZO), zinc oxide (ZnO), indium tin zinc oxide (ITZO), and/or the like. For example, the first electrode EL1 may have a three-layer structure of ITO/Ag/ITO, but is not limited thereto. In embodiments, the first electrode EL1 may include the above-described metal materials, a combination of two or more metal materials selected from the above-described metal materials, and/or oxides of the above-described metal materials. The first electrode EL1 may have a thickness of about 700 Å to about 10000 Å. For example, the first electrode EL1 may have a thickness of about 1000 Å to about 3000 Å.

The hole transport region HTR is provided on the first electrode EL1. The hole transport region HTR may include at least one selected from a hole injection layer HIL, a hole transport layer HTL, an auxiliary emission layer EAL, and an electron blocking layer EBL. The hole transport region HTR may have, for example, a thickness of about 50 Å to about 15000 Å. The auxiliary emission layer EAL may also be referred to as a buffer layer.

The hole transport region HTR may have a single layer formed of a single material, a single layer formed of a plurality of different materials, or a multilayer structure having a plurality of layers formed of a plurality of different materials.

For example, the hole transport region HTR may have a single-layer structure formed of the hole injection layer HIL or the hole transport layer HTL, or a single-layer structure formed of a hole injection material or a hole transport material. For example, the hole transport region HTR may have a single-layer structure formed of a plurality of different materials, or a structure of hole injection layer HIL/hole transport layer HTL, hole injection layer HIL/hole transport layer HTL/auxiliary emission layer EAL, hole injection layer HIL/auxiliary emission layer EAL, hole transport layer HTL/auxiliary emission layer EAL, hole injection layer HIL/hole transport layer HTL/auxiliary emission layer EAL, or hole injection layer HIL/hole transport layer HTL/electron blocking layer EBL stacked in order from the first electrode EL1, but an embodiment is not limited thereto.

The hole transport region HTR may be formed using various suitable methods such as a vacuum deposition method, a spin coating method, a cast method, a Langmuir-Blodgett (LB) method, an inkjet printing method, a laser printing method, and/or a laser induced thermal imaging (LITI) method.

The light emitting element ED according to an embodiment includes the amine compound according to an embodiment represented by Formula 1 below in the hole transport region HTR. At least one selected from the hole injection layer HIL, the hole transport layer HTL, the electron blocking layer EBL, and the auxiliary emission layer EAL may include the amine compound according to an embodiment. For example, the light emitting element ED according to an embodiment may include the amine compound according to an embodiment in the hole transport layer HTL.

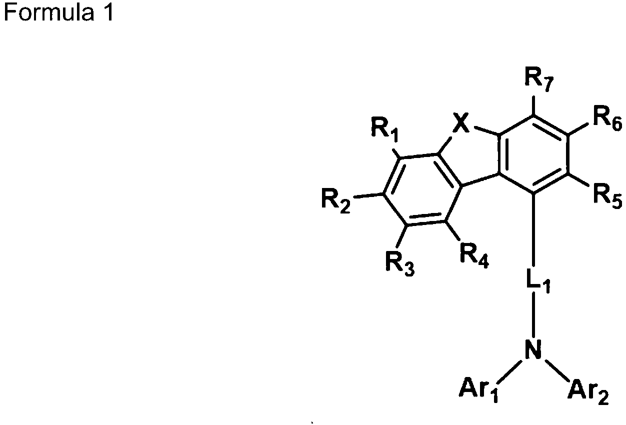

The amine compound according to an embodiment includes first to third substituents directly or indirectly bonded to a nitrogen atom (N). The first substituent is a substituted or unsubstituted dibenzoheteroyl group. The second and third substituents are each independently a substituted or unsubstituted naphthyl group. The first substituent may be bonded directly to the nitrogen atom (N) or may be bonded to the nitrogen atom (N) through a linker. The second and third substituents are bonded to the nitrogen atom (N) through a linker.

In the amine compound according to an embodiment, the substituted or unsubstituted dibenzoheteroyl group may be bonded directly to the nitrogen atom at a c1 position, or may be bonded to the nitrogen atom through a linker, as shown below. Examples of the dibenzoheteroyl group listed below only show a core structure of the dibenzoheteroyl group and bonding positions thereof, and other substituents are not provided. Herein, the dibenzoheteroyl group that is bonded to the nitrogen atom of an amine compound may be referred to as a 1-dibenzoheteroyl group.

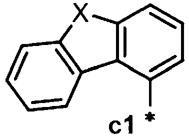

In the amine compound according to an embodiment, the substituted or unsubstituted naphthyl group may be bonded to the nitrogen atom through a linker at a c2 position or a c3 position, as shown below. Examples of the naphthyl group listed below only show a core structure of the naphthyl group and bonding positions thereof, and other substituents are not provided.

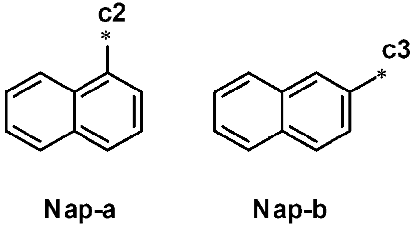

In the amine compound according to an embodiment, at least two of the dibenzoheteroyl group and two different naphthyl groups may include an aromatic compound (aryl or heteroaryl) as a substituent.

The amine compound according to an embodiment includes the first to third substituents, which control charge balance through a steric effect, thereby exhibiting excellent hole transport properties. For example, the amine compound according to an embodiment includes a substituted or unsubstituted 1-dibenzoheteroyl group that is directly bonded to a nitrogen atom or that is bonded to a nitrogen atom through a linker at a set or specific position, and two different substituted or unsubstituted naphthyl groups that are bonded to a nitrogen atom through a linker, and may thus have excellent hole transport properties and material stability, thereby contributing to high efficiency and long lifespan of a light emitting element. In embodiments, this allows the light emitting element to operate at low voltage. The amine compound according to an embodiment may be a monoamine compound including no amine group.

In Formula 1, L₁ is a direct linkage, a substituted or unsubstituted arylene group having 6 to 30 ring-forming carbon atoms, or a substituted or unsubstituted heteroarylene group having 2 to 30 ring-forming carbon atoms.

In an embodiment, L₁ may be a direct linkage, or a substituted or unsubstituted divalent phenyl group. The substituted or unsubstituted divalent phenyl group may include a hydrogen atom or a deuterium atom as a substituent.

X is O or S. In the amine compound according to an embodiment, the 1-dibenzoheteroyl group bonded to the nitrogen atom may be a substituted or unsubstituted 1-dibenzofuran, or a substituted or unsubstituted 1-dibenzothiophene.

R₁ to R₇ are each independently a hydrogen atom, a deuterium atom, a halogen atom, a substituted or unsubstituted alkyl group having 1 to 10 carbon atoms, a substituted or unsubstituted aryl group having 6 to 30 ring-forming carbon atoms, or a substituted or unsubstituted heteroaryl group having 5 to 30 ring-forming carbon atoms.

In an embodiment, at least any one selected from R₁ to R₇ may be a substituted or unsubstituted phenyl group, and the others of R₁ to R₇ may be a hydrogen atom or a deuterium atom.

For example, R₁ to R₇ may each independently be a hydrogen atom or a deuterium atom.

In Formula 1, Ar₁ and Ar₂ are each a substituted or unsubstituted naphthyl group bonded to a nitrogen atom (N) through a linker. However, a case in which Ar₁ and Ar₂ include a 4-(8-phenylnaphthalen-1-yl)phenyl group, a 4-(2-phenylnaphthalen-1-yl)phenyl group, or a 1,1'-binapthyl group is excluded. The 4-(8-phenylnaphthylen-1-yl)phenyl group is a compound in which a phenyl group is bonded to carbon at the 8-position of naphthalene in the 4-(naphthalen-1-yl)phenyl group. A 4-(2-phenylnaphthylen-1-yl)phenyl group is a compound in which a phenyl group is bonded to a carbon at the 2-position of naphthalene in the 4-(naphthalen-1-yl)phenyl group.

In an embodiment, each of Ar₁ and Ar₂ does not include compounds represented by Ar-x1 to Ar-x3 below.
Ar-x1

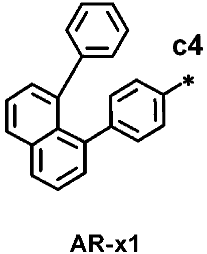

In Ar-x1, c4 is a position that is bonded to a nitrogen atom. Ar-x1 only shows a core structure of the 4-(8-phenylnaphthylen-1-yl)phenyl group and bonding positions thereof, and other substituents are not provided.
Ar-x2

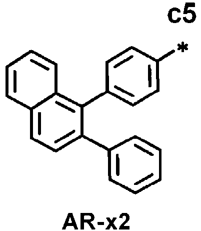

In Ar-x2, c5 is a position that is bonded to a nitrogen atom. Ar-x2 only shows a core structure of the 4-(2-phenylnaphthylen-1-yl)phenyl group and bonding positions thereof, and other substituents are not provided.
Ar-x3

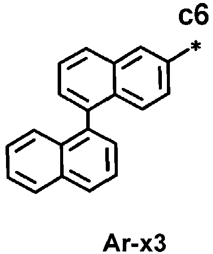

In Ar-x3, c6 is a position that is bonded to a nitrogen atom. Ar-x3 only shows a core structure of the 1,1'-binaphthyl group and bonding positions thereof, and other substituents are not provided.

In an embodiment, Ar₁ and Ar₂ are different from each other.

In an embodiment, Ar₁ and Ar₂ are each independently represented by Formula 2 below.

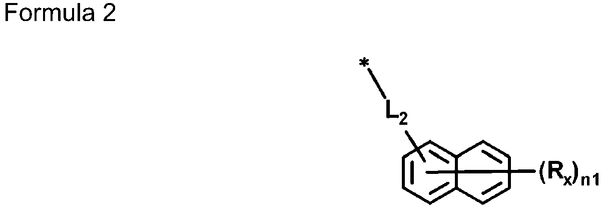

In Formula 2,
L₂ is a substituted or unsubstituted arylene group having 6 to 30 ring-forming carbon atoms, or a substituted or unsubstituted heteroarylene group having 5 to 30 ring-forming carbon atoms.

In an embodiment, L₂ may be a substituted or unsubstituted divalent phenyl group, or a substituted or unsubstituted divalent naphthyl group. The substituted or unsubstituted divalent phenyl group may include a hydrogen atom or a deuterium atom as a substituent. The substituted or unsubstituted divalent naphthyl group may include a hydrogen atom or a deuterium atom as a substituent.

For example, L₂ may be represented by any one selected from L-a and L-b below.

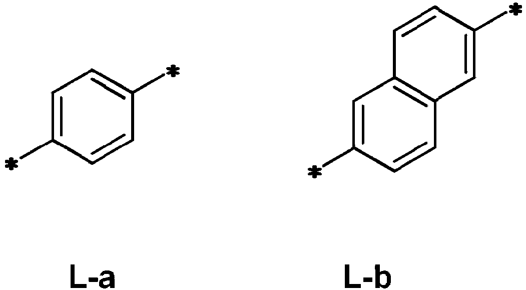

In an embodiment, at least one hydrogen atom of L-a and L-b may be substituted with a deuterium atom.

Rₓ is a hydrogen atom, a deuterium atom, a halogen atom, a substituted or unsubstituted alkyl group having 1 to 10 carbon atoms, a substituted or unsubstituted aryl group having 6 to 30 ring-forming carbon atoms, or a substituted or unsubstituted heteroaryl group having 5 to 30 ring-forming carbon atoms wherein a heteroatom is not a bonding position.

n1 is an integer of 0 to 7. If (e.g., when) n1 is an integer of 2 or greater, a plurality of Rₓ's may all be the same or at least one may be different from the others.

However, in Formula 2, if (e.g., when) Rₓ is a fused ring in which rings having three or more rings are fused, a case in which the fused ring Rₓ is bonded to a carbon adjacent to a carbon bonded to L₂ is excluded. In the amine compound according to an embodiment, if (e.g., when) Rₓ is a fused ring in which rings having three or more rings are fused, cases in which Rₓ is bonded to a carbon adjacent to a carbon bonded to L₂, such as Ar-y1 to Ar-y3 below, are excluded. Ar-y1 to Ar-y3 listed below only show one Rₓ bonding position as a substituent, and other Rₓ substituents are not provided.

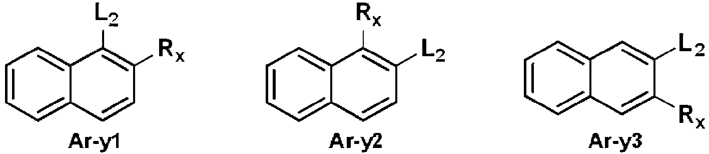

In the amine compound according to an embodiment, at least two selected from i) a substituted or unsubstituted 1-dibenzoheteroyl group bonded to a nitrogen atom, ii) a first naphthyl group bonded to the nitrogen atom through a linker, and iii) a second naphthyl group bonded to a nitrogen atom through a linker include a substituted or unsubstituted aryl group having 6 to 30 carbon atoms or a substituted or unsubstituted heteroaryl group having 5 to 30 ring-forming carbon atoms as a substituent. If (e.g., when) the first and second naphthyl groups include a substituted or unsubstituted heteroaryl group having 5 to 30 ring-forming carbon atoms as a substituent, a heteroatom may not be used as a bonding position.

In an embodiment, the substituted or unsubstituted 1-dibenzoheteroyl group bonded to the nitrogen atom and the naphthyl group included in Ar₁ may include at least one aryl group or heteroaryl group as a substituent. In embodiments, at least one selected from R₁ to R₇ and Rₓ of Ar₁ is a substituted or unsubstituted aryl group having 6 to 30 ring-forming carbon atoms, or a substituted or unsubstituted heteroaryl group having 5 to 30 ring-forming carbon atoms. If (e.g., when) Rₓ of Ar₁ includes a substituted or unsubstituted heteroaryl group having 5 to 30 ring-forming carbon atoms as a substituent, a heteroatom may not be used as a bonding position.

In an embodiment, the naphthyl group included in Ar₂ may include at least one aryl group or heteroaryl group as a substituent. In embodiments, at least one selected from Rₓ's of Ar₂ is a substituted or unsubstituted aryl group having 6 to 30 ring-forming carbon atoms, or a substituted or unsubstituted heteroaryl group having 5 to 30 ring-forming carbon atoms where a heteroatom is not a bonding position.

In an embodiment, the amine compound may be represented by Formula 1-1 below.

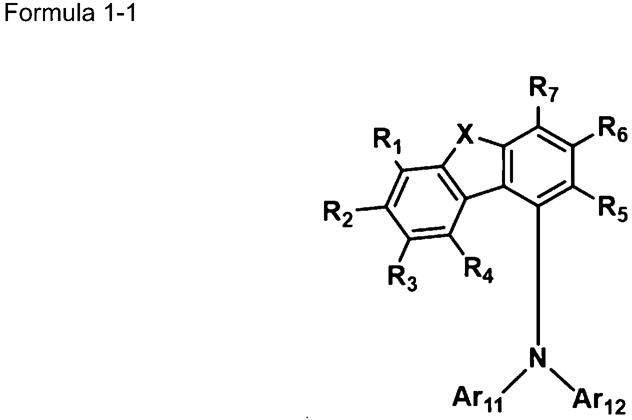

In Formula 1-1, the description of Formula 1 also applies to R₁ to R₇, and X.

Ar₁₁ and Ar₁₂ are each represented by one selected from Ar-a to Ar-k below, and Ar₁₁ and Ar₁₂ are different from each other.

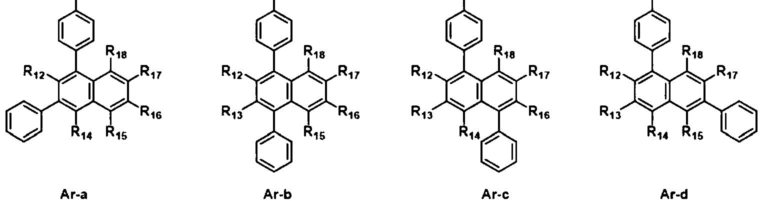

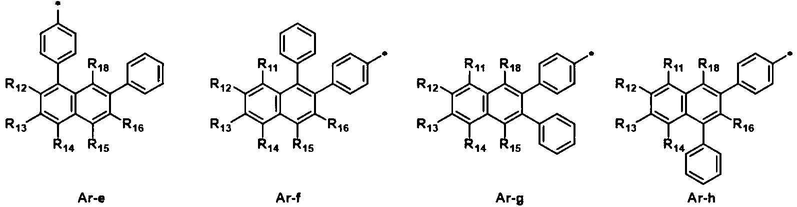

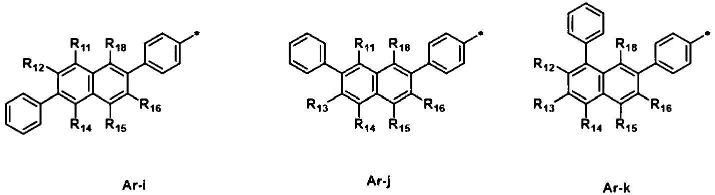

In Ar-a to Ar-k, R₁₁ to R₁₈ are each independently a hydrogen atom, a deuterium atom, a halogen atom, or a substituted or unsubstituted alkyl group having 1 to 10 carbon atoms.

For example, R₁₁ to R₁₈ may each independently be substituted with a hydrogen atom or a deuterium atom.

In an embodiment, the amine compound may be represented by any one selected from Formulas 1-a to 1-c below.

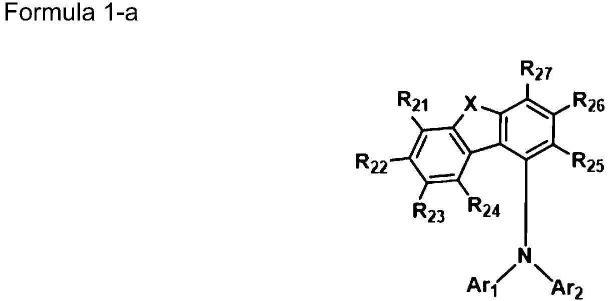

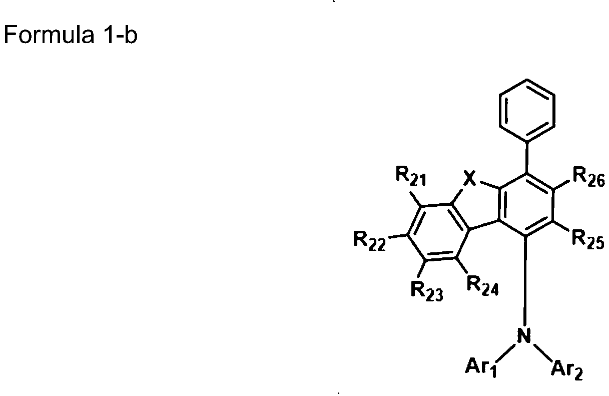

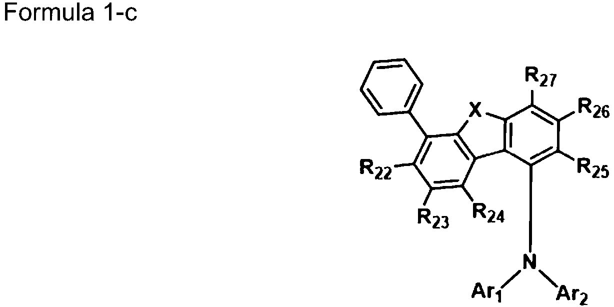

In Formulas 1-a to 1-c above, R₂₁ to R₂₇ are each independently a hydrogen atom, a deuterium atom, a halogen atom, or a substituted or unsubstituted alkyl group having 1 to 10 carbon atoms, and
the description of Formula 1 also applies to X, Ar₁, and Ar₂.

The amine compound according to an embodiment may be a compound in which at least one hydrogen atom is substituted with a deuterium atom from the compound represented by Formula 1 above.

The amine compound according to an embodiment may be represented by one selected from compounds from Compound Group 1 below. The hole transport region HTR of the light emitting element ED according to an embodiment may include at least one selected from the amine compounds disclosed in Compound Group 1 below. In Compound Group 1 below, D is a deuterium atom.

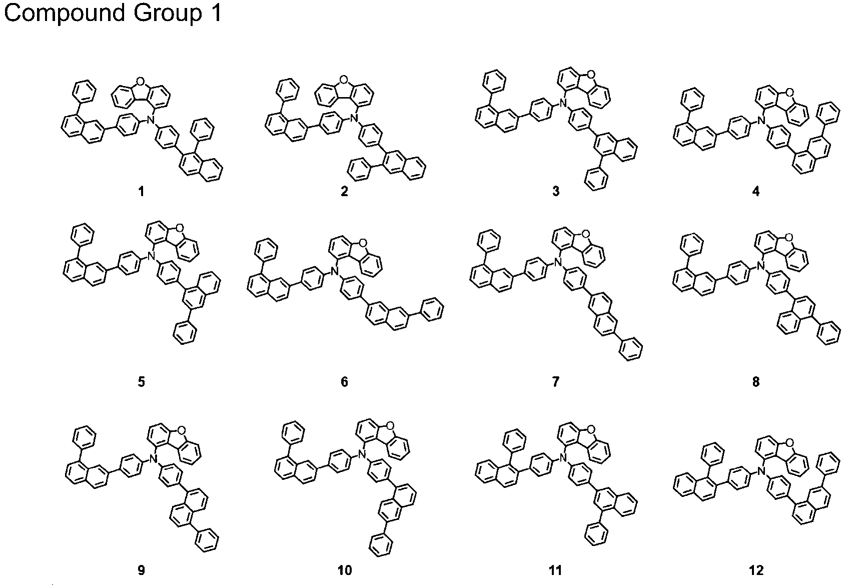

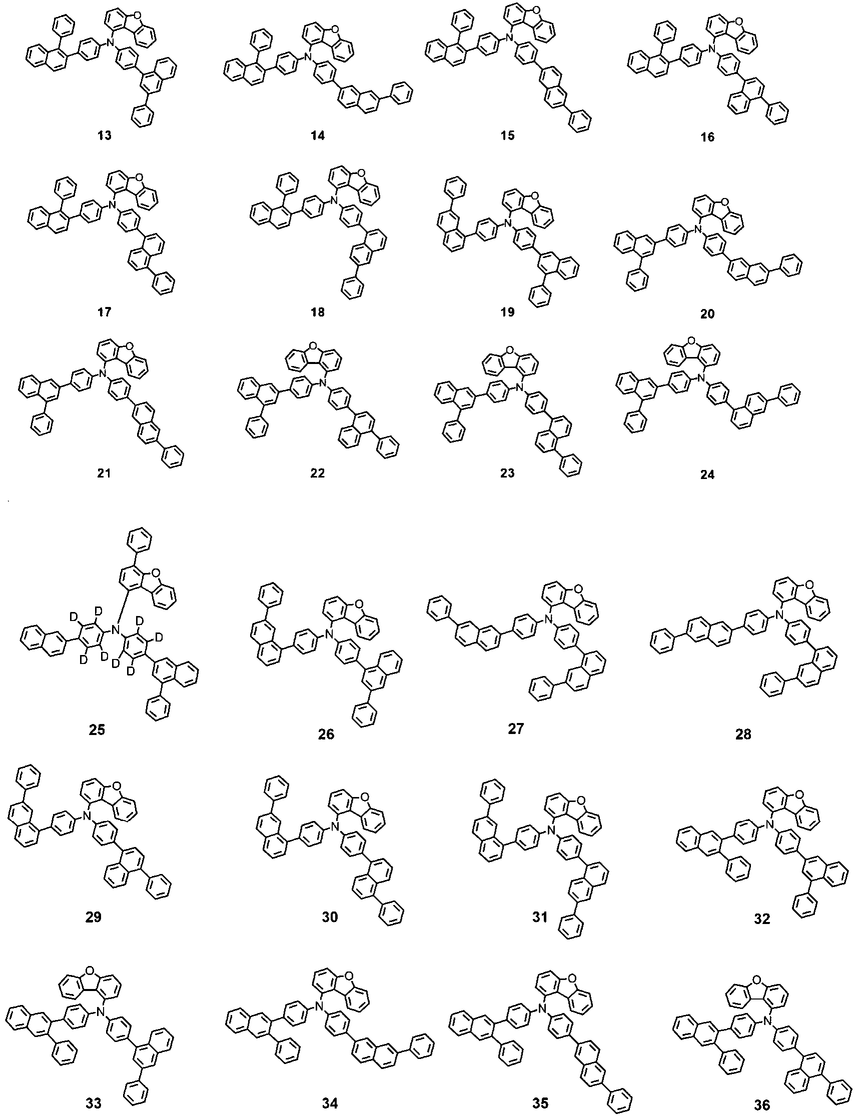

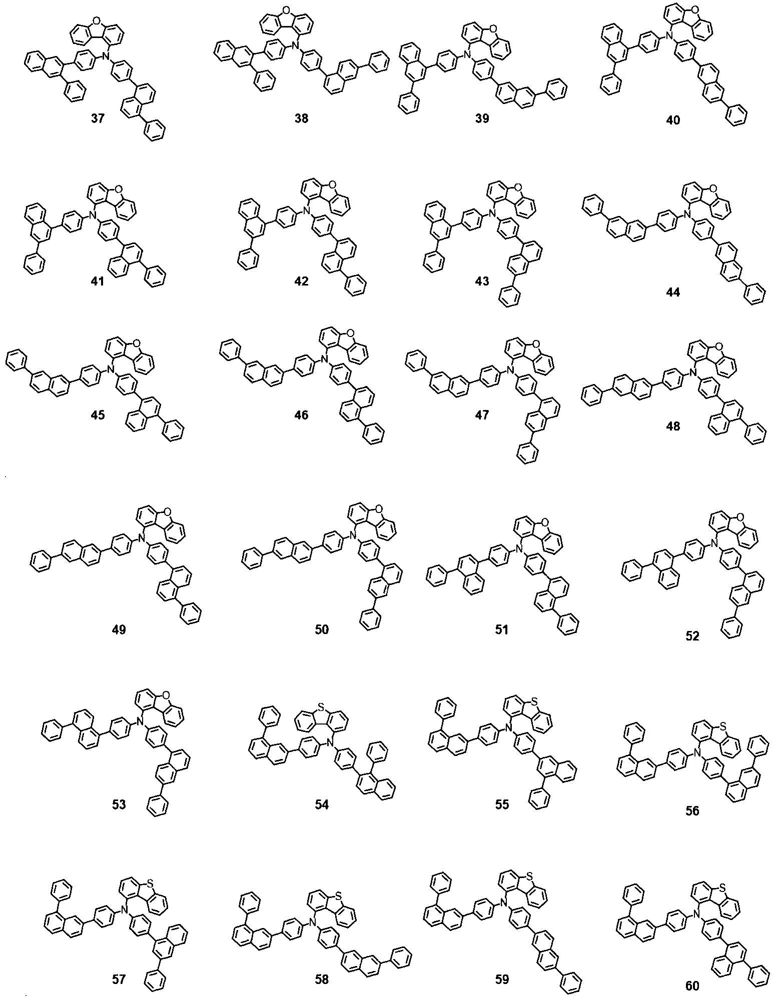

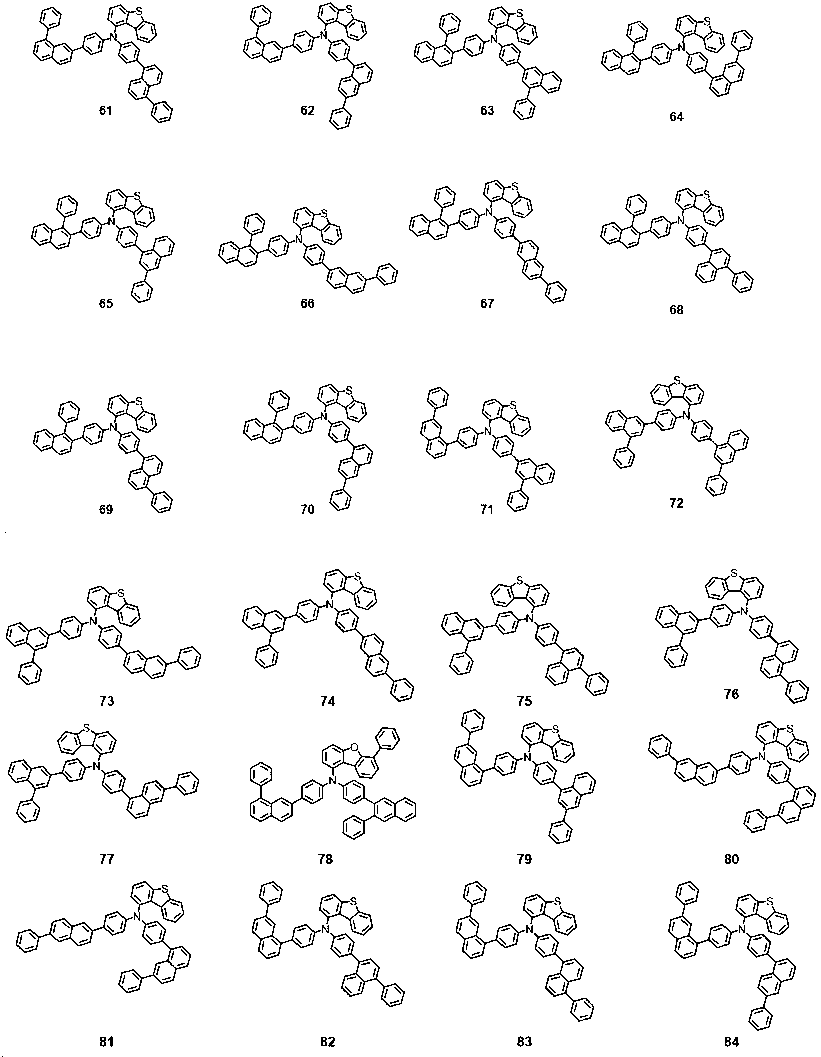

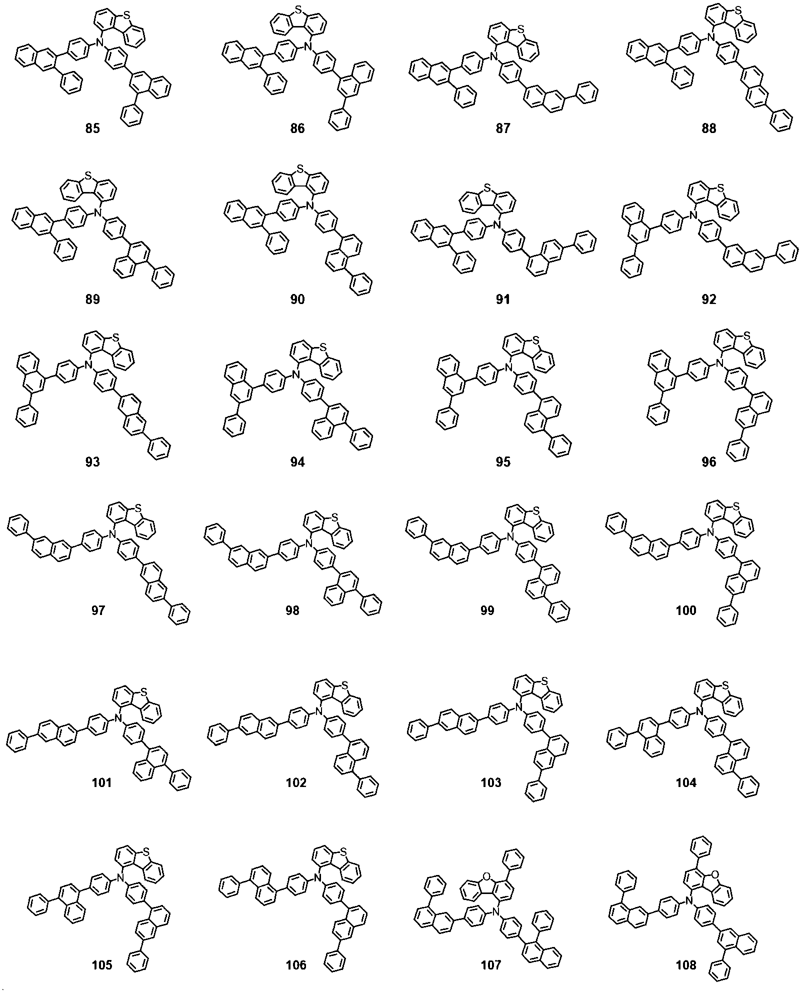

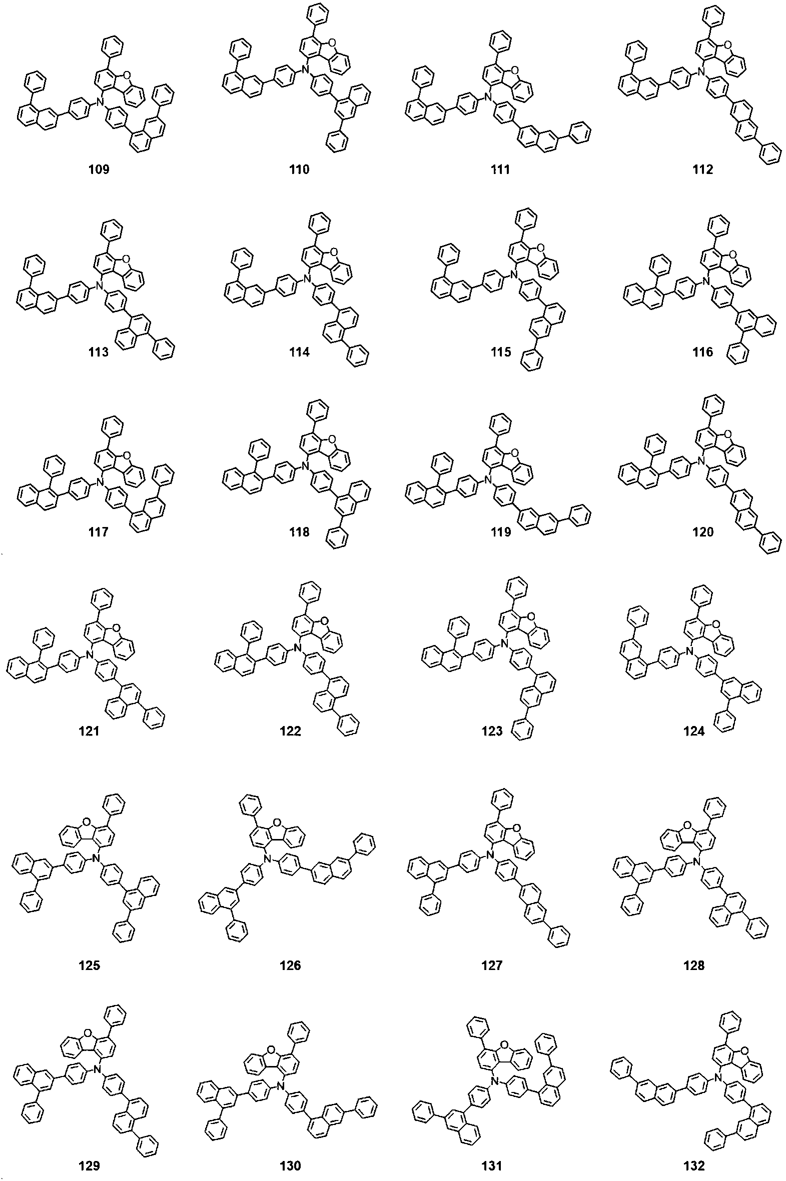

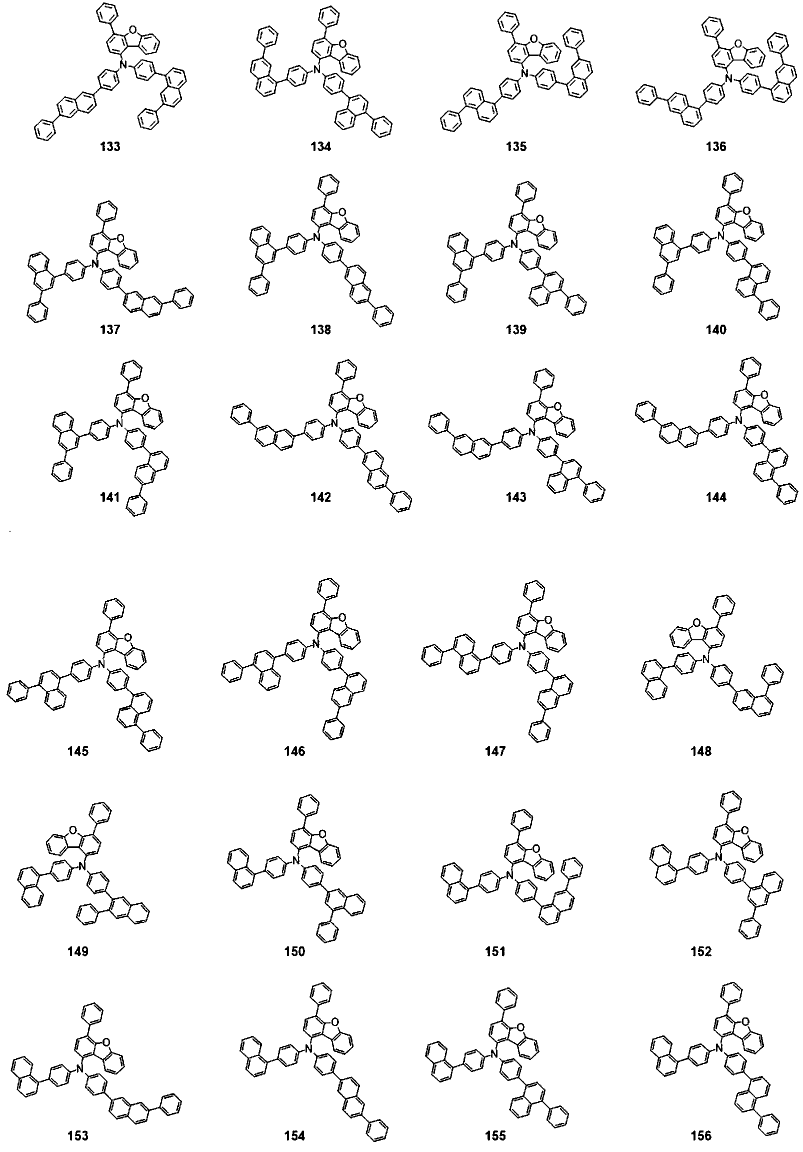

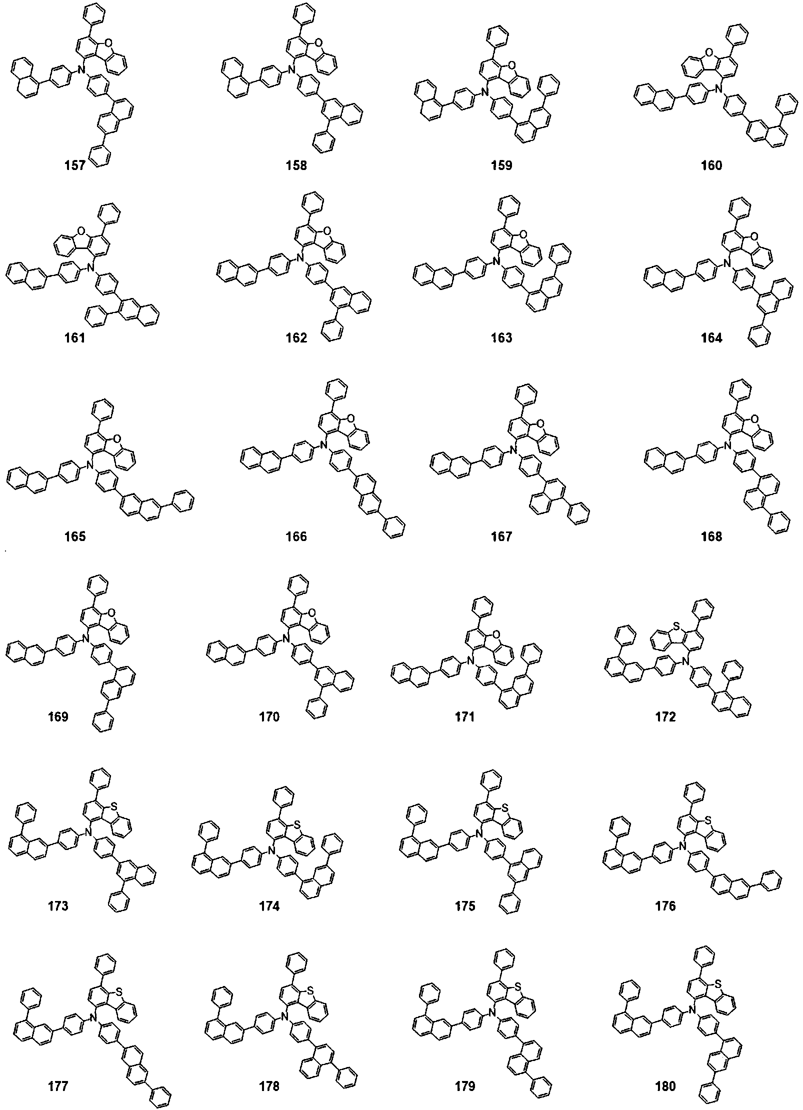

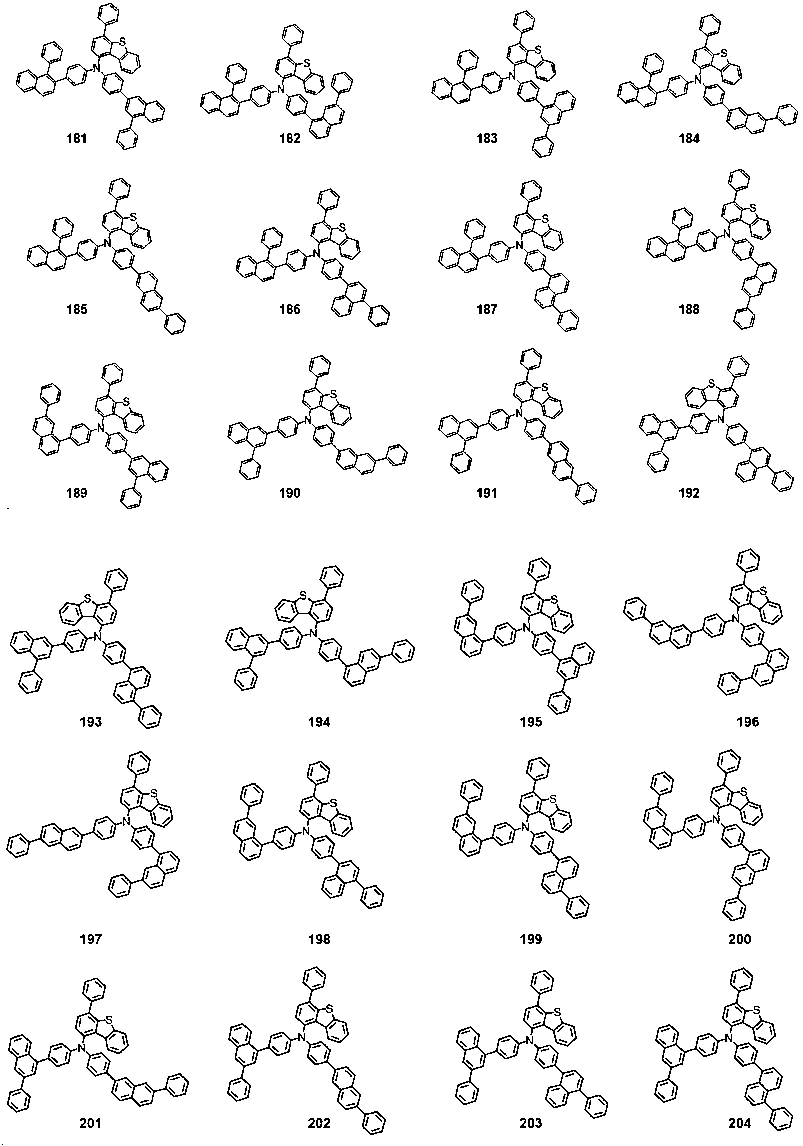

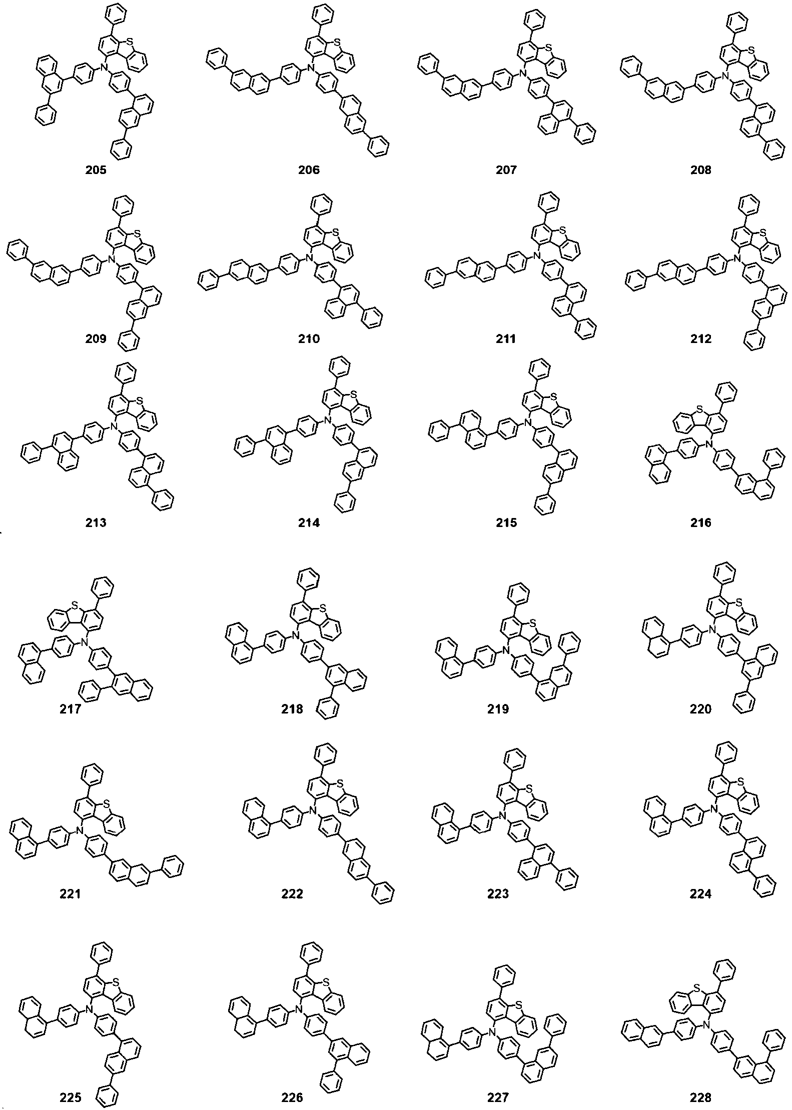

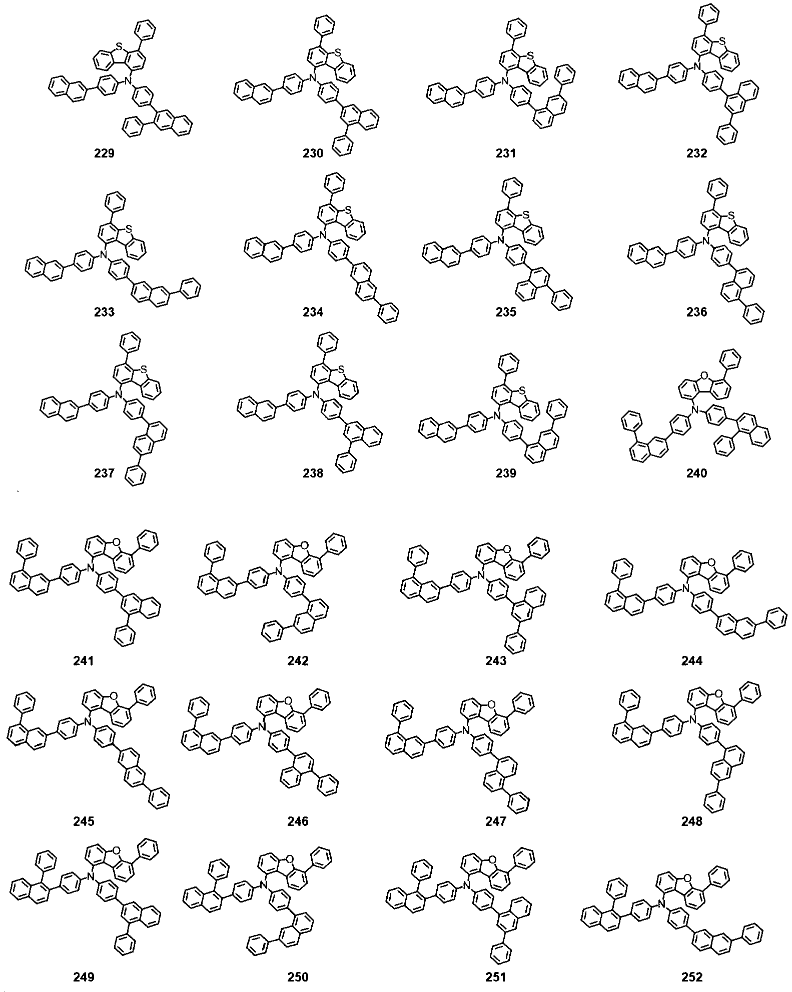

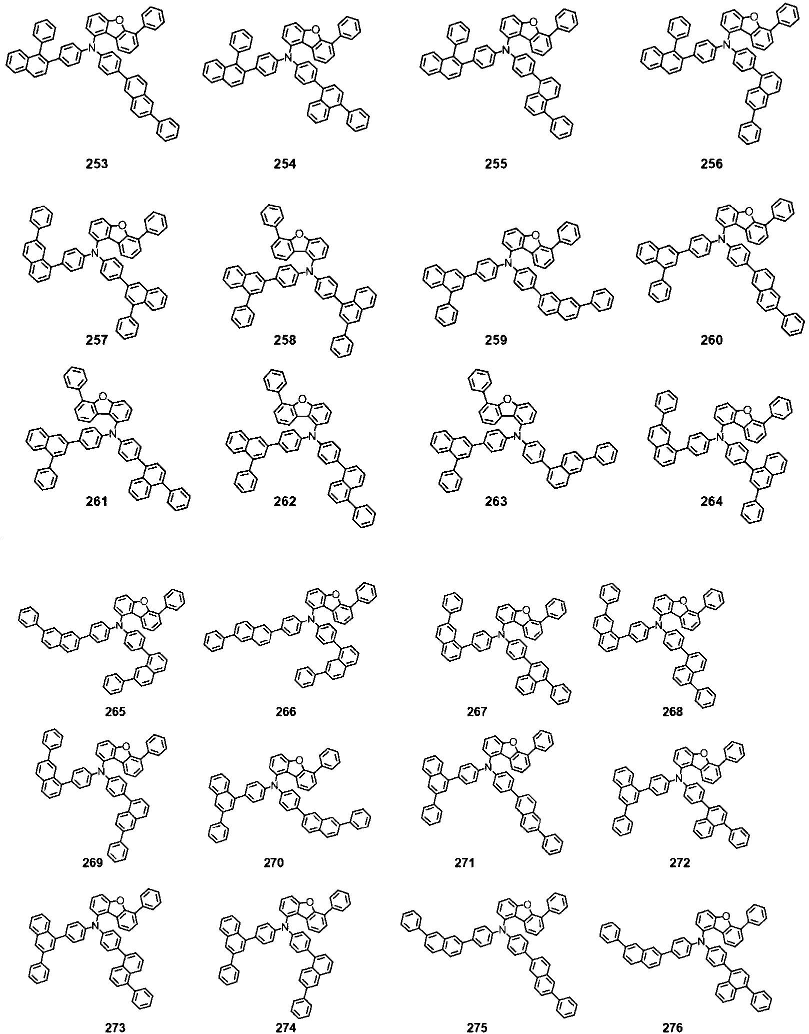

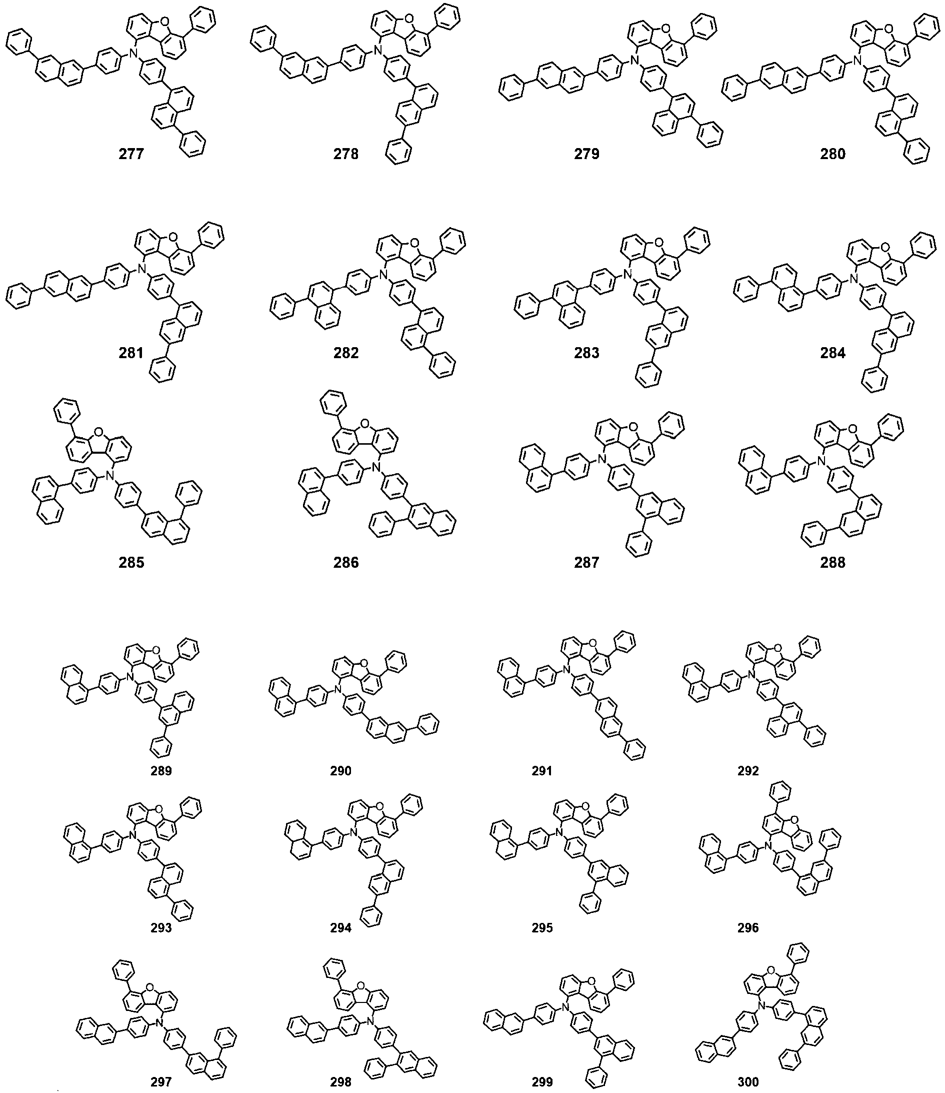

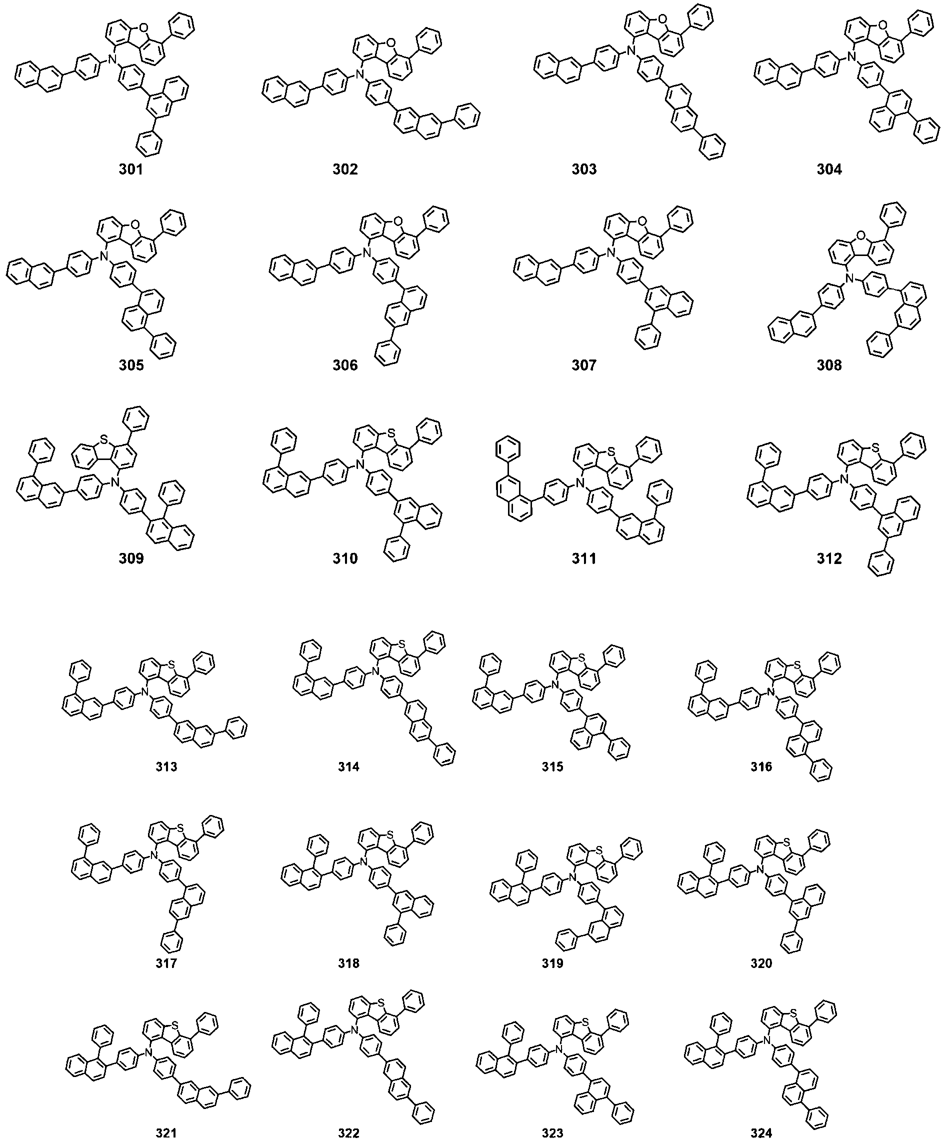

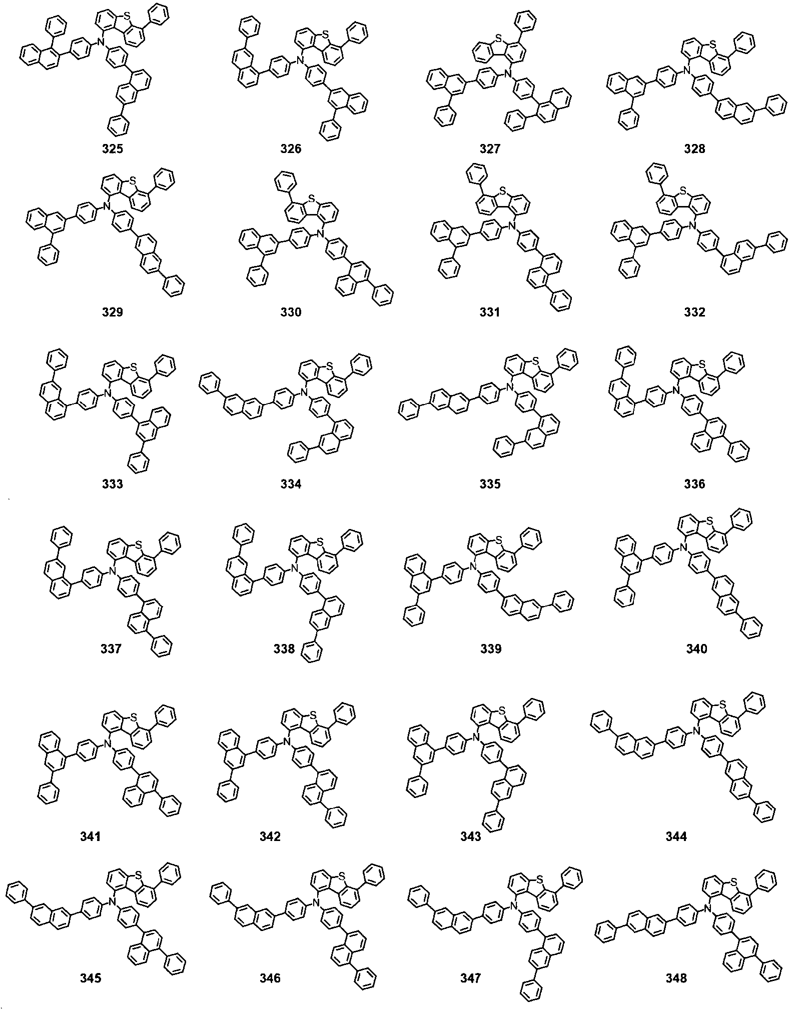

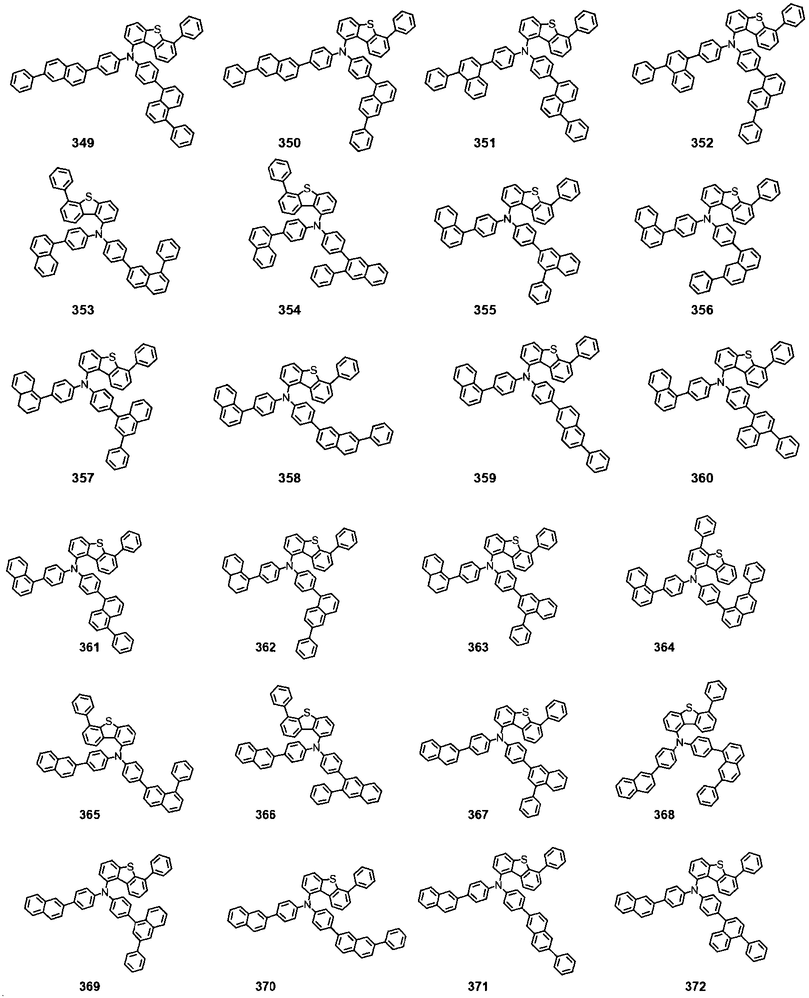

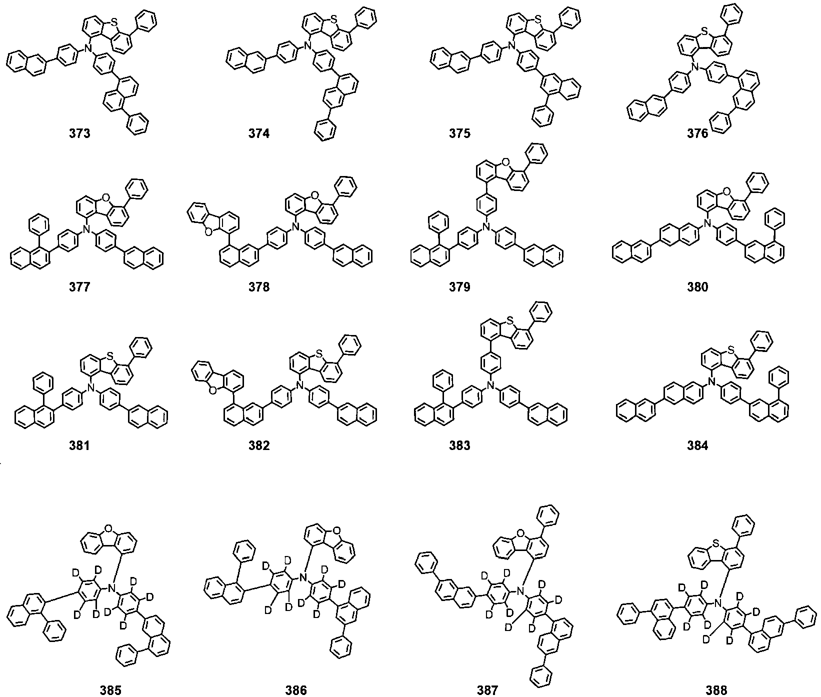

The amine compound according to an embodiment disclosed in Compound Group 1 includes a substituted or unsubstituted 1-dibenzoheteroyl group bonded directly to a nitrogen atom or bonded to a nitrogen atom through a linker at a set or specific position, and two different substituted or unsubstituted naphthyl groups bonded to a nitrogen atom through a linker. In embodiments, at least two of the 1-dibenzoheteroyl group and the two different naphthyl groups may be substituted with an aromatic compound. This results in excellent hole transport properties and material stability, thereby improving the efficiency and lifespan of a light emitting element.

In the light emitting element ED according to an embodiment, the hole transport region HTR may further include a compound represented by Formula H-1 below. For example, the light emitting element ED according to an embodiment may include a compound represented by Formula H-1 in another layer of the hole transport region HTR that does not include the amine compound according to an embodiment in Formula 1 described above. However, an embodiment is not limited thereto.

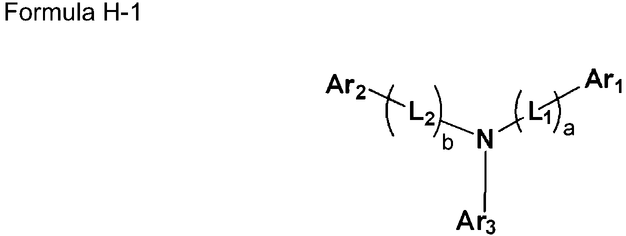

In Formula H-1 above, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. a and b may each independently be an integer of 0 to 10. If (e.g., when) a or b is an integer of 2 or greater, a plurality of L₁'s and L₂'s may each independently be a substituted or unsubstituted arylene group having 6 to 30 ring-forming carbon atoms, or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. In embodiments, in Formula H-1, Ar₃ may be a substituted or unsubstituted aryl group having 6 to 30 ring-forming carbon atoms, or a substituted or unsubstituted heteroaryl group having 2 to 30 ring-forming carbon atoms.

The compound represented by Formula H-1 above may be a monoamine compound. In embodiments, the compound represented by Formula H-1 may be a diamine compound in which at least one selected from Ar₁ to Ar₃ includes an amine group as a substituent. In embodiments, the compound represented by Formula H-1 above may be a carbazole-based compound including a substituted or unsubstituted carbazole moiety in at least one selected from Ar₁ and Ar₂ or a substituted or unsubstituted fluorene-based group including a substituted or unsubstituted fluorene moiety in at least one selected from Ar₁ and Ar₂.

The compound represented by Formula H-1 may be represented by any one selected from compounds from Compound Group H below. However, the compounds listed in Compound Group H below are presented as an example, and the compound represented by Formula H-1 is not limited to the those listed in Compound Group H below.

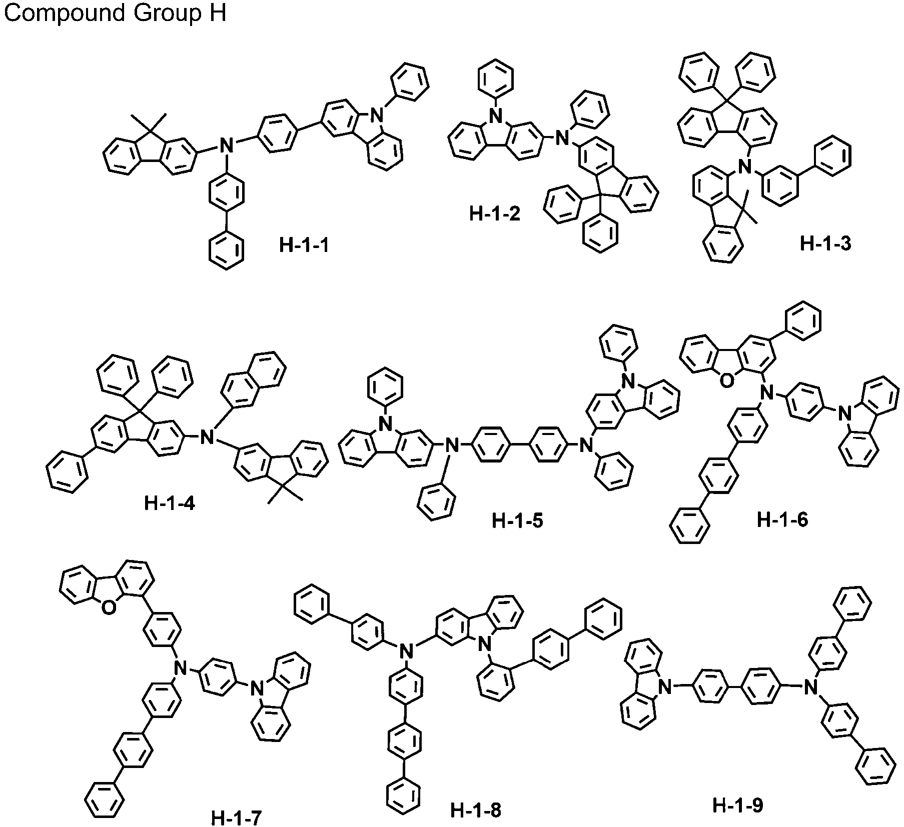

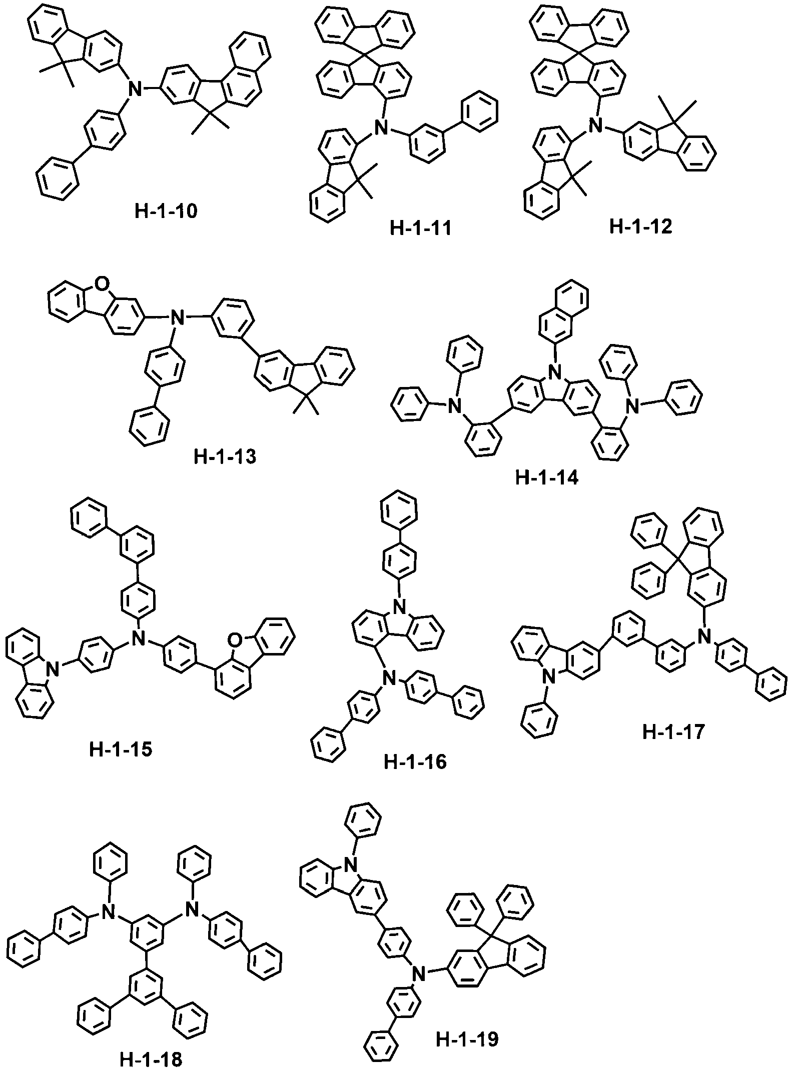

The hole transport region HTR may further include a phthalocyanine compound such as copper phthalocyanine, N¹,N^{1'}-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4'4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonicacid (PANI/CSA), polyaniline/poly(4-styrenesulfonate) (PANI/PSS), N,N'-di(naphthalene-I-yl)-N,N'-diphenyl-benzidine (NPB), triphenylamine-containing polyetherketone (TPAPEK), 4-Isopropyl-4'-methyldiphenyliodonium [tetrakis(pentafluorophenyl)borate], dipyrazino[2,3-f: 2',3'-h] quinoxaline-2,3,6,7,10,11-hexacarbonitrile (HAT-CN), and/or the like.

The hole transport region HTR may further include carbazole-based derivatives such as N-phenyl carbazole and polyvinyl carbazole, fluorene-based derivatives, N,N'-bis(3-methylphenyl)-N,N'-diphenyl-[1,1'-biphenyl]-4,4'-diamine (TPD), triphenylamine-based derivatives such as 4,4',4"-tris(N-carbazolyl)triphenylamine (TCTA), N,N'-di(naphthalene-I-yl)-N,N'-diphenyl-benzidine (NPB), 4,4'-Cyclohexylidene bis[N,N-bis(4-methylphenyl)benzenamine] (TAPC), 4,4'-bis[N,N'-(3-tolyl)amino]-3,3'-dimethylbiphenyl (HMTPD), 1,3-bis(N-carbazolyl)benzene (mCP), and/or the like.

In embodiments, the hole transport region HTR may further include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), and/or the like.

The hole transport region HTR may include the compounds of the hole transport region described above in at least one selected from the hole injection layer HIL, the hole transport layer HTL, the auxiliary emission layer EAL, and the electron blocking layer EBL.

The hole transport region HTR may have a thickness of about 100 Å to about 10000 Å, for example, about 100 Å to about 5000 Å. If (e.g., when) the hole transport region HTR includes the hole injection layer HIL, the hole injection layer HIL may have a thickness of, for example, about 30 Å to about 1000 Å. If (e.g., when) the hole transport region HTR includes the hole transport layer HTL, the hole transport layer HTL may have a thickness of about 30 Å to about 1000 Å. If (e.g., when) the hole transport region HTR includes the electron blocking layer EBL, the electron blocking layer EBL may have a thickness of, for example, about 10 Å to about 1000 Å. If (e.g., when) the thicknesses of the hole transport region HTR, the hole injection layer HIL, the hole transport layer HTL, and the electron blocking layer EBL satisfy the above-described ranges, suitable or satisfactory hole transport properties may be obtained without a substantial increase in driving voltage.

The hole transport region HTR may further include, in addition to the above-described materials, a charge generation material to increase conductivity (e.g., electrical conductivity). The charge generation material may be uniformly or non-uniformly dispersed in the hole transport region HTR. The charge generation material may be, for example, a p-dopant. The p-dopant may include at least one selected from halogenated metal compounds, quinone derivatives, metal oxides, and cyano group-containing compounds, but is not limited thereto. For example, the p-dopant may include halogenated metal compounds such as Cul and/or Rbl, quinone derivatives such as tetracyanoquinodimethane (TCNQ) and/or 2,3,5,6-tetrafluoro-7,7,8,8-tetracyanoquinodimethane (F4-TCNQ), metal oxides such as tungsten oxides and/or molybdenum oxides, cyano group-containing compounds such as dipyrazino[2,3-f: 2',3'-h] quinoxaline-2,3,6,7,10,11-hexacarbonitrile (HATCN) and/or 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), and/or the like, but an embodiment is not limited thereto.

As described above, the hole transport region HTR may further include at least one selected from the auxiliary emission layer EAL and the electron blocking layer EBL, in addition to the hole injection layer HIL and the hole transport layer HTL. The auxiliary emission layer EAL may compensate a resonance distance according to the wavelength of light emitted from the emission layer EML and regulate a hole charge balance to increase light emitting efficiency. In embodiments, the auxiliary emission layer EAL may serve to prevent or reduce injection of electrons into the hole transport region HTR. Materials which may be included in the hole transport region HTR may be included in the auxiliary emission layer EAL. The electron blocking layer EBL is a layer that serves to prevent or reduce injection of electrons from the electron transport region ETR to the hole transport region HTR.

In the light emitting element ED according to an embodiment, the emission layer EML is provided on the hole transport region HTR. The emission layer EML may have, for example, a thickness of about 100 Å to about 1000 Å, or about 100 Å to about 300 Å. The emission layer EML may have a single layer formed of a single material, a single layer formed of a plurality of different materials, or a multilayer structure having a plurality of layers formed of a plurality of different materials.

In the light emitting element ED according to an embodiment, the emission layer EML may emit blue light. The light emitting element ED according to an embodiment includes the amine compound according to an embodiment described above in the hole transport region HTR, and may thus exhibit high efficiency and long lifespan in a blue light emitting region. The light emitting element ED according to an embodiment may include the amine compound according to an embodiment described above in the hole transport region HTR, and the emission layer EML may emit blue fluorescence.

In embodiments, the emission layer EML of the light emitting element ED according to an embodiment may emit light in a wavelength range other than blue light. The light emitting element ED according to an embodiment includes the amine compound according to an embodiment described above in the hole transport region HTR, and may thus exhibit high efficiency and long lifespan in an emission region of a wavelength range other than blue. The light emitting element ED according to an embodiment may include the amine compound according to an embodiment described above in the hole transport region HTR, and the emission layer EML emit light of fluorescence. However, an embodiment is not limited thereto.

In the light emitting element ED according to an embodiment, the emission layer EML may include an anthracene derivative, a pyrene derivative, a fluoranthene derivative, a chrysene derivative, a dihydrobenzanthracene derivative, and/or a triphenylene derivative. For example, the emission layer EML may include an anthracene derivative and/or a pyrene derivative.

In the light emitting element ED according to an embodiment shown in FIGS. 5 to 9, the emission layer EML may include a host and a dopant, and the emission layer EML may include a compound represented by Formula E-1 below. The compound represented by Formula E-1 below may be used as a fluorescent host material.

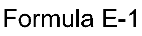

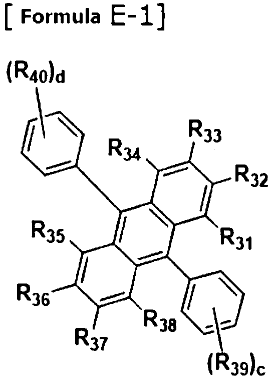

In Formula E-1, R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring. In embodiments, R₃₁ to R₄₀ may be bonded to an adjacent group to form a saturated hydrocarbon ring, an unsaturated hydrocarbon ring, a saturated heterocycle, or an unsaturated heterocycle.

In Formula E-1, c and d are each independently an integer of 0 to 5.

Formula E-1 may be represented by any one selected from compounds E1 to E19 below.

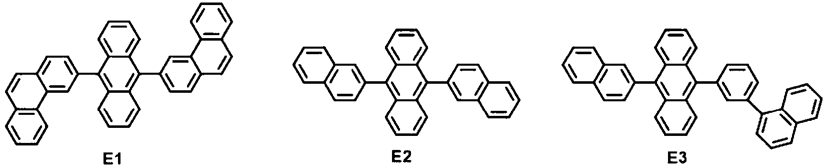

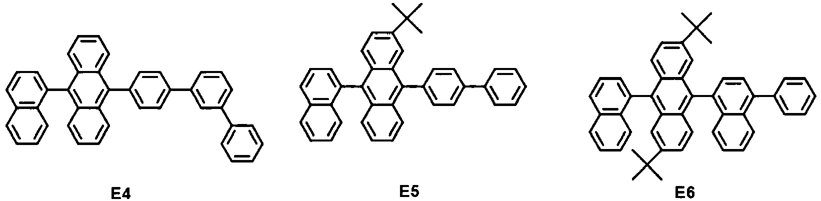

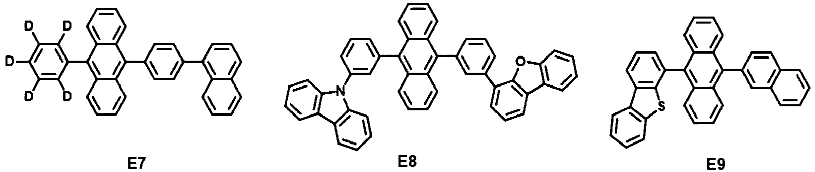

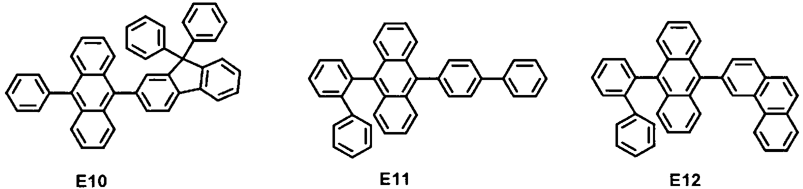

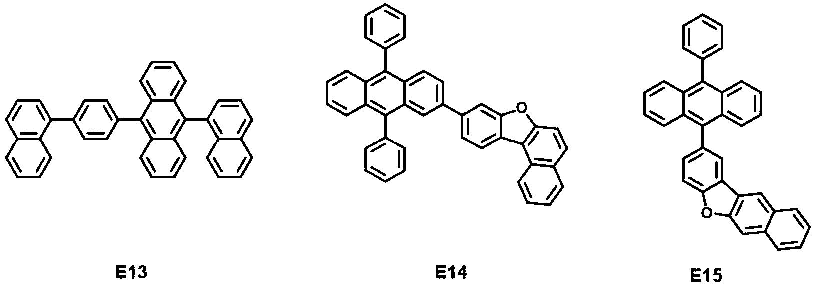

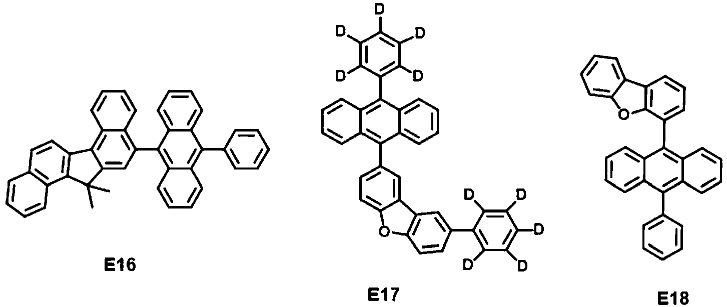

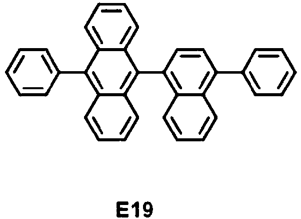

In an embodiment, the emission layer EML may further include a compound represented by Formula E-2a or Formula E-2b below. The compound represented by Formula E-2a or Formula E-2b may be used as a phosphorescent host material.

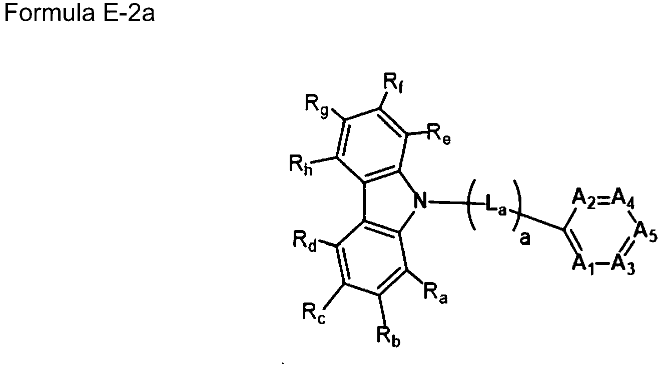

In Formula E-2a, a may be an integer of 0 to 10, and Lₐ may be a direct linkage, a substituted or unsubstituted arylene group having 6 to 30 ring-forming carbon atoms, or a substituted or unsubstituted heteroarylene group having 2 to 30 ring-forming carbon atoms. If (e.g., when) a is an integer of 2 or greater, a plurality of Lₐ's may each independently be a substituted or unsubstituted arylene group having 6 to 30 ring-forming carbon atoms, or a substituted or unsubstituted heteroarylene group having 2 to 30 ring-forming carbon atoms.

In embodiments, in Formula E-2a, A₁ to A₅ may each independently be N or CRᵢ. Rₐ to Rᵢ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring. Rₐ to Rᵢ may be bonded to an adjacent group to form a hydrocarbon ring or a heterocycle containing N, O, S, and/or the like as a ring-forming atom.

In embodiments, in Formula E-2a, two or three selected from A₁ to A₅ may be N, and the others may be CRᵢ.

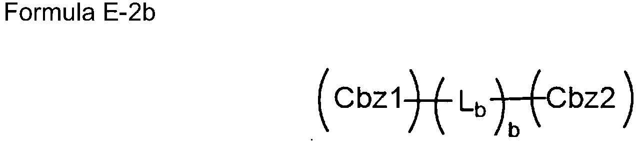

In Formula E-2b, Cbz1 and Cbz2 may each independently be an unsubstituted carbazole group or an aryl-substituted carbazole group having 6 to 30 ring-forming carbon atoms. L_{b} may be a direct linkage, a substituted or unsubstituted arylene group having 6 to 30 ring-forming carbon atoms, or a substituted or unsubstituted heteroarylene group having 2 to 30 ring-forming carbon atoms, b may be an integer of 0 to 10, and if (e.g., when) b is an integer of 2 or greater, a plurality of L_{b}'s may each independently be a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one selected from compounds from Compound Group E-2 below. However, the compounds listed in Compound Group E-2 below are presented as an example, and the compound represented by Formula E-2a or Formula E-2b is not limited to those listed in Compound Group E-2 below.

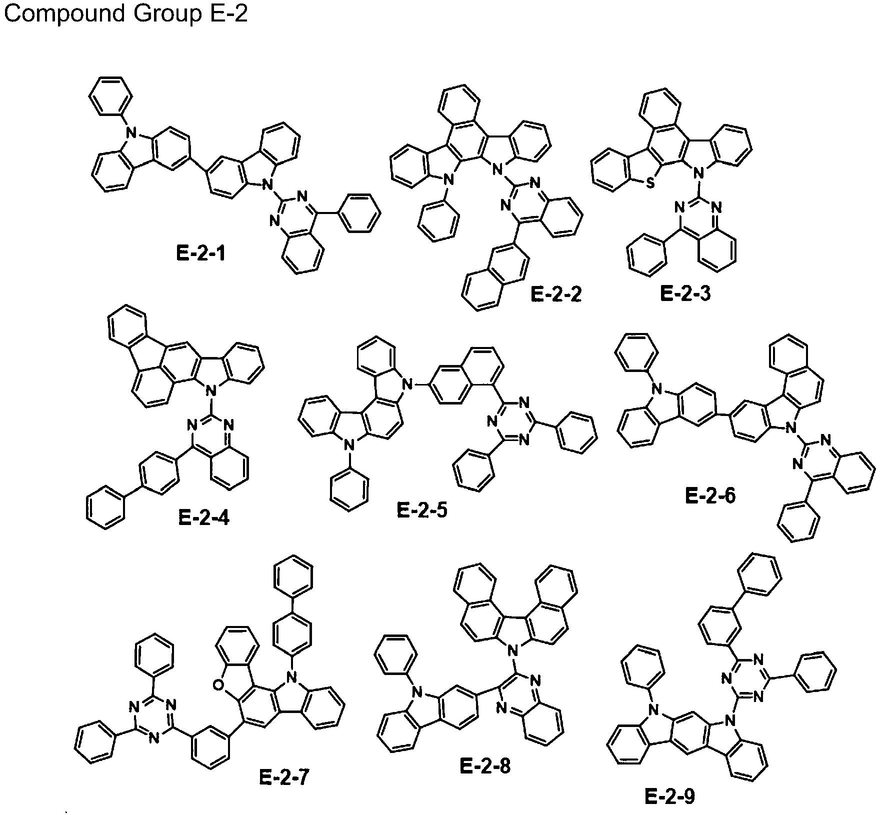

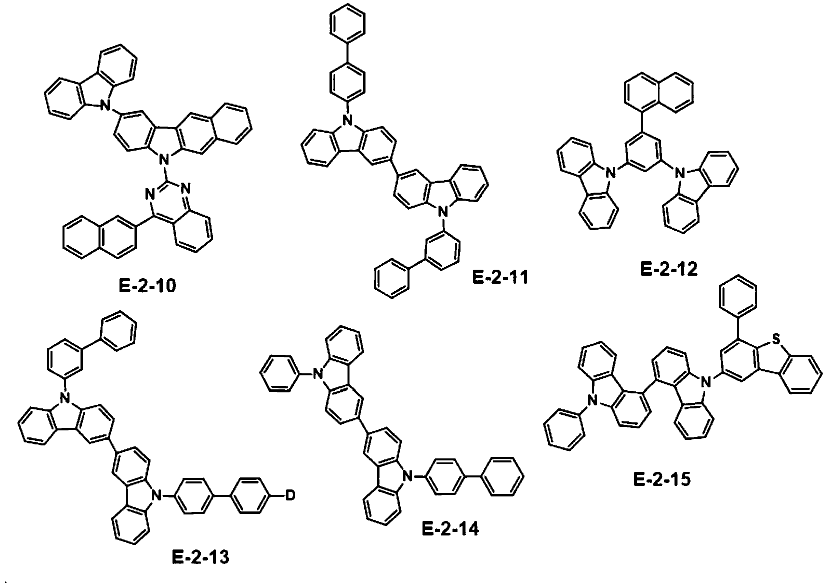

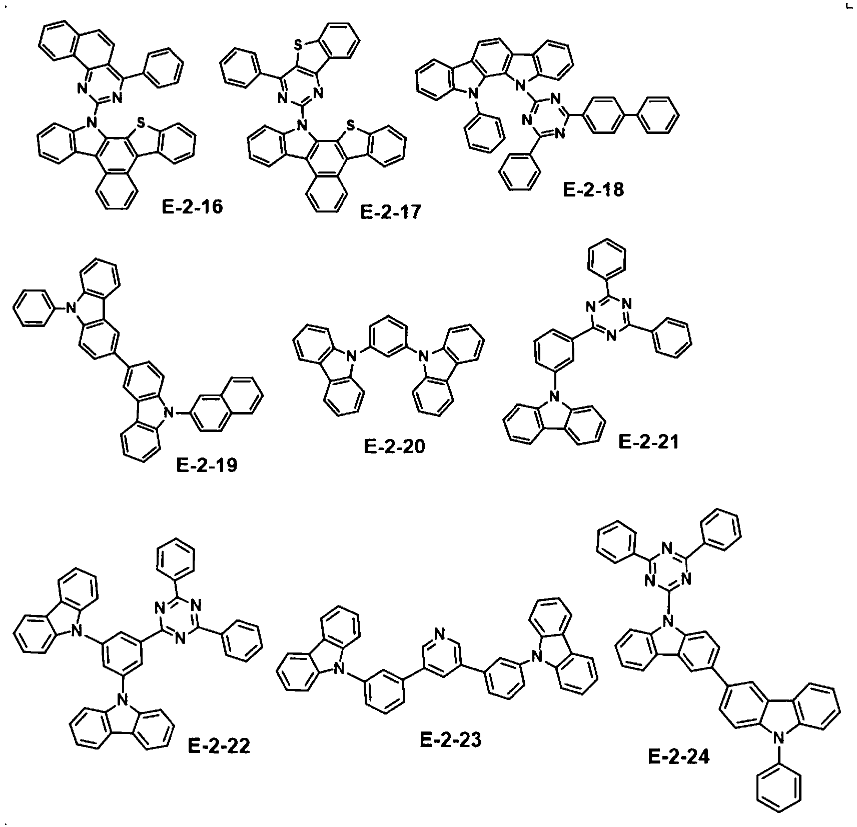

The emission layer EML may further include any suitable material generally used in the art as a host material. For example, the emission layer EML may include, as a host material, at least one selected from bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1'-biphenyl (CBP), 1,3-bis(carbazolyl-9-yl)benzene (mCP), 2,8-bis(diphenylphosphoryl)dibenzofuran (PPF), 4,4',4"-tris(carbazol-9-yl)-triphenylamine (TCTA), and 1,3,5-tris(1-phenyl-1H-benzo[d]imidazol-2-yl)benzene (TPBi). However, an embodiment is not limited thereto, and for example, tris(8-hydroxyquinolino)aluminium (Alq₃), 9,10-di(naphthalene-2-yl)anthracene (ADN), 3-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetrasiloxane (DPSiO₄), and/or the like may be used as a host material.

The emission layer EML may include a compound represented by Formula M-a or Formula M-b below. The compound represented by Formula M-a or Formula M-b below may be used as a phosphorescent dopant material.

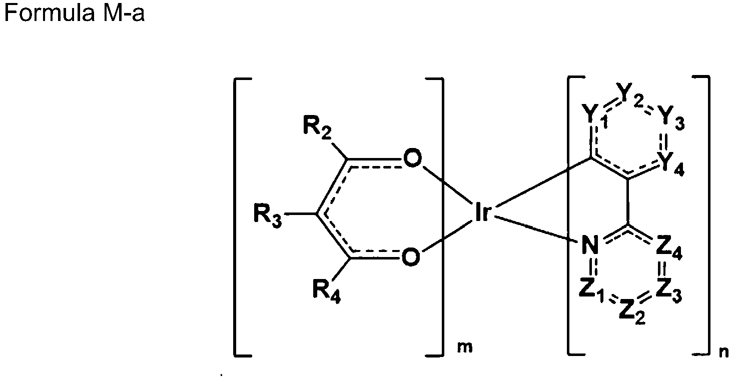

In Formula M-a above, Y₁ to Y₄ and Z₁ to Z₄ may each independently be CR₁ or N, and R₁ to R₄ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring. In Formula M-a, m is 0 or 1, and n is 2 or 3. In Formula M-a, if (e.g., when) m is 0, n is 3, and if (e.g., when) m is 1, n is 2.

The compound represented by Formula M-a may be represented by any one selected from compounds M-a1 to M-a25 below. However, the compounds Ma1 to M-a25 below are presented as an example, and the compound represented by Formula M-a is not limited to those represented by the compounds M-a1 to M-a25 below.

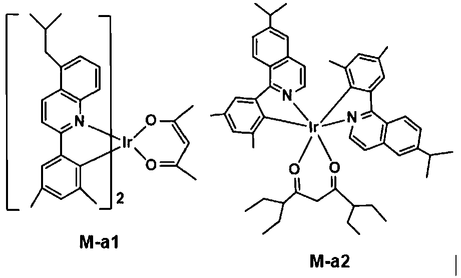

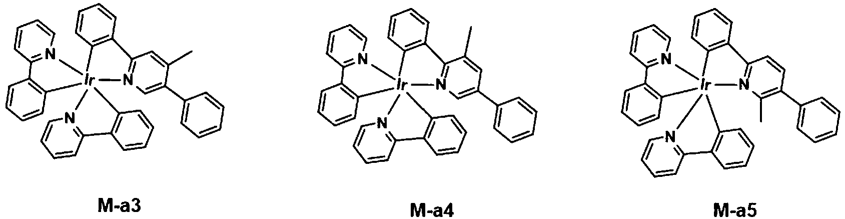

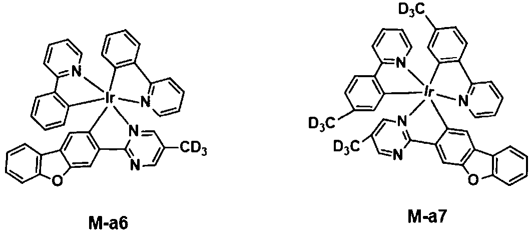

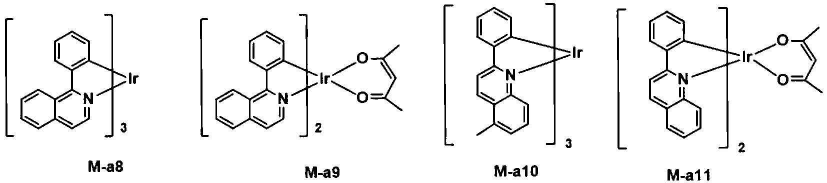

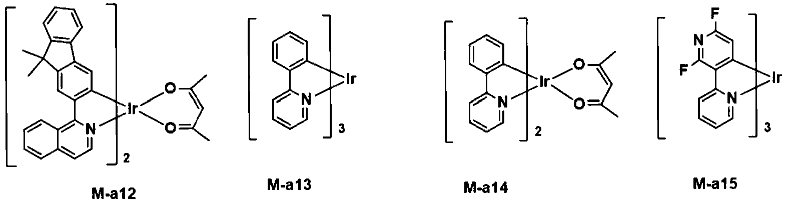

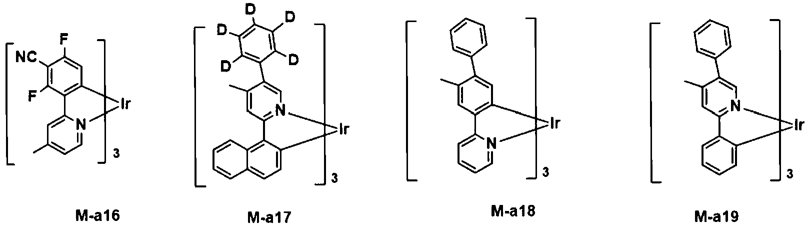

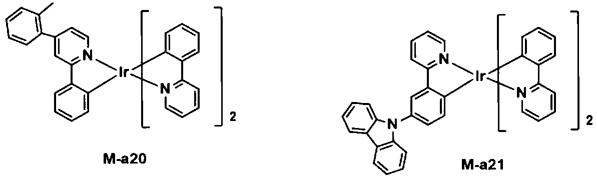

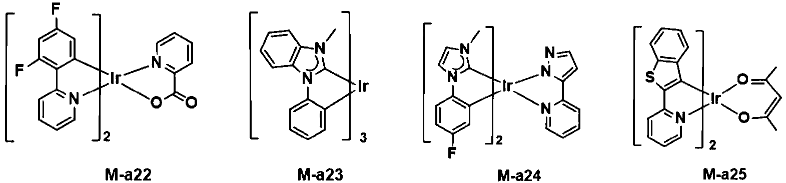

The compounds M-a1 and M-a2 may be used as a red dopant material, and the compounds M-a3 to M-a5 may be used as a green dopant material.

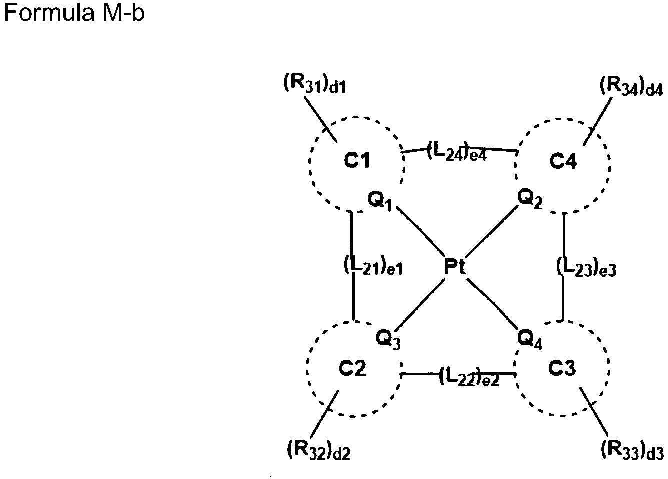

In Formula M-b, Q1 to Q4 are each independently C or N, and C1 to C4 are each independently a substituted or unsubstituted hydrocarbon ring having 5 to 30 ring-forming carbon atoms, or a substituted or unsubstituted heterocycle having 2 to 30 ring-forming carbon atoms. L21 to L24 are each independently a direct linkage, 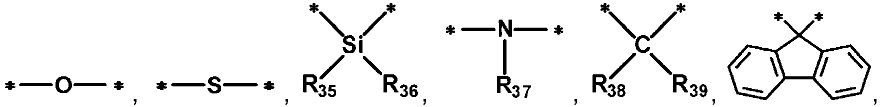 a substituted or unsubstituted divalent alkyl group having 1 to 20 carbon atoms, a substituted or unsubstituted arylene group having 6 to 30 ring-forming carbon atoms, or a substituted or unsubstituted heteroarylene group having 2 to 30 ring-forming carbon atoms, and e1 to e4 are each independently 0 or 1. R₃₁ to R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring, and d1 to d4 are each independently an integer of 0 to 4.

A compound represented by Formula M-b may be used as a blue phosphorescent dopant or a green phosphorescent dopant.

The compound represented by Formula M-b may be represented by any one selected from compounds below. However, the compounds below are presented as an example, and the compound represented by Formula M-b is not limited to those represented by the compounds below.

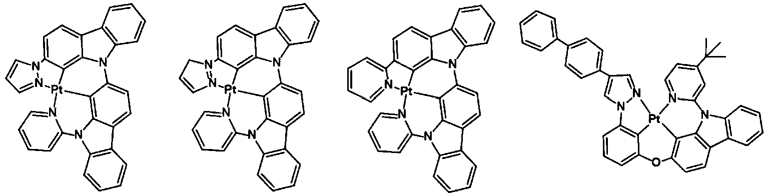

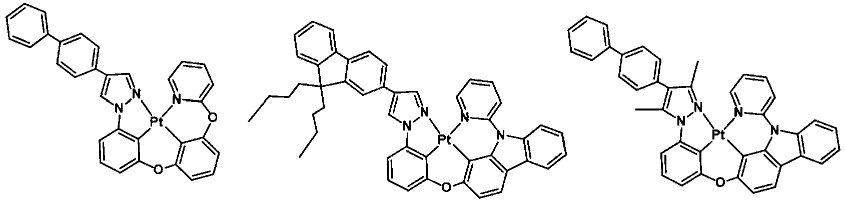

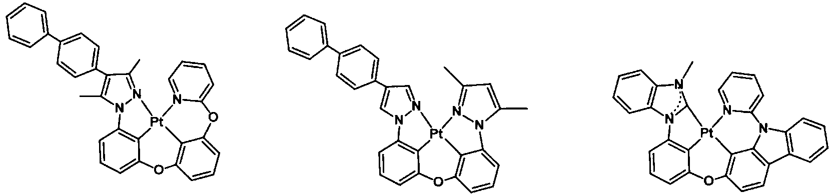

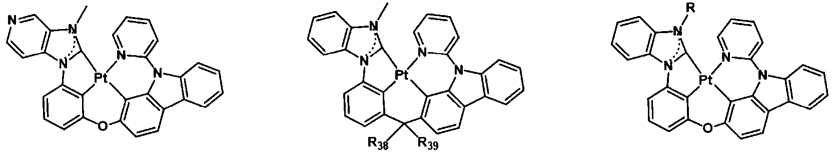

In the compounds above, R, R₃₈, and R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

The emission layer EML may include a compound represented by any one selected from Formulas F-a to F-c below. The compounds represented by Formulas F-a to F-c below may be used as a fluorescence dopant material.

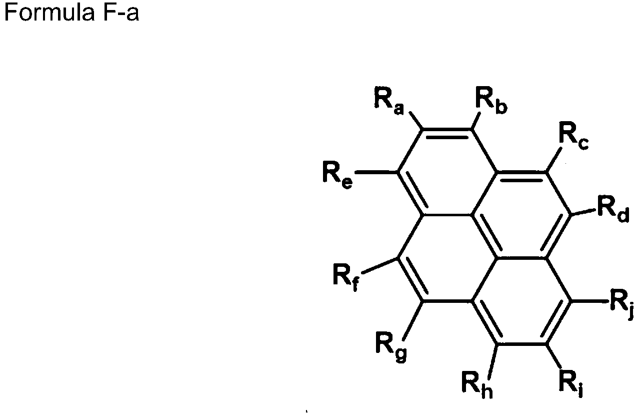

In Formula F-a, two selected from Rₐ to Rⱼ may each independently be substituted with *-NAr₁Ar₂. The others among Rₐ to Rⱼ which are not substituted with *-NAr₁Ar₂ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In *-NAr₁Ar₂, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. For example, at least one selected from Ar₁ and Ar₂ may be a heteroaryl group containing O and/or S as a ring-forming atom.

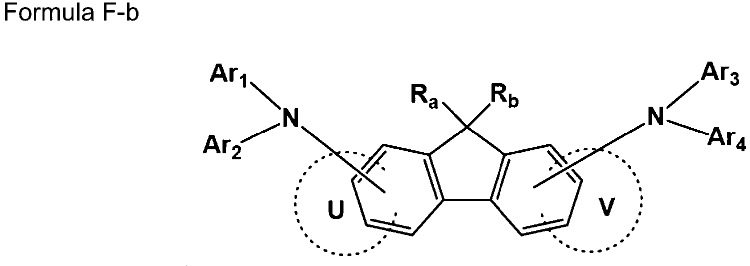

In Formula F-b above, Rₐ and R_{b} may each independently be a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, a substituted or unsubstituted heteroaryl group having 2 to 30 ring-forming carbon atoms, or bonded to an adjacent group to form a ring. Ar₁ to Ar₄ may each independently be a substituted or unsubstituted aryl group having 6 to 30 ring-forming carbon atoms, or a substituted or unsubstituted heteroaryl group having 2 to 30 ring-forming carbon atoms.

In Formula F-b, U and V may each independently be a substituted or unsubstituted hydrocarbon ring having 5 to 30 ring-forming carbon atoms, or a substituted or unsubstituted heterocycle having 2 to 30 ring-forming carbon atoms. At least one selected from Ar₁ to Ar₄ may be a heteroaryl group containing O and/or S as a ring-forming atom.

In Formula F-b, the number of rings represented by U and V may each independently be 0 or 1. For example, in Formula F-b, if (e.g., when) the number of U or V is 1, one ring forms a fused ring in a portion indicated by U or V, and if (e.g., when) the number of U or V is 0, it means that no ring indicated by U or V is present. For example, if (e.g., when) the number of U is 0 and the number of V is 1, or if (e.g., when) the number of U is 1 and the number of V is 0, a fused ring having a fluorene core of Formula F-b may be a cyclic compound having four rings. In embodiments, if (e.g., when) the number of U and V are both 0, the fused ring of Formula F-b may be a cyclic compound having three rings. In embodiments, if (e.g., when) the number of U and V are both 1, the fused ring having a fluorene core of Formula F-b may be a cyclic compound having five rings.

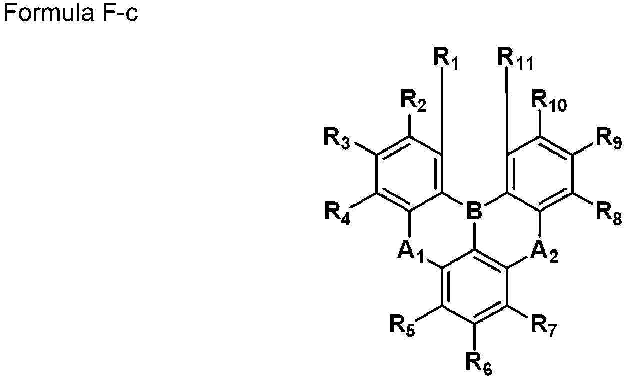

In Formula F-c, A₁ and A₂ may each independently be O, S, Se, or NRₘ, and Rₘ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ are each independently a hydrogen atom, a deuterium atom, a halogen atom, a cyano group, a substituted or unsubstituted amine group, a substituted or unsubstituted boron group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring.

In Formula F-c, A₁ and A₂ may each independently be bonded to substituents of neighboring rings to form a fused ring. For example, if (e.g., when) A₁ and A₂ are each independently NRₘ, A₁ may be bonded to R₄ or R₅ to form a ring. In embodiments, A₂ may be bonded to R₇ or R₈ to form a ring.

In an embodiment, the emission layer EML may further include, as a dopant material, styryl derivatives (e.g., 1,4-bis[2-(3-N-ethylcarbazoryl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi), 4,4'-bis[2-(4-(N,N-diphenylamino)phenyl)vinyl]biphenyl (DPAVBi), perylene and derivatives thereof (e.g., 2,5,8,11-tetra-t-butylperylene (TBP)), pyrene and derivatives thereof (e.g., 1,1-dipyrene, 1,4-dipyrenylbenzene, 1,4-bis(N,N-diphenylamino)pyrene), and/or the like.

If (e.g., when) the light emitting element is a phosphorescent light emitting element, the emission layer EML may further include any suitable dopant material generally used in the art. For example, as a phosphorescent dopant, a metal complex including iridium (Ir), platinum (Pt), osmium (Os), gold (Au), titanium (Ti), zirconium (Zr), hafnium (Hf), europium (Eu), and terbium (Tb), and/or thulium (Tm) may be used. For example, iridium(III) bis(4,6-difluorophenylpyridinato-C2,N) (Flrpic), bis(2,4-difluorophenylpyridinato)-tetrakis(1-pyrazolyl)borate iridium(III) (Fir6), and/or platinum octaethyl porphyrin (PtOEP) may be used as a dopant for a phosphorescent light emitting element. However, an embodiment is not limited thereto.

In the light emitting element ED according to an embodiment, the emission layer EML may be a delayed fluorescence emission layer including a host and a dopant. For example, the emission layer EML may emit light of thermally activated delayed fluorescence (TADF). In the light emitting element ED according to an embodiment, the emission layer EML may include any suitable thermally activated delayed fluorescence dopant generally used in the art.

In an embodiment, the emission layer EML of the light emitting element ED may include a plurality of host materials, a thermally activated delayed fluorescence dopant, and a phosphorescent sensor.

The emission layer EML may include a quantum dot material. The core of a quantum dot may be selected from a Group II-VI compound, a Group I-II-VI compound, a Group II-IV-VI compound, a Group I-II-IV-VI compound, a Group II-IV-V compound, a Group III-VI compound, a Group I-III-VI element, a Group III-V compound, a Group III-II-V compound, a Group IV-VI compound, a Group IV element, a Group IV compound, and a combination thereof.
The Group II-VI compound may be selected from the group consisting of a binary compound selected from the group consisting of CdSe, CdTe, CdS, ZnS, ZnSe, ZnTe, ZnO, HgS, HgSe, HgTe, MgSe, MgS, and a mixture thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, and a mixture thereof. The II-VI group compound may further include a group I metal and/or a group IV element. The I-II-VI group compound may be selected from CuZnS or the like, and the II-IV-VI group compound may be selected from ZnSnS or the like. The I-II-IV-VI group compound may be selected from a quaternary compound selected from the group consisting of Cu₂ZnSnS₂, Cu₂ZnSnS₄, Cu₂ZnSnSe₄, Ag₂ZnSnS₂ and a mixture thereof.

Examples of the II-IV-V group compound may be a ternary compound selected from the group consisting of ZnSnP, ZnSnP₂, ZnSnAs₂, ZnGeP₂, ZnGeAs₂, CdSnP₂, CdGeP₂ and a mixture thereof.

The Group III-VI compound may include a binary compound such as In₂S₃ and/or In₂Se₃, a ternary compound such as InGaS₃ and/or InGaSe₃, or any combination thereof.

The Group I-III-VI compound may be selected from a ternary compound selected from the group consisting of AgInS, AgInS₂, CulnS, CuInS₂, AgGaS₂, CuGaS₂ CuGaO₂, AgGaO₂, AgAlO₂, or a mixture thereof, or a quaternary compound such as AgInGaS₂ and CuInGaS₂.

The Group III-V compound may be selected from the group consisting of a binary compound selected from the group consisting of GaN, GaP, GaAs, GaSb, AIN, AlP, AlAs, AlSb, InN, InP, InAs, InSb, and a mixture thereof, a ternary compound selected from the group consisting of GaNP, GaNAs, GaNSb, GaPAs, GaPSb, AINP, AlNAs, AlNSb, AlPAs, AlPSb, InGaP, InAlP, InNP, InNAs, InNSb, InPAs, InPSb, and a mixture thereof, and a quaternary compound selected from the group consisting of GaAlNP, GaAINAs, GaAINSb, GaAlPAs, GaAlPSb, GalnNP, GalnNAs, GalnNSb, GalnPAs, GalnPSb, InAlNP, InAINAs, InAlNSb, InAlPAs, InAlPSb, and a mixture thereof. In embodiments, the Group III-V compound may further include a Group II metal. For example, InZnP and/or the like may be selected as a Group III-II-V compound.

The Group IV-VI compound may be selected from the group consisting of a binary compound selected from the group consisting of SnS, SnSe, SnTe, PbS, PbSe, PbTe, and a mixture thereof, a ternary compound selected from the group consisting of SnSeS, SnSeTe, SnSTe, PbSeS, PbSeTe, PbSTe, SnPbS, SnPbSe, SnPbTe, and a mixture thereof, and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

Each element included in the multi-element compound such as the binary compound, ternary compound, and quaternary compound may be present in particles at a uniform concentration or a non-uniform concentration. In embodiments, Formula above indicates the types (or kinds) of elements included in a compound, and element ratios in the compound may be different. For example, AglnGaS₂ may indicate AglnₓGa₁₋ₓS₂ (x is a real number between 0 and 1).

In embodiments, the quantum dot may have a single structure having a uniform (e.g., substantially uniform) concentration of each element included in the corresponding quantum dot or a dual structure of core-shell. For example, materials included in the core may be different from materials included in the shell.

The shell of the quantum dot may serve as a protection layer to prevent or reduce the chemical deformation of the core so as to keep semiconductor properties, and/or a charging layer to impart electrophoresis properties to the quantum dot. The shell may be single-layered or multi-layered. An interface between the core and the shell may have a concentration gradient in which the concentration of an element present in the shell becomes lower along a direction towards the center of the core.

Examples of the shell of the quantum dot may be a metal and/or non-metal oxide, a semiconductor compound, or a combination thereof. For example, the metal and/or non-metal oxide may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO, and/or a ternary compound such as MgAl₂O₄, CoFe₂O₄, NiFe₂O₄, and/or CoMn₂O₄, but the present disclosure is not limited thereto.

In embodiments, the semiconductor compound may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and/or the like, but the present disclosure is not limited thereto.

Each element included in the multi-element compound such as the binary compound and the ternary compound may be present in particles at a uniform concentration or a non-uniform concentration. In embodiments, Formula above indicates the types (or kinds) of elements included in a compound, and element ratios in the compound may be different.

The quantum dot may have, in a light emitting wavelength spectrum, a full width of half maximum (FWHM) of about 45 nm or less, for example, about 40 nm or less, or about 30 nm or less, and in this range, color purity and/or color reproducibility may be improved. In embodiments, light emitted through the quantum dot is emitted in all directions, and thus a wide viewing angle may be improved.

In embodiments, the form of the quantum dot is not particularly limited as long as it is a form generally used in the art, but, for example, a quantum dot in the form of spherical, pyramidal, multi-arm, and/or cubic nanoparticles, nanotubes, nanowires, nanofibers, nanoplatelets, and/or the like may be used.

As the size of the quantum dot or the ratio of elements in the quantum dot compound is regulated, the energy band gap may be accordingly controlled to obtain light of various wavelengths from the quantum dot emission layer. Therefore, by using the quantum dots as described above (using quantum dots of different sizes or having different element ratios in the quantum dot compound), a light emitting element emitting light of various suitable wavelengths may be obtained. In embodiments, the size of the quantum dot or the ratio of elements in the quantum dot compound may be regulated to emit red, green, and/or blue light. In embodiments, the quantum dots may be configured to emit white light by combining light of various suitable colors.

In the light emitting element ED according to an embodiment shown in FIGS. 5 to 9, an electron transport region ETR is provided on the emission layer EML. The electron transport region ETR may include at least one among a hole blocking layer HBL, an electron transport layer ETL, and an electron injection layer EIL, but an embodiment is not limited thereto.

The electron transport region ETR may have a single layer formed of a single material, a single layer formed of a plurality of different materials, or a multilayer structure having a plurality of layers formed of a plurality of different materials.

For example, the electron transport region ETR may have a single layer structure of an electron injection layer EIL or an electron transport layer ETL, and may have a single layer structure formed of an electron injection material and an electron transport material. In embodiments, the electron transport region ETR may have a single layer structure formed of a plurality of different materials, or may have a structure in which an electron transport layer ETL/electron injection layer EIL, or a hole blocking layer HBL/electron transport layer ETL/electron injection layer EIL are stacked in order from the emission layer EML, but is not limited thereto. The electron transport region ETR may have a thickness of, for example, about 1000 Å to about 1500 Å.

The electron transport region ETR may be formed using various suitable methods such as a vacuum deposition method, a spin coating method, a cast method, a Langmuir-Blodgett (LB) method, an inkjet printing method, a laser printing method, and/or a laser induced thermal imaging (LITI) method.

The electron transport region ETR may include a compound represented by Formula ET-2 below.

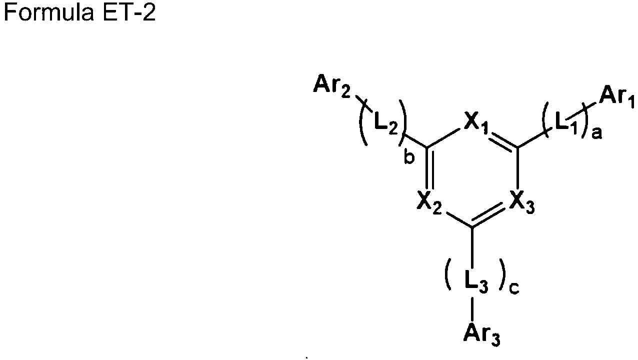

In Formula ET-2, at least one selected from X₁ to X₃ is N and the others are CRₐ. Rₐ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ may each independently be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-2, a to c may each independently be an integer of 0 to 10. In Formula ET-2, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. If (e.g., when) a to c are an integer of 2 or greater, L₁ to L₃ may each independently be a substituted or unsubstituted arylene group having 6 to 30 ring-forming carbon atoms, or a substituted or unsubstituted heteroarylene group having 2 to 30 ring-forming carbon atoms.

The electron transport region ETR may include an anthracene-based compound. However, an embodiment is not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazolyl-1-ylphenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminium (BAlq), berylliumbis(benzoquinolin-10-olate (Bebq₂), 9,10-di(naphthalene-2-yl)anthracene (ADN), 1,3-bis[3,5-di(pyridin-3-yl)phenyl]benzene (BmPyPhB), or a mixture thereof.

The electron transport region ETR may include at least one selected from compounds ET1 to ET36 below.

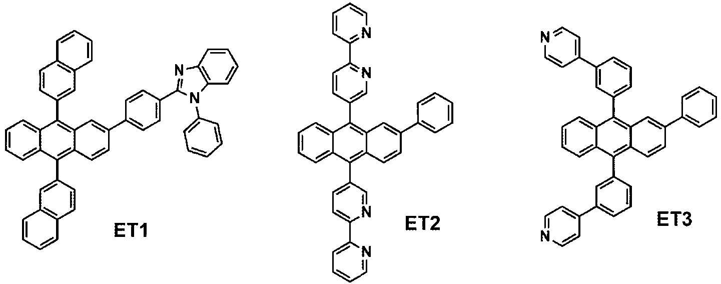

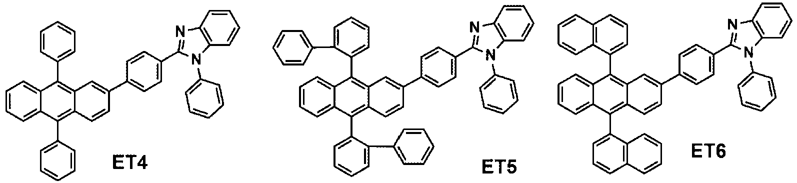

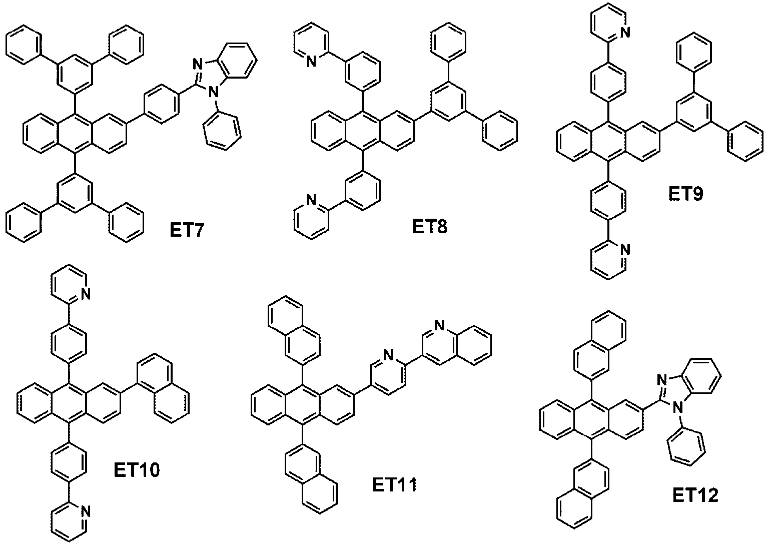

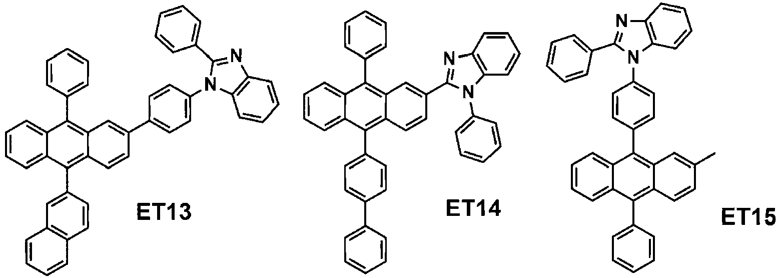

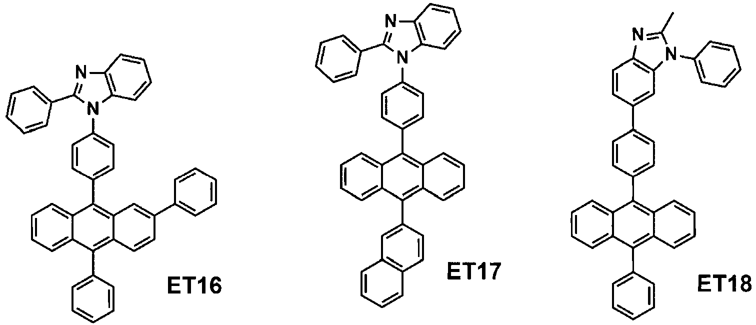

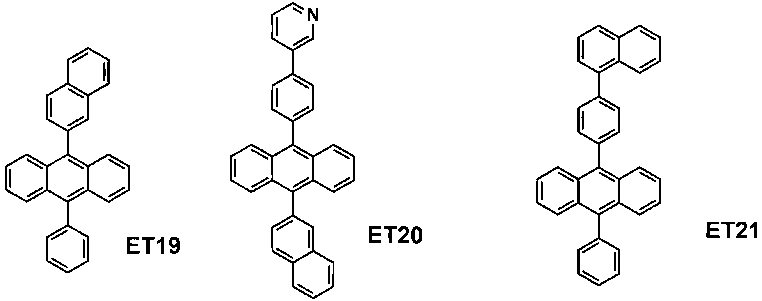

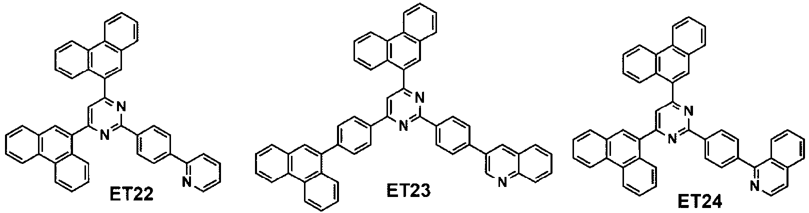

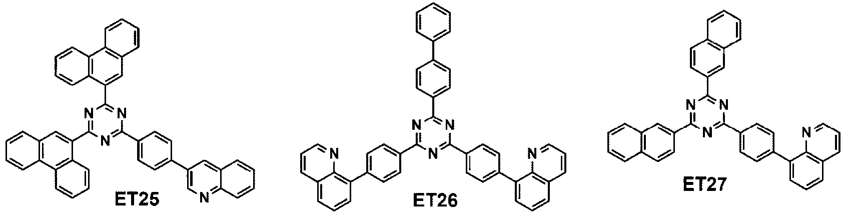

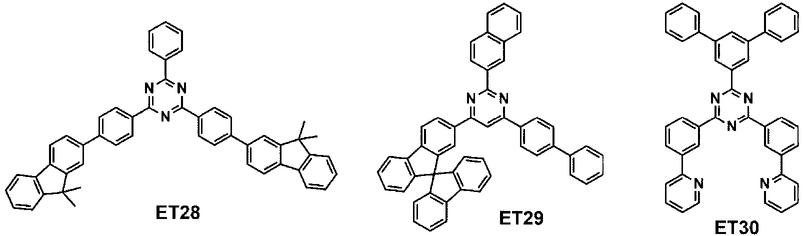

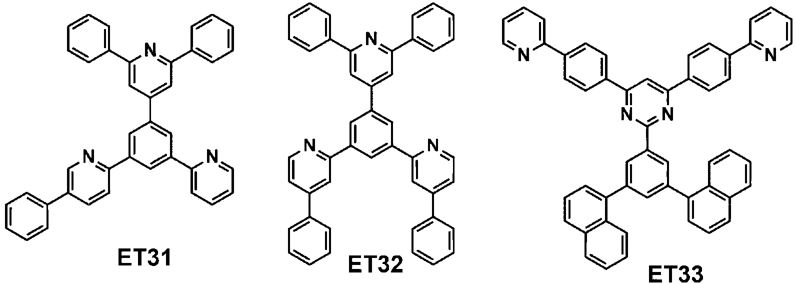

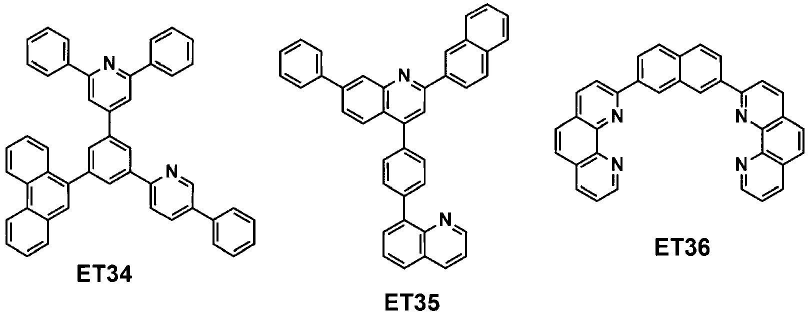

In embodiments, the electron transport region ETR may include halogenated metals such as LiF, NaCl, CsF, RbCl, Rbl, Cul, and/or Kl, lanthanide metals such as Yb, and/or co-deposition materials of a halogenated metal and a lanthanide metal. For example, the electron transport region ETR may include KI:Yb, Rbl:Yb, LiF:Yb, and/or the like as a co-deposition material. In embodiments, for the electron transport region ETR, a metal oxide such as Li₂O and/or BaO, 8-hydroxyl-lithium quinolate (Liq), and/or the like may be used, but an embodiment is limited thereto. The electron transport region ETR may also be formed of a mixture material of an electron transport material and an insulating organo-metal salt (e.g., an electrically insulating organo-metal salt). The organo metal salt may be a material having an energy band gap of about 4 eV or greater. In embodiments, the organo-metal salt may include, for example, metal acetates, metal benzoates, metal acetoacetates, metal acetylacetonates, and/or metal stearates.

The electron transport region ETR may further include, for example, at least one selected from 2,9-dimethyl-4,7-diphenyl-1,10-phenanthroline (BCP), diphenyl(4-(triphenylsilyl)phenyl)phosphine oxide (TSPO1), and 4,7-diphenyl-1,10-phenanthroline (Bphen) in addition to the materials described above, but an embodiment is not limited thereto.

The electron transport region ETR may include the compounds of the electron transport region described above in at least one among the electron injection layer EIL, the electron transport layer ETL, and the hole blocking layer HBL.

If (e.g., when) the electron transport region ETR includes the electron transport layer ETL, the electron transport layer ETL may have a thickness of about 100 A to about 1000 Å, for example, about 150 A to about 500 Å. If (e.g., when) the thickness of the electron transport layer ETL satisfies the above-described range, suitable or satisfactory electron transport properties may be obtained without a substantial increase in driving voltage. If (e.g., when) the electron transport region ETR includes the electron injection layer EIL, the electron injection layer EIL may have a thickness of about 1 Å to about 100 Å, for example, about 3 Å to about 90 Å. If (e.g., when) the thickness of the electron injection layer EIL satisfies the above-described ranges, suitable or satisfactory electron injection properties may be obtained without a substantial increase in driving voltage.

The second electrode EL2 is provided on the electron transport region ETR. The second electrode EL2 may be a common electrode. The second electrode EL2 may be a cathode or an anode but an embodiment is not limited thereto. For example, if (e.g., when) the first electrode EL1 is an anode, the second electrode EL2 may be a cathode, and if (e.g., when) the first electrode EL1 is a cathode, the second electrode EL2 may be an anode.

The second electrode EL2 may be a transmissive electrode, a transflective electrode, or a reflective electrode. If (e.g., when) the second electrode EL2 is a transmissive electrode, the second electrode EL2 may be formed of a transparent metal oxide, for example, indium tin oxide (ITO), indium zinc oxide (IZO), zinc oxide (ZnO), indium tin zinc oxide (ITZO), and/or the like.

If (e.g., when) the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, a compound thereof, or a mixture thereof (e.g., AgMg, AgYb, or MgYb). In embodiments, the second electrode EL2 may have a multilayer structure including a reflective film or a transflective film formed of the above-described materials, and a transparent conductive film (e.g., a transparent electrically conductive film) formed of indium tin oxide (ITO), indium zinc oxide (IZO), zinc oxide (ZnO), indium tin zinc oxide (ITZO), and/or the like. For example, the second electrode EL2 may include the above-described metal materials, a combination of two or more metal materials selected from the above-described metal materials, and/or oxides of the above-described metal materials.

In embodiments, the second electrode EL2 may be connected with an auxiliary electrode. If (e.g., when) the second electrode EL2 is connected with the auxiliary electrode, the resistance of the second electrode EL2 may decrease.

In embodiments, a capping layer CPL may be further on the second electrode EL2 of the light emitting element ED according to an embodiment. The capping layer CPL may include a multi-layer or a single layer.

In an embodiment, the capping layer CPL may be an organic layer and/or an inorganic layer. For example, if (e.g., when) the capping layer CPL includes an inorganic material, the inorganic material may include an alkali metal compound such as LiF, an alkaline earth metal compound such as MgF₂, SiON, SiN_{X}, SiOy, and/or the like.

For example, if (e.g., when) the capping layer CPL includes an organic material, the organic material may include α-NPD, NPB, TPD, m-MTDATA, Alq₃ CuPc, N4,N4,N4',N4'-tetra(biphenyl-4-yl) biphenyl-4,4'-diamine (TPD15), 4,4',4"-tris(carbazol sol-9-yl)triphenylamine (TCTA), and/or the like, and/or may include epoxy resins and/or acrylates such as methacrylates. However, an embodiment is not limited thereto, and the capping layer CPL may include at least one selected from compounds P1 to P5 below.

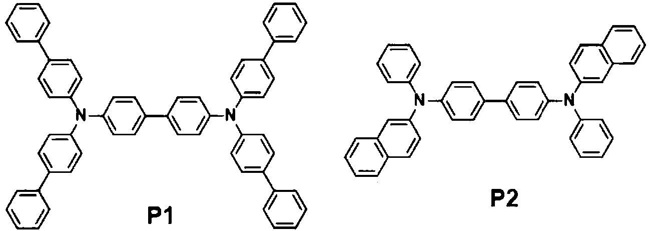

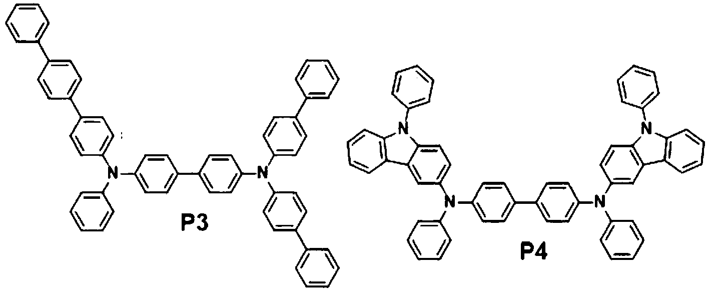

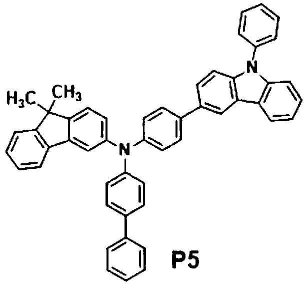

In embodiments, the capping layer CPL may have a refractive index of about 1.6 or greater. For example, the capping layer CPL may have a refractive index of about 1.6 or greater in a wavelength range of about 550 nm to about 660 nm.

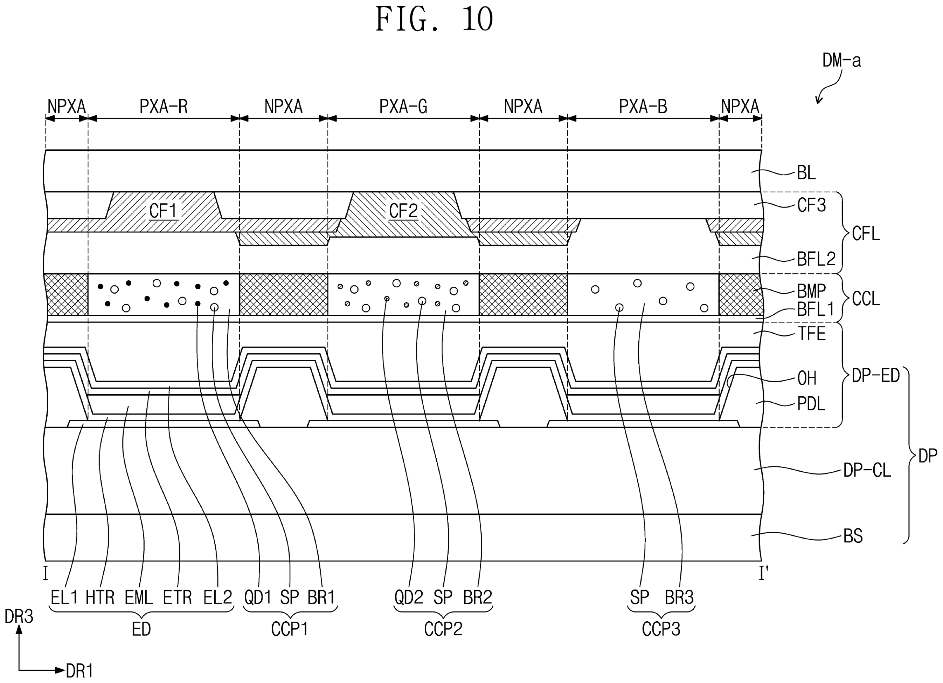

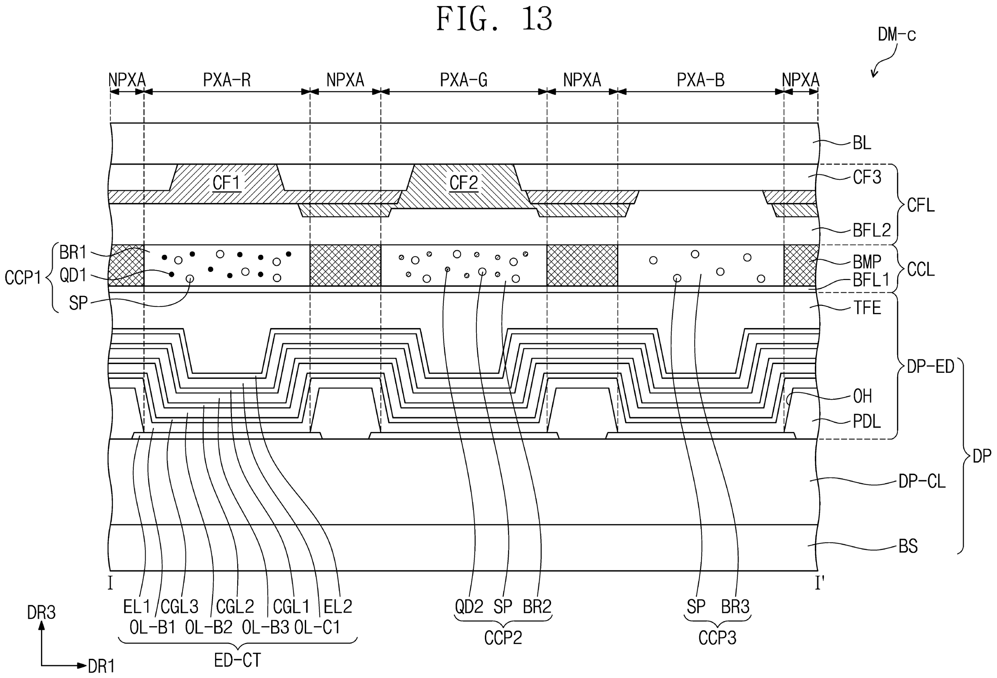

FIGS. 10 to 13 are each a cross-sectional view of a display module according to an embodiment. Hereinafter, in the description of the display module according to an embodiment with reference to FIGS. 10 and 13, content overlapping the one described above with reference to FIGS. 1 to 9 may not be described again, and the differences will be mainly described.

Referring to FIG. 10, a display module DM-a according to an embodiment may include a display panel DP having a display element layer DP-ED, a light control layer CCL on the display panel DP, and a color filter layer CFL. In an embodiment shown in FIG. 10, the display panel DP may include a base layer BS, a circuit layer DP-CL provided on the base layer BS, and a display element layer DP-ED, and the element layer DP-ED may include a light emitting element ED.

The light emitting element ED may include a first electrode EL1, a hole transport region HTR on the first electrode EL1, an emission layer EML on the hole transport region HTR, an electron transport region ETR on the emission layer EML, and a second electrode EL2 on the electron transport region ETR. A structure of the light emitting element ED shown in FIG. 10 may be the same as the structure of the light emitting element of FIGS. 5 to 9 described above. The light emitting element ED shown in FIG. 10 includes the amine compound according to an embodiment. The light emitting element ED according to an embodiment includes the amine compound according to an embodiment in the hole transport region HTR, and may thus exhibit long lifespan characteristics. In embodiments, the light emitting element ED according to an embodiment may emit blue fluorescence and exhibit long lifespan characteristics.

Referring to FIG. 10, the emission layer EML may be provided in the openings OH defined in the pixel defining films PDL. For example, the emission layer EML separated by the pixel defining films PDL and provided corresponding to each of light emitting regions PXA-R, PXA-G, and PXA-B may emit light in the same wavelength ranges. In the display module DM-a according to an embodiment, the emission layer EML may emit blue light. In an embodiment, the emission layer EML may be provided as a common layer throughout the light emitting regions PXA-R, PXA-G, and PXA-B.

The light control layer CCL may be on the display panel DP. Although the light control layer CCL is shown to be on an upper side of the display element layer DP-ED, an embodiment is not limited thereto, and the light control layer CCL may be on a lower side of the display element layer DP-ED. The light control layer CCL may include a light converter. The light converter may be a quantum dot and/or a phosphor. The light converter may wavelength-convert the provided light and emit the wavelength-converted light. In embodiments, the light control layer CCL may be a layer containing quantum dots and/or phosphors.

The light control layer CCL may include a plurality of light control units CCP1, CCP2, and CCP3. The light control units CCP1, CCP2, and CCP3 may be spaced apart from one another.

Referring to FIG. 10, a division pattern BMP may be between the light control units CCP1, CCP2, and CCP3 spaced apart from each other, but an embodiment is not limited thereto. In FIG. 10, the division pattern BMP is shown to nonoverlap the light control units CCP1, CCP2, and CCP3, but edges of the light control units CCP1, CCP2, and CCP3 may overlap at least a portion of the division pattern BMP.

The light control layer CCL may include a first light control unit CCP1 including a first quantum dot QD1 for converting first color light provided from the light emitting element ED into second color light, a second light control unit CCP2 including a second quantum dot QD2 for converting the first color light into third color light, and a third light control unit CCP3 transmitting the first color light.

In an embodiment, the first light control unit CCP1 may provide red light, which is the second color light, and the second light control unit CCP2 may provide green light, which is the third color light. The third light control unit CCP3 may transmit and provide blue light, which is the first color light provided from the light emitting element ED. For example, the first quantum dot QD1 may be a red quantum dot and the second quantum dot QD2 may be a green quantum dot. The same descriptions above may be applied to the quantum dots QD1 and QD2.

In embodiments, the light control layer CCL may further include scatterers SP (e.g., light scatterers SP). The first light control unit CCP1 may include the first quantum dot QD1 and the scatterers SP, the second light control unit CCP2 may include the second quantum dot QD2 and the scatterers SP, and the third light control unit CCP3 may not include a quantum dot but may include the scatterers SP.

The scatterers SP may be inorganic particles. For example, the scatterers SP may include at least one selected from among TiO₂, ZnO, Al₂O₃, SiO₂, and hollow silica. The scatterers SP may include any one selected from TiO₂, ZnO, Al₂O₃, SiO₂, and hollow silica, or may be a mixture of two or more materials selected from TiO₂, ZnO, Al₂O₃, SiO₂, and hollow silica.

The first light control unit CCP1, the second light control unit CCP2, and the third light control unit CCP3 may each include base resins BR1, BR2, and BR3 for dispersing the quantum dots QD1 and QD2 and the scatterers SP. In an embodiment, the first light control unit CCP1 may include the first quantum dot QD1 and the scatterers SP dispersed in the first base resin BR1, the second light control unit CCP2 may include the second quantum dot QD2 and the scatterers SP dispersed in the second base resin BR2, and the third light control unit CCP3 may include the scatterers SP dispersed in the third base resin BR3.

The base resins BR1, BR2, and BR3 are a medium in which the quantum dots QD1 and QD2 and the scatterers SP are dispersed, and may be formed of various suitable resin compositions, which may be generally referred to as a binder. For example, the base resins BR1, BR2, and BR3 may be an acrylic-based resin, a urethane-based resin, a silicone-based resin, an epoxy-based resin, and/or the like. Base resins BR1, BR2, and BR3 may be transparent resins. In an embodiment, the first base resin BR1, the second base resin BR2, and the third base resin BR3 may each be the same as or different from each other.

The light control layer CCL may include a barrier layer BFL1. The barrier layer BFL1 may serve to prevent or reduce introduction of moisture and/or oxygen (hereinafter may be referred to as "moisture/oxygen"). The barrier layer BFL1 may be on the light control units CCP1, CCP2, and CCP3 to prevent or reduce exposure of the light control units CCP1, CCP2, and CCP3 to moisture/oxygen. The barrier layer BFL1 may cover the light control units CCP1, CCP2, and CCP3. In embodiments, a barrier layer BFL2 may be provided between the light control units CCP1, CCP2, and CCP3 and the color filter layer CFL.

The barrier layers BFL1 and BFL2 may include at least one inorganic layer. In embodiments, the barrier layers BFL1 and BFL2 may be formed of an inorganic material. For example, the barrier layers BFL1 and BFL2 may be formed including silicon nitride, aluminium nitride, zirconium nitride, titanium nitride, hafnium nitride, tantalum nitride, silicon oxide, aluminium oxide, titanium oxide, tin oxide, cerium oxide, silicon oxynitride, or a metal thin film in which light transmittance is secured, and the like. The barrier layers BFL1 and BFL2 may further include an organic film. The barrier layers BFL1 and BFL2 may be formed of a single layer or a plurality of layers.

In the display module DM-a according to an embodiment, the color filter layer CFL may be on the light control layer CCL. For example, the color filter layer CFL may be directly on the light control layer CCL. In embodiments, the barrier layer BFL2 may be omitted.

The color filter layer CFL may include filters CF1, CF2, and CF3. In embodiments, the color filter layer CFL may include a first filter CF1 transmitting second color light, a second filter CF2 transmitting third color light, and a third filter CF3 transmitting first color light. For example, the first filter CF1 may be a red filter, the second filter CF2 may be a green filter, and the third filter CF3 may be a blue filter. The filters CF1, CF2, and CF3 may each include a polymer photosensitive resin, a pigment and/or a dye. The first filter CF1 may include a red pigment and/or a red dye, the second filter CF2 may include a green pigment and/or a green dye, and the third filter CF3 may include a blue pigment and/or a blue dye.

An embodiment is not limited thereto, and the third filter CF3 may not include a pigment or a dye. The third filter CF3 may include a polymer photosensitive resin, but not include a pigment or a dye. The third filter CF3 may be transparent. The third filter CF3 may be formed of a transparent photosensitive resin.

In an embodiment, the first filter CF1 and the second filter CF2 may be yellow filters. The first filter CF1 and the second filter CF2 may not be separated and may be provided as a single body.

In embodiments, the color filter layer CFL may further include a light blocking unit. The light blocking unit may be a black matrix. The light blocking unit may be formed including an organic light blocking material or an inorganic light blocking material, both including a black pigment and/or a black dye. The light blocking unit may prevent or reduce light leakage, and separate boundaries between the adjacent filters CF1, CF2, and CF3. In an embodiment, the light blocking unit may be formed with a blue filter.

The first to third color filters CF1, CF2, and CF3 may respectively correspond to the first light emitting region PXA-R, the second light emitting region PXA-G, and the third light emitting region PXA-B.

The base substrate BL may be on the color filter layer CFL. The base substrate BL may be a member providing a base surface on which the color filter layer CFL and the light control layer CCL are provided. The base substrate BL may be a glass substrate, a metal substrate, a plastic substrate, and/or the like. However, an embodiment is not limited thereto, and the base substrate BL may be an inorganic layer, an organic layer, or a composite material layer. In embodiments, the base substrate BL may not be provided in an embodiment.

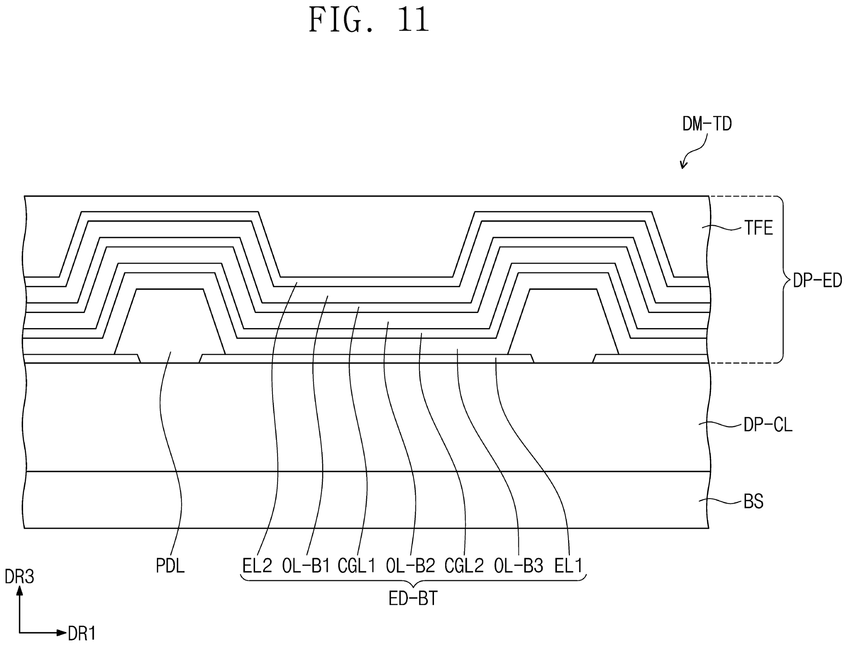

FIG. 11 is a cross-sectional view showing a portion of a display module according to an embodiment; In a display module DM-TD according to an embodiment, a light emitting element ED-BT may include a plurality of light emitting structures OL-B1, OL-B2, and OL-B3. At least one selected from the plurality of light emitting structures OL-B1, OL-B2, and OL-B3 includes the amine compound of an embodiment. Accordingly, the light emitting element ED-BT may exhibit long lifespan characteristics. In embodiments, the light emitting element ED-BT according to an embodiment may emit blue light and exhibit long lifespan characteristics.

The light emitting element ED-BT may include the first electrode EL1 and the second electrode EL2 facing each other, and the plurality of light emitting structures OL-B1, OL-B2, and OL-B3 provided by being sequentially stacked in a thickness direction between the first electrode EL1 and the second electrode EL2. The light emitting structures OL-B1, OL-B2, and OL-B3 each may include the emission layer EML (FIG. 5), a hole transport region HTR and an electron transport region ETR provided with the emission layer EML (FIG. 8) therebetween. In embodiments, the light emitting element ED-BT included in the display module DM-TD according to an embodiment may be a light emitting element having a tandem structure including a plurality of emission layers.

In an embodiment shown in FIG. 11, light emitted from each of the light emitting structures OL-B1, OL-B2, and OL-B3 may all be blue light. However, an embodiment is not limited thereto, and wavelength ranges of light emitted from each of the light emitting structures OL-B1, OL-B2, and OL-B3 may be different from each other. For example, the light emitting element ED-BT including the plurality of light emitting structures OL-B1, OL-B2, and OL-B3 emitting light in different wavelength ranges may emit white light.

Charge generation layers CGL1 and CGL2 may be between neighboring light emitting structures OL-B1, OL-B2, and OL-B3. In an embodiment shown in FIG. 11, the charge generation layers CGL1 and CGL2 may include a first charge generation layer CGL1 between the first light emitting structure OL-B1 and the second light emitting structure OL-B2, and a second charge generation layer CGL2 between the second light emitting structure OL-B2 and the third light emitting structure OL-B3. The first and second charge generation layers CGL1 and CGL2 may each independently include a p-type charge generation layer and/or an n-type charge generation layer.

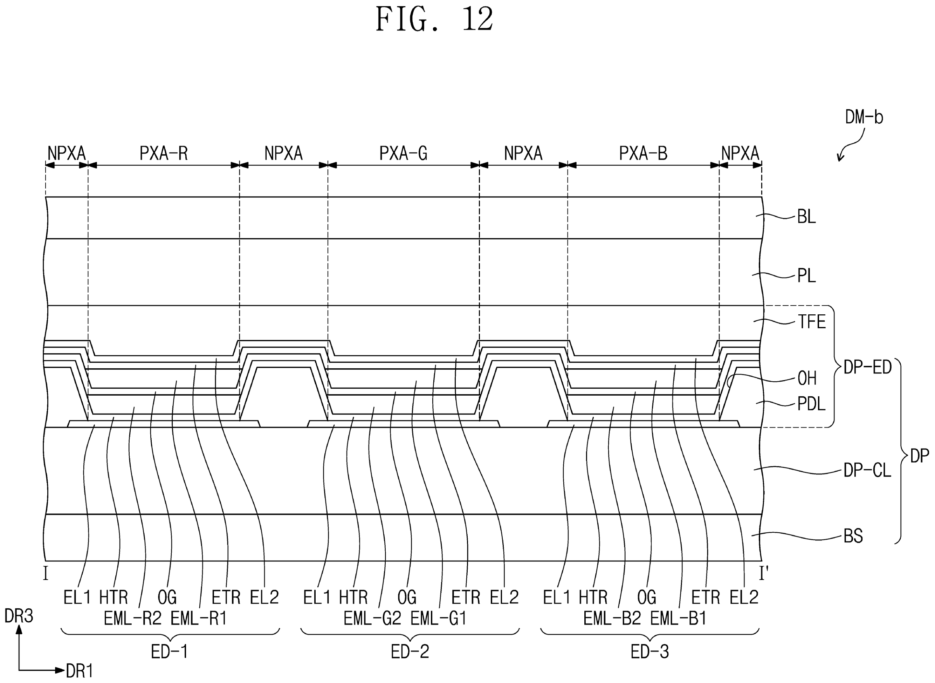

Referring to FIG. 12, a display module DM-b according to an embodiment may include light emitting elements ED-1, ED-2, and ED-3 in which two emission layers are stacked. At least one selected from the light emitting elements ED-1, ED-2, and ED-3 includes the amine compound according to an embodiment in the hole transport region HTR. Accordingly, the light emitting elements ED-1, ED-2, and ED-3 may exhibit long lifespan characteristics. The light emitting element ED-3 according to an embodiment may exhibit long lifespan characteristics in a blue light emission region.

Compared to the display module DM according to an embodiment shown in FIG. 4, the difference is that in an embodiment shown in FIG. 12, the first to third light emitting elements ED-1, ED-2, and ED-3 each include two emission layers stacked in a thickness direction. In each of the first to third light emitting elements ED-1, ED-2, and ED-3, the two emission layers may emit light in the same wavelength range.

The first light emitting element ED-1 may include a first red emission layer EML-R1 and a second red emission layer EML-R2. The second light emitting element ED-2 may include a first green emission layer EML-G1 and a second green emission layer EML-G2. In embodiments, the third light emitting element ED-3 may include a first blue emission layer EML-B1 and a second blue emission layer EML-B2. A light emitting auxiliary portion OG may be between the first red emission layer EML-R1 and the second red emission layer EML-R2, between the first green emission layer EML-G1 and the second green emission layer EML-G2, and between the first blue emission layer EML-B1 and the second blue emission layer EML-B2.

The light emitting auxiliary portion OG may include a single layer or a plurality of layers. The light emitting auxiliary portion OG may include a charge generation layer. For example, the light emitting auxiliary portion OG may include an electron transport region, a charge generation layer, and a hole transport region that are sequentially stacked. The light emitting auxiliary portion OG may be provided as a common layer throughout the first to third light emitting elements ED-1, ED-2, and ED-3. However, an embodiment is not limited thereto, and the light emitting auxiliary portion OG may be provided to be patterned inside the openings OH defined in the pixel defining films PDL.

The first red emission layer EML-R1, the first green emission layer EML-G1, and the first blue emission layer EML-B1 may be between the light emitting auxiliary portion OG and the electron transport region ETR. The second red emission layer EML-R2, the second green emission layer EML-G2, and the second blue emission layer EML-B2 may be between the hole transport region HTR and the light emitting auxiliary portion OG.

In embodiments, the light emitting element ED-1 may include the first electrode EL1, the hole transport region HTR, the second red emission layer EML-R2, the emission auxiliary portion OG, the first red emission layer EML-R1, the electron transport region ETR, and the second electrode EL2, which are sequentially stacked. The second light emitting element ED-2 may include the first electrode EL1, the hole transport region HTR, the second green emission layer EML-G2, the emission auxiliary portion OG, the first green emission layer EML-G1, the electron transport region ETR, and the second electrode EL2, which are sequentially stacked. The third light emitting element ED-3 may include the first electrode EL1, the hole transport region HTR, the second blue emission layer EML-B2, the emission auxiliary portion OG, the first blue emission layer EML-B1, the electron transport region ETR, and the second electrode EL2, which are sequentially stacked.

An optical auxiliary layer PL may be on the display element layer DP-ED. The optical auxiliary layer PL may include a polarizing layer. The optical auxiliary layer PL may be on the display panel DP to control reflected light in the display panel DP due to external light. In embodiments, the optical auxiliary layer PL may be omitted in the display device according to an embodiment.

A display module DM-c according to an embodiment shown in FIG. 13 is shown to include four light emitting structures OL-B3, OL-B2, OL-B1, and OL-C1. A light emitting element ED-CT may include the first electrode EL1 and the second electrode EL2 facing each other, and the first to fourth light emitting structures L-B1, OL-B2, OL-B3, and OL-C1 sequentially stacked in a thickness direction between the first electrode EL1 and the second electrode EL2. At least one selected from the first to fourth light emitting structures OL-B1, OL-B2, OL-B3, and OL-C1 includes the amine compound according to an embodiment. Accordingly, the light emitting element ED-CT may exhibit long lifespan characteristics. The light emitting element ED-CT according to an embodiment may exhibit improved lifespan characteristics in a blue light emission region.

Charge generation layers CGL3, CGL2, and CGL1 may be between the first to fourth light emitting structures OL-B1, OL-B2, OL-B3, and OL-C1. Among the four light emitting structures, the first to third light emitting structures OL-B1, OL-B2, and OL-B3 may emit blue light, and the fourth light emitting structure OL-C1 may emit green light. However, an embodiment is not limited thereto, and the first to fourth light emitting structures OL-B1, OL-B2, OL-B3, and OL-C1 may emit light having different wavelength ranges. The charge generation layers CGL3, CGL2 and CGL1 between the neighboring light emitting structures OL-B1, OL-B2, OL-B3, and OL-C1 may include a p-type charge generation layer and/or an n-type charge generation layer.

In an embodiment, an electronic device may include a display device including a plurality of light emitting elements, and a control portion configured to control the display device. In the electronic device according to an embodiment, at least one selected from the plurality of light emitting elements may include the amine compound according to an embodiment in a hole transport region. For example, at least one selected from the plurality of light emitting elements included in the electronic device according to an embodiment may include the amine compound according to an embodiment in a hole transport region.

The electronic device according to an embodiment may be a device activated in response to electrical signals. The electronic device according to an embodiment may include display devices including the display modules according to an embodiment described with reference to FIG. 4, and FIGS. 10 to 13. For example, the electronic device may include large-sized display devices such as televisions, monitors, and/or outdoor billboards, as well as small- and medium-sized display devices such as personal computers, laptop computers, personal digital terminals, in-vehicle display devices, game consoles, portable electronic devices, and/or cameras.

The electronic device according to an embodiment includes a display module including the amine compound according to an embodiment, and may thus exhibit improved reliability and long lifespan characteristics. The electronic device according to an embodiment may have improved display lifespan and exhibit excellent display quality.

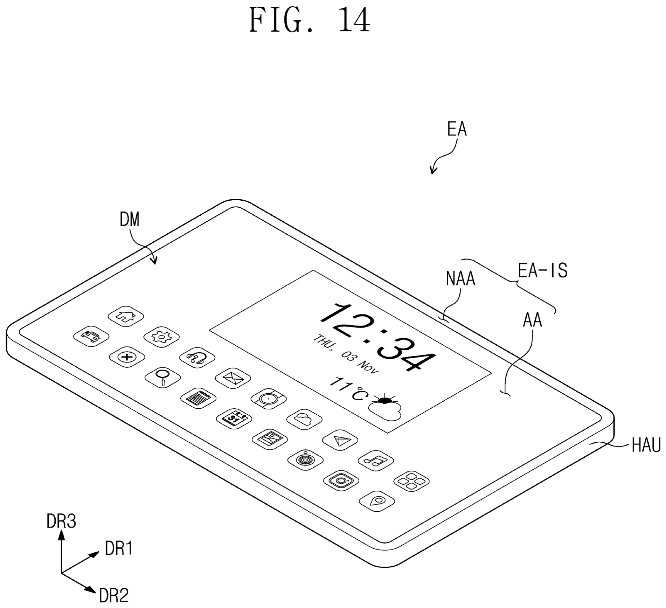

FIG. 14 shows a tablet terminal as an example of the electronic device EA. The electronic device EA according to an embodiment may include a display module DM according to an embodiment. For example, electronic modules, a camera module, a power module, and/or the like mounted on a main board together with the display module DM are provided in a bracket/housing HAU to form a tablet terminal.

The electronic device EA according to an embodiment shown in FIG. 14 may include the display modules according to an embodiment described with reference to FIG. 4 and FIGS. 10 to 13.

The electronic device EA including the display module DM provided with a flat display surface is shown in an embodiment, but an embodiment is not limited thereto. The electronic device EA may include a curved display surface or a three-dimensional display surface. For example, the three-dimensional display surface may include a plurality of display regions indicating different directions, and may also include a bent display surface. The electronic device EA according to the present embodiment may be a flexible electronic device. The flexible electronic device may be a foldable electronic device.

As shown in FIG. 14, the display surface EA-IS includes an active region AA on which images are displayed and a bezel region NAA adjacent to the active region AA. The bezel region NAA is a region on which images are not displayed. In FIG. 14, icon images are shown as an example of images. The active region AA may be referred to as a display region of the display module DM, and the bezel region NAA may be referred to as a non-display region of the display module DM.

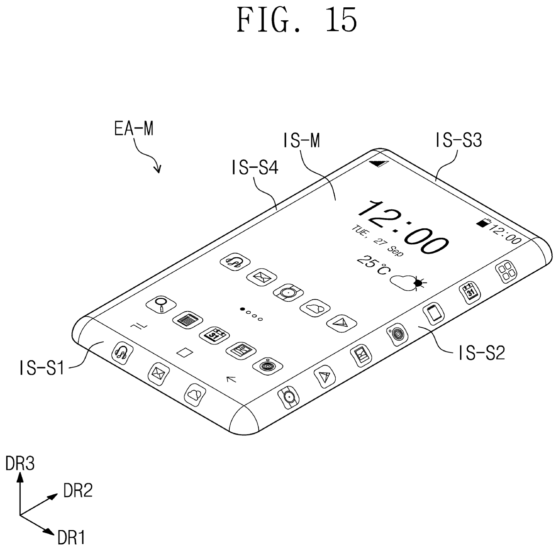

FIG. 15 shows a portable terminal as an example of an electronic device EA-M. Referring to FIG. 15, the electronic device EA-M according to an embodiment may include a plurality of display surfaces. The electronic device EA-M according to an embodiment may include display surfaces IS-M, IS-S1, IS-S2, IS-S3, and IS-S4 with different primary display directions.

In an embodiment, the electronic device EA-M may be a stereoscopic display device including an upper display surface IS-M and a plurality of side display surfaces IS-S1, IS-S2, IS-S3, and IS-S4. Each of the side display surfaces IS-S1, IS-S2, IS-S3, and IS-S4 may be a display surface extending from one side of the upper display surface IS-M. In an embodiment, the electronic device EA-M may include a main display surface that primarily displays images in one direction and a plurality of sub-display surfaces that display images in a direction different from the main display surface. In an embodiment of the electronic device EA-M shown in FIG. 15, the primary display surface may be the upper display surface IS-M, and the sub-display surfaces may be the side display surfaces IS-S1, IS-S2, IS-S3, and IS-S4.

The side display surfaces IS-S1, IS-S2, IS-S3, and IS-S4 may have display surfaces that are not parallel to the upper display surface IS-M. In embodiments, the plurality of side display surfaces IS-S1, IS-S2, IS-S3, and IS-S4 may be display regions that are each bent and extended from one side of the upper display surface IS-M. For example, the plurality of side display surfaces IS-S1, IS-S2, IS-S3, and IS-S4 may be bending display regions.

The electronic device EA-M according to an embodiment shown in FIG. 15 may include the display modules according to an embodiment described with reference to FIG. 4 and FIGS. 10 to 13.

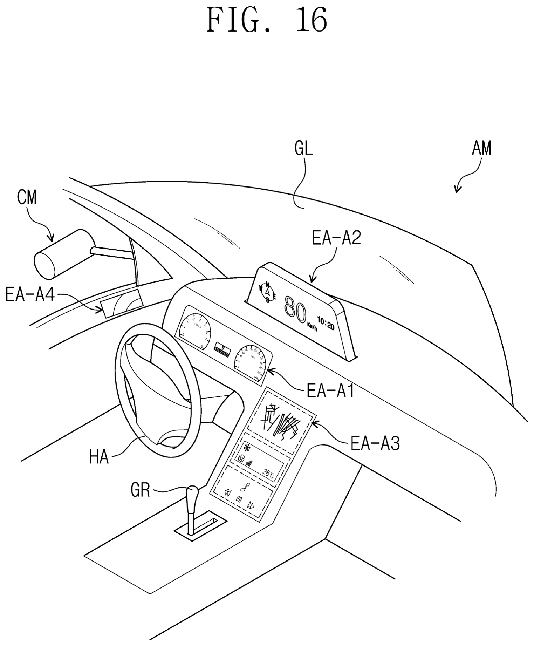

FIG. 16 is a view showing a vehicle AM in which first to fourth electronic devices EA-A1, EA-A2, EA-A3, and EA-A4 are provided. At least one selected from the first to fourth electronic devices EA-A1, EA-A2, EA-A3, and EA-A4 may include the display modules according to an embodiment described with reference to FIG. 4 and FIGS. 10 to 13.

FIG. 16 shows a car as the vehicle AM, but this is presented as an example, and the first to fourth electronic devices EA-A1, EA-A2, EA-A3, and EA-A4 may be on other means of transportation, such as bicycles, motorcycles, trains, ships, and airplanes.

At least one selected from the first to fourth electronic devices EA-A1, EA-A2, EA-A3, and EA-A4 may include the light emitting element ED according to an embodiment described with reference to FIGS. 5 to 9. At least one selected from the first to fourth electronic devices EA-A1, EA-A2, EA-A3, and EA-A4 includes the amine compound according to an embodiment. Accordingly, the first to fourth electronic devices EA-A1, EA-A2, EA-A3, and EA-A4 including the amine compound according to an embodiment may exhibit improved display lifespan. In embodiments, the first to fourth electronic devices EA-A1, EA-A2, EA-A3, and EA-A4 including the amine compound according to an embodiment may exhibit excellent display quality and improved reliability.

Referring to FIG. 16, the vehicle AM may include a wheel HA and a gear GR for operation control, and include a front window GL provided to face a driver.

The first electronic device EA-A1 may be a digital cluster displaying first information of the vehicle AM. The first information may include a first scale indicating driving speed of the vehicle AM, a second scale indicating engine revolutions (revolutions per minute (RPM)), and/or an image indicating fuel gauge, and/or the like. The first scale and the second scale may be displayed as digital images. In an embodiment shown in FIG. 16, the first display device EA-A1 may be provided in a first region overlapping the wheel HA. However, an embodiment is not limited thereto, and the first electronic device EA-A1 may be provided across the entire dashboard, or may be provided separately on a portion facing a driver seat and a portion facing a passenger seat.

The second display device EA-A2 may be provided in a second region between a driver seat and the front window GL. For example, the second electronic device EA-A2 may be a head up display HUD displaying second information of the vehicle AM. The second electronic device EA-A2 may be optically transparent. The second information includes digital numbers indicating driving speed of the vehicle AM and may further include information such as current time. In embodiments, the second information of the second electronic device EA-A2 may be projected and displayed on the front window GL. The display surface of the second electronic device EA-A2 may face a driver seat. In embodiments, the second electronic device EA-A2 may also provide images toward the front window GL.

The third electronic device EA-A3 may be provided in a third region adjacent to the gear GR. For example, the third display device EA-A3 may be a center information display CID for a vehicle, which is between a driver seat and a passenger seat, and displays third information. The passenger seat may be a seat spaced apart from the driver seat with the gear GR therebetween. The third information may include information about road conditions (e.g., navigation information), music and/or radio play, dynamic video play, temperature inside the vehicle AM, and/or the like.

The fourth display device EA-A4 may be provided in a fourth region spaced apart from the wheel HA and the gear GR and adjacent to a side of the vehicle AM. For example, the fourth electronic device EA-A4 may be a digital side mirror displaying fourth information. The fourth electronic device EA-A4 may display images of conditions outside the vehicle AM, which are taken by the camera module CM provided outside the vehicle AM. The fourth information may include images of conditions outside the vehicle AM.

The first to fourth information described above are presented as an example, and the first to fourth electronic devices EA-A1, EA-A2, EA-A3, and EA-A4 may further display information about inside or outside a vehicle. The first to fourth information may include different information. However, an embodiment is not limited thereto, and some of the first to fourth information may include the same information.

FIGS. 14 to 16 show an example of an electronic device or an example including an electronic device, and a display module including a light emitting element including an amine compound according to an embodiment may be adopted for other electronic devices without departing from the present disclosure.

Hereinafter, with reference to Examples and Comparative Examples, an amine compound according to an embodiment of the present disclosure and a light emitting element according to an embodiment will be described in more detail. The Examples below are shown only for the understanding of the subject matter of the present disclosure, and the scope of the present disclosure is not limited thereto.

### Examples

### 1. Synthesis of amine compounds of Examples

A process of synthesizing amine compounds according to an embodiment of the present disclosure will be described in detail by presenting a process of synthesizing amine compounds 1, 14, 25, 35, 91, 101, 63, 78, 125, 153, 160, 162, 173, 379, and 383 as an example. A process of synthesizing amine compounds, which will be further described hereinafter, is provided as an example, and thus a process of synthesizing compounds according to an embodiment of the present disclosure is not limited to Examples below.

In the synthetic method of the amine compound, which will be further described below, a molecular weight of the compound was measured by fast-atom bombardment-mass spectrometry (FAB-MS) using JMS-700V (JEOL Ltd.).

### (1) Synthesis of amine compound 1

Amine compound 1 according to an embodiment may be synthesized by, for example, processes of Reaction Formula 1 below.

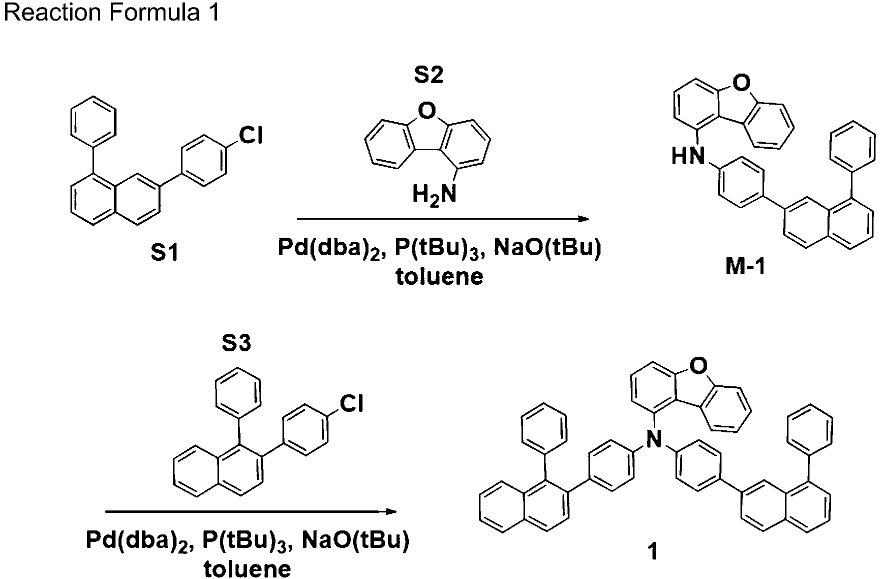

### Synthesis of Compound M-1

In an argon atmosphere, Compound S1 (10.00 g, 31.8 mmol), Pd(dba)₂(0.91 g, 0.05 equiv, 1.59 mmol), NaOtBu (3.06 g, 1 equiv, 31.8 mmol), toluene (464 mL), Compound **S2** (5.83 g, 1.0 equiv, 31.8 mmol), and P(tBu)₃ (1.29 g, 0.2 equiv, 6.36 mmol) were sequentially added to a 1000 mL three-necked flask, and the resultant mixture was heated and stirred under reflux for 6 hours. After the resulting product was cooled to room temperature, water was added to a reaction solvent, and an organic layer was separated and collected. Toluene was added to an aqueous layer to further extract the organic layer, and then the organic layers were collected, washed with brine, and dried over MgSO₄. The crude product obtained by filtration of MgSO₄ and concentration of the organic layer was purified through silica gel column chromatography to obtain a white solid Compound M-1 (12.8 g, yield: 87%).

FAB-MS measurement showed a molecular ion peak at m/z (mass number) pf 461, thereby identifying Compound **M-1.**

### Synthesis of Compound 1

In an argon atmosphere, Compound **M-1** (10.00 g, 21.7 mmol), Pd(dba)₂ (0.62 g, 0.05 equiv, 1.09 mmol), NaOtBu (2.09 g, 1 equiv, 31.8 mmol), toluene (464 mL), Compound **S3** (6.83 g, 1.0 equiv, 21.7 mmol), and P(tBu)₃ (0.878 g, 0.2 equiv, 4.34 mmol) were sequentially added to a 1000 mL three-necked flask, and the resultant mixture was heated and stirred under reflux for 6 hours. After the resulting product was cooled to room temperature, water was added to a reaction solvent, and an organic layer was separated and collected. Toluene was added to an aqueous layer to further extract the organic layer, and then the organic layers were collected, washed with brine, and dried over MgSO₄. The crude product obtained by filtration of MgSO₄ and concentration of the organic layer was purified through silica gel column chromatography to obtain a white solid **Compound 1** (13.9 g, yield: 87%).

FAB-MS measurement showed a molecular ion peak at m/z (mass number) of 739, thereby identifying Compound 1.

### (2) Synthesis of Compound 173

Amine compound 173 according to an embodiment may be synthesized by, for example, a process of Reaction Formula 2 below.

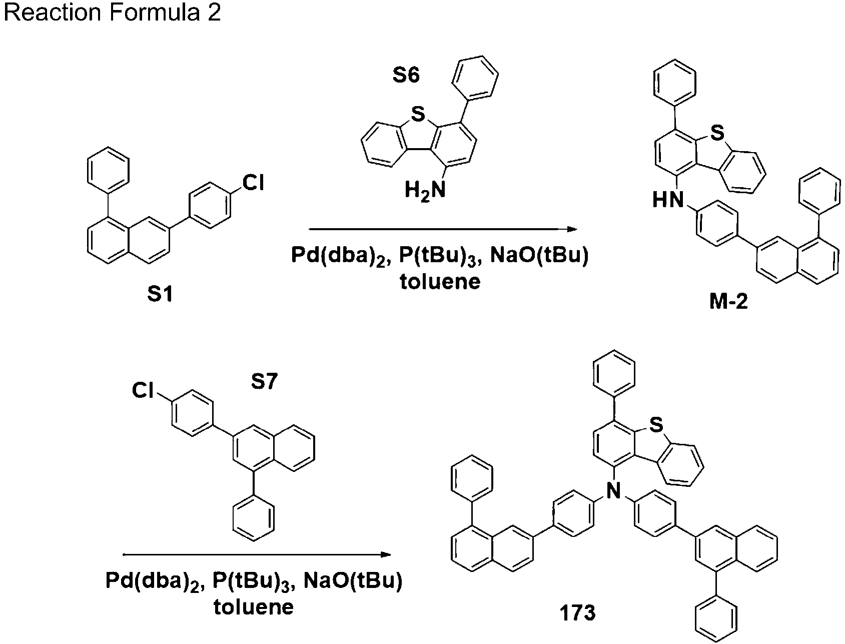

Compound M-2 was synthesized in substantially the same manner as in the synthesis of Compound M-1 described above, except that Compound S6 was used instead of Compound S2. In addition, Compound 173 was synthesized in substantially the same manner as in the synthesis of Compound 1, except that Compound S7 was used instead of Compound S3.

FAB-MS measurement showed a molecular ion peak at m/z (mass number) of 832, thereby identifying Compound 173.

### (3) Synthesis of amine compound 78

Amine compound 78 according to an embodiment may be synthesized by, for example, processes of Reaction Formula 3 below.

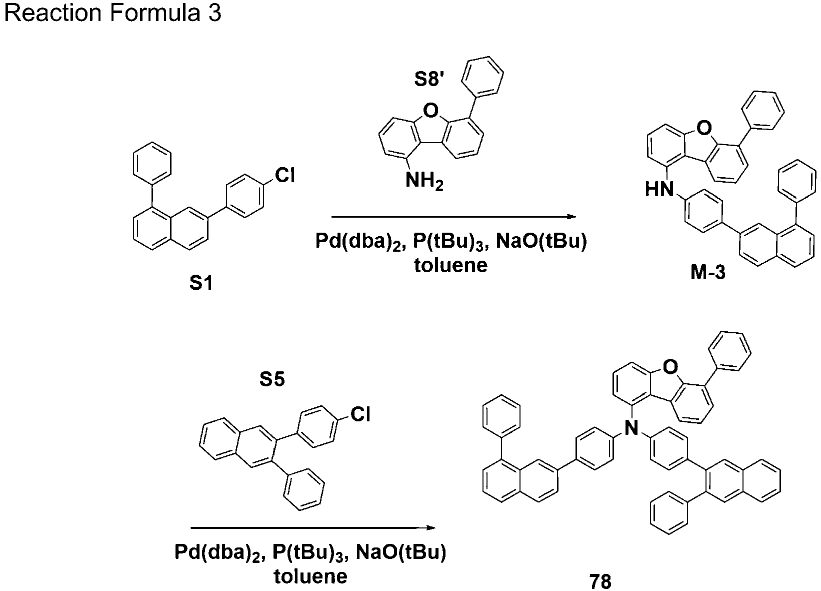

Compound M-3 was synthesized in substantially the same manner as in the synthesis of Compound M-1 described above, except that Compound S8' was used instead of Compound S2. In addition, Compound 78 was synthesized in substantially the same manner as in the synthesis of Compound 1, except that Compound S5 was used instead of Compound S3.

FAB-MS measurement showed a molecular ion peak at m/z (mass number) of 816, thereby identifying **Compound** 78.

### (4) Synthesis of amine compound 160

Amine compound 160 according to an embodiment may be synthesized by, for example, processes of Reaction Formula 4 below. 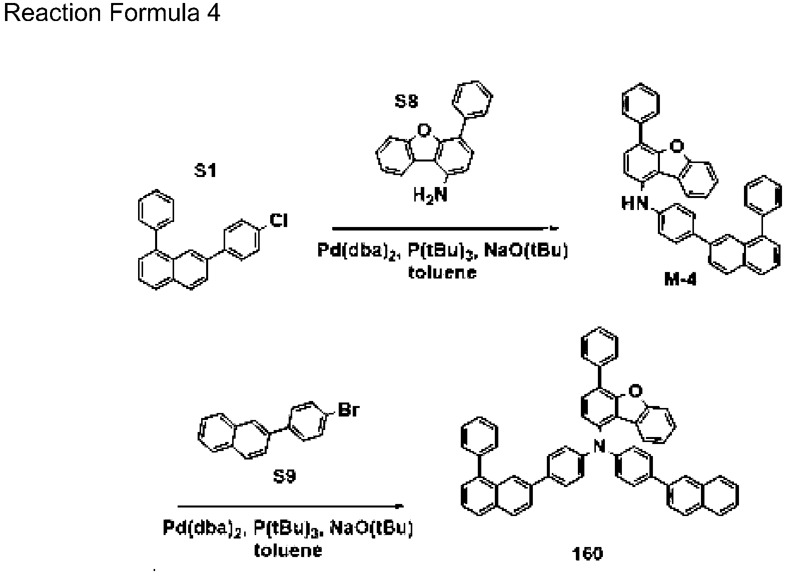 Compound M-4 was synthesized in substantially the same manner as in the synthesis of Compound M-1 described above, except that Compound S9 was used instead of Compound S1 and Compound S8 was used instead of Compound S2. In addition, Compound 160 was synthesized in substantially the same manner as in the synthesis of Compound 1, except that Compound S1 was used instead of Compound S3.

FAB-MS measurement showed a molecular ion peak at m/z (mass number) of 739.92, thereby identifying **Compound 160.**

### (5) Synthesis of amine compound 63

Amine compound 63 according to an embodiment may be synthesized by, for example, processes of Reaction Formula 5 below.

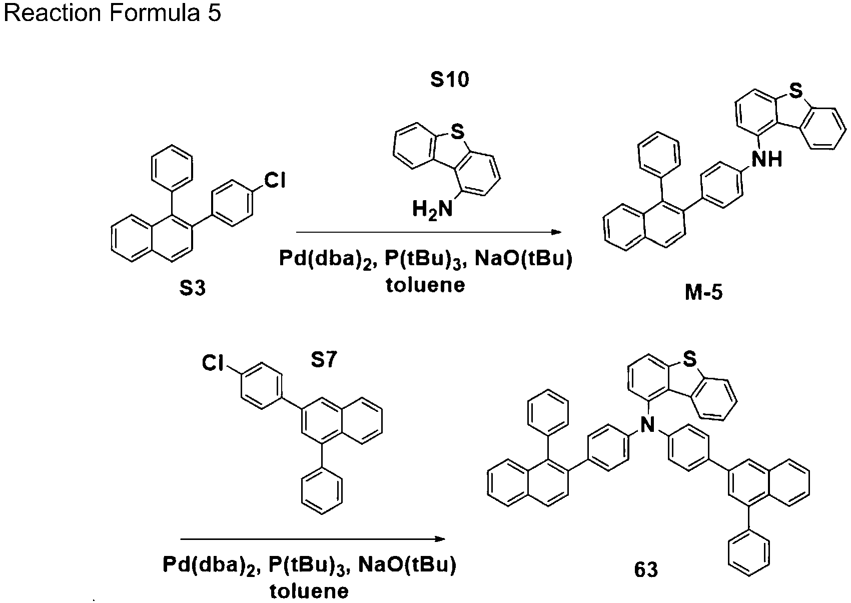

Compound M-5 was synthesized in substantially the same manner as in the synthesis of Compound M-1 described above, except that Compound S3 was used instead of Compound S1 and Compound S10 was used instead of Compound S2. In addition, Compound 63 was synthesized in substantially the same manner as in the synthesis of Compound 1, except that Compound S7 was used instead of Compound S3.

FAB-MS measurement showed a molecular ion peak at m/z (mass number) of 755, thereby identifying Compound 63.

### (6) Synthesis of amine compound 14

Amine compound 14 according to an embodiment may be synthesized by, for example, processes of Reaction Formula 6 below. 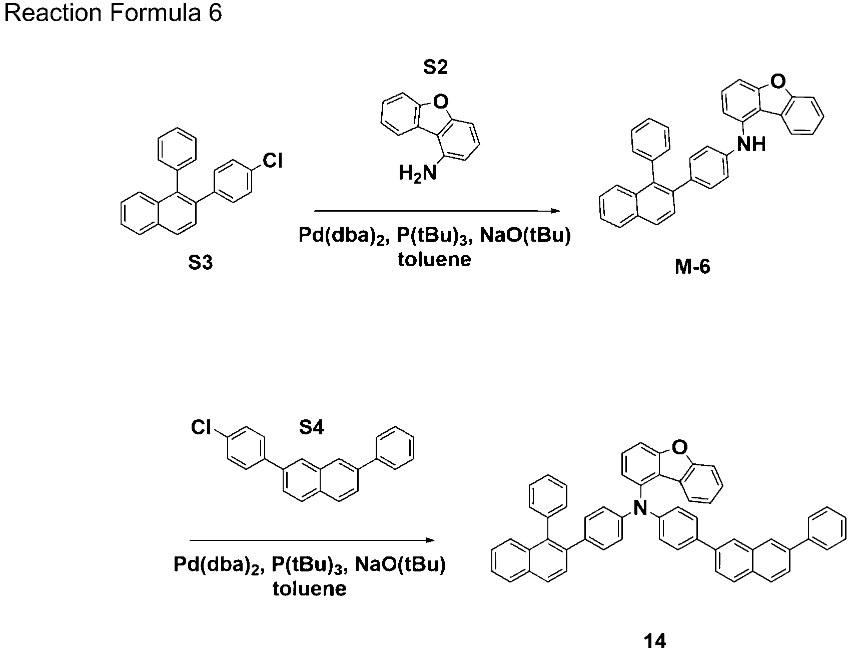 Compound M-6 was synthesized in substantially the same manner as in the synthesis of Compound M-1 described above, except that Compound S3 was used instead of Compound S1. In addition, Compound 14 was synthesized in substantially the same manner as in the synthesis of Compound 1, except that Compound S4 was used instead of Compound S3.

FAB-MS measurement showed a molecular ion peak at m/z (mass number) of 816, thereby identifying Compound 14.

### (7) Synthesis of amine compound 35

Amine compound 35 according to an embodiment may be synthesized by, for example, processes of Reaction Formula 7 below. 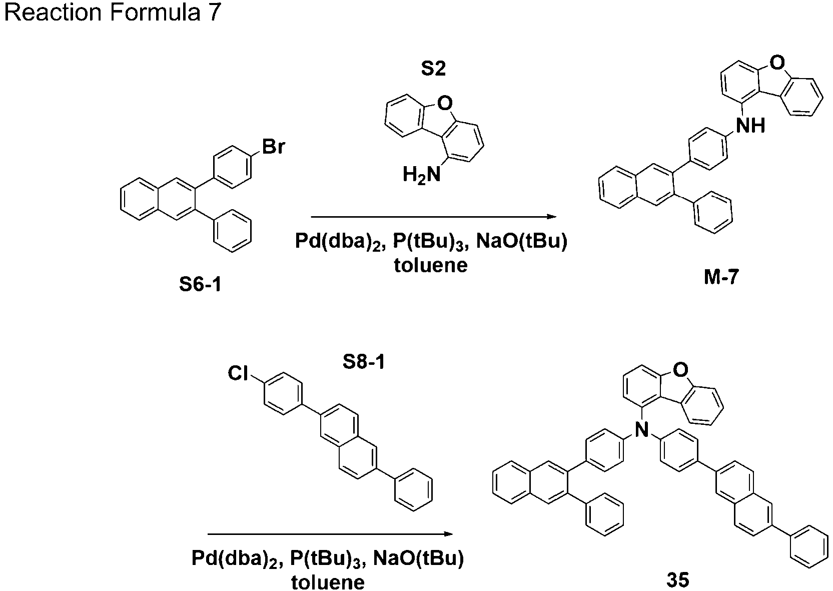 Compound M-7 was synthesized in substantially the same manner as in the synthesis of Compound M-1 described above, except that Compound S6-1 was used instead of Compound S1. In addition, Compound 35 was synthesized in substantially the same manner as in the synthesis of Compound 1, except that Compound S8-1 was used instead of Compound S3.

FAB-MS measurement showed a molecular ion peak at m/z (mass number) of 816, thereby identifying Compound 35.

### (8) Synthesis of amine compound 91

Amine compound 91 according to an embodiment may be synthesized by, for example, processes of Reaction Formula 8 below. 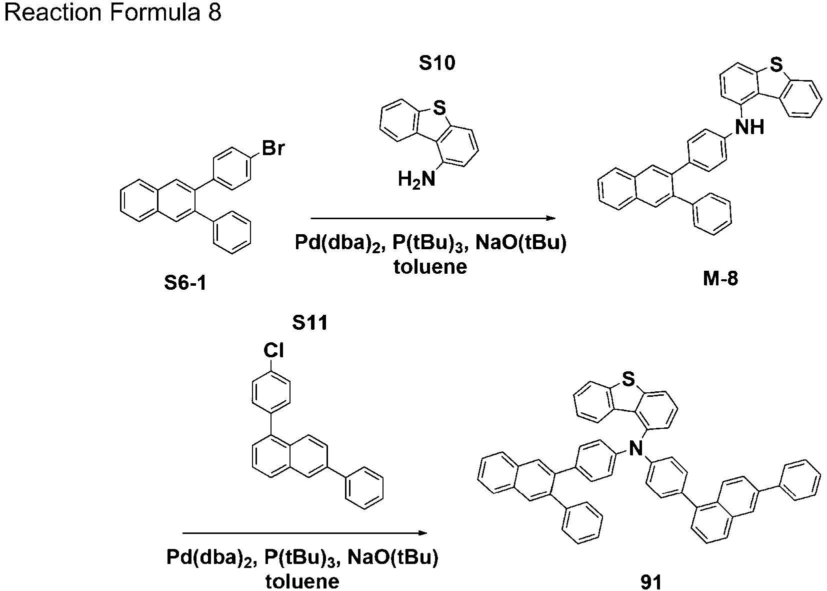 Compound M-8 was synthesized in substantially the same manner as in the synthesis of Compound M-1 described above, except that Compound S6-1 was used instead of Compound S1 and Compound S10 was used instead of Compound S2. In addition, Compound 91 was synthesized in substantially the same manner as in the synthesis of Compound 1, except that Compound S11 was used instead of Compound S3.

FAB-MS measurement showed a molecular ion peak at m/z (mass number) of 755, thereby identifying Compound 91.

### (9) Synthesis of amine compound 101

Amine compound 101 according to an embodiment may be synthesized by, for example, processes of Reaction Formula 9 below. 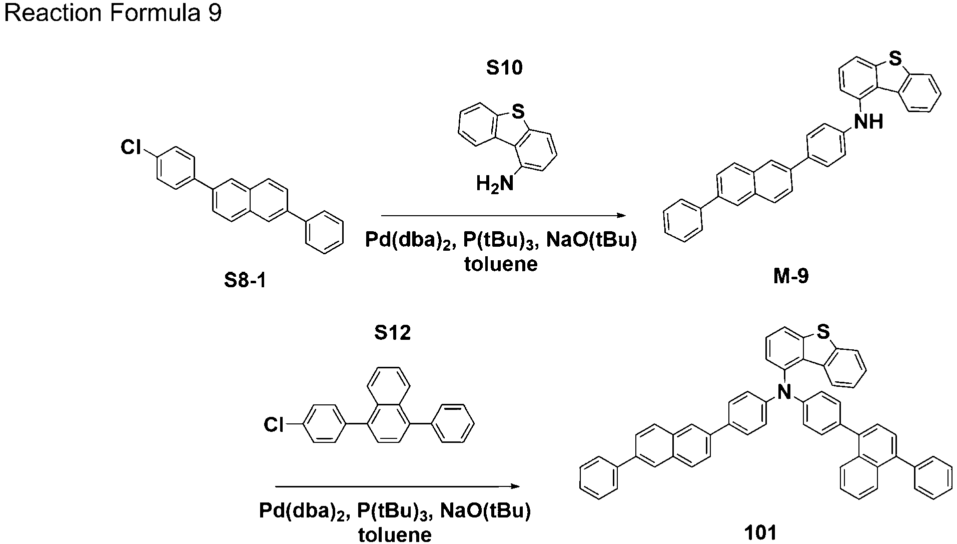 Compound M-9 was synthesized in substantially the same manner as in the synthesis of Compound M-1 described above, except that Compound S8-1 was used instead of Compound S1 and Compound S10 was used instead of Compound S2. In addition, Compound 101 was synthesized in substantially the same manner as in the synthesis of Compound 1, except that Compound S12 was used instead of Compound S3.

FAB-MS measurement showed a molecular ion peak at m/z (mass number) of 755, thereby identifying Compound 101.

### (10) Synthesis of amine compound 125

Amine compound 125 according to an embodiment may be synthesized by, for example, processes of Reaction Formula 10 below.

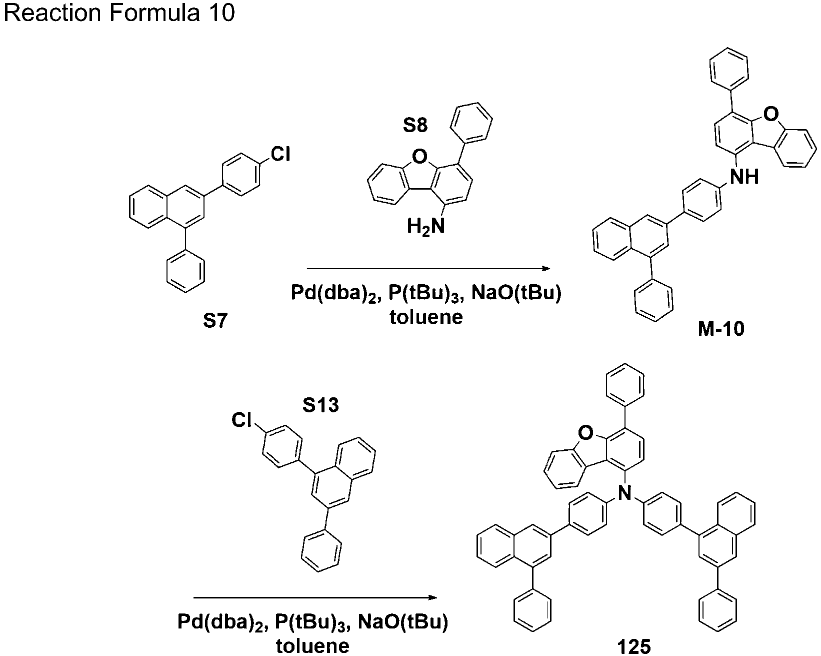

Compound M-10 was synthesized in substantially the same manner as in the synthesis of Compound M-1 described above, except that Compound S7 was used instead of Compound S1 and Compound S8 was used instead of Compound S2. In addition, Compound 125 was synthesized in substantially the same manner as in the synthesis of Compound 1, except that Compound S13 was used instead of Compound S3.

FAB-MS measurement showed a molecular ion peak at m/z (mass number) of 816, thereby identifying Compound 125.

### (11) Synthesis of amine compound 153

Amine compound 153 according to an embodiment may be synthesized by, for example, processes of Reaction Formula 11 below.

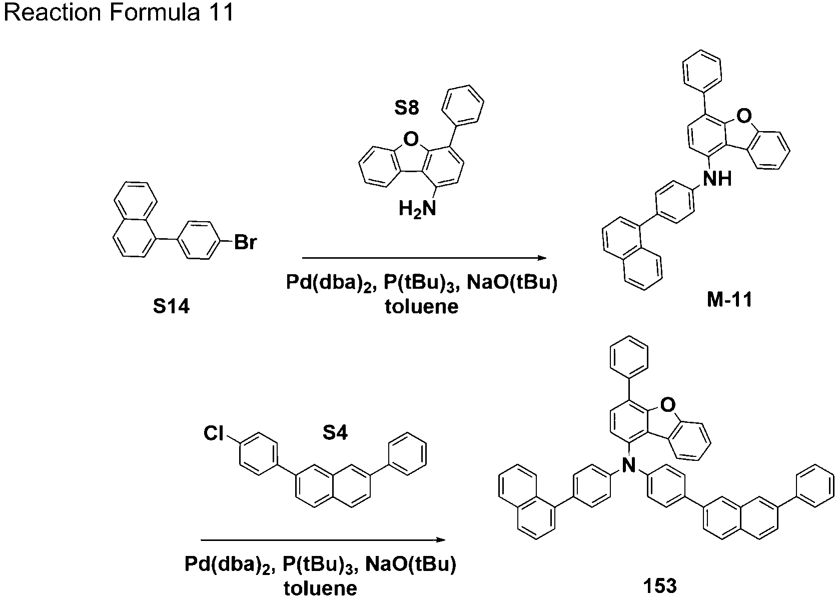

Compound M-11 was synthesized in substantially the same manner as in the synthesis of Compound M-1 described above, except that Compound S14 was used instead of Compound S1 and Compound S8 was used instead of Compound S2. In addition, Compound 153 was synthesized in substantially the same manner as in the synthesis of Compound 1, except that Compound S4 was used instead of Compound S3.

FAB-MS measurement showed a molecular ion peak at m/z (mass number) of 739, thereby identifying Compound 153.

### (12) Synthesis of amine compound 162

Amine compound 162 according to an embodiment may be synthesized by, for example, processes of Reaction Formula 12 below.

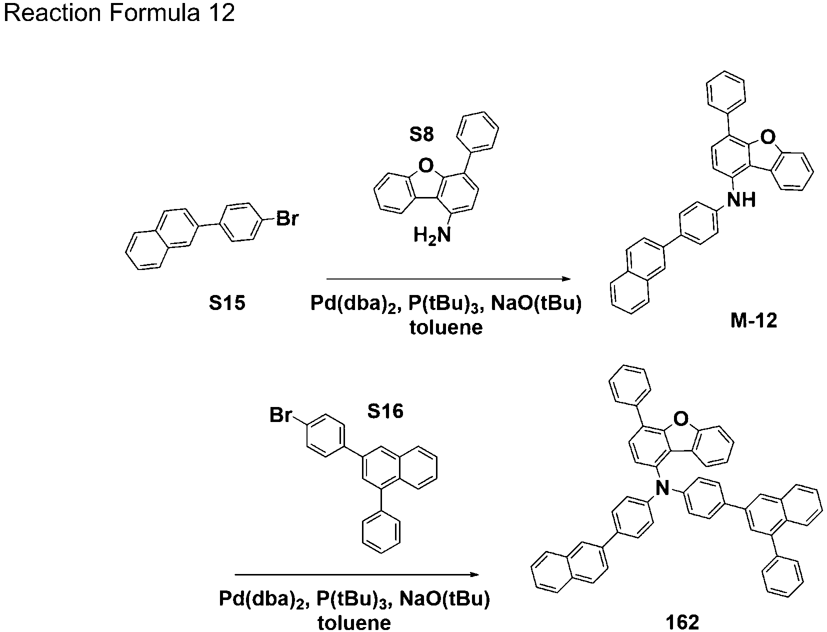

Compound M-12 was synthesized in substantially the same manner as in the synthesis of Compound M-1 described above, except that Compound S15 was used instead of Compound S1 and Compound S8 was used instead of Compound S2. In addition, Compound 162 was synthesized in substantially the same manner as in the synthesis of Compound 1, except that Compound S16 was used instead of Compound S3.

FAB-MS measurement showed a molecular ion peak at m/z (mass number) of 739, thereby identifying Compound 162.

### (13) Synthesis of amine compound 25

Amine compound 25 according to an embodiment may be synthesized by, for example, processes of Reaction Formula 13 below.

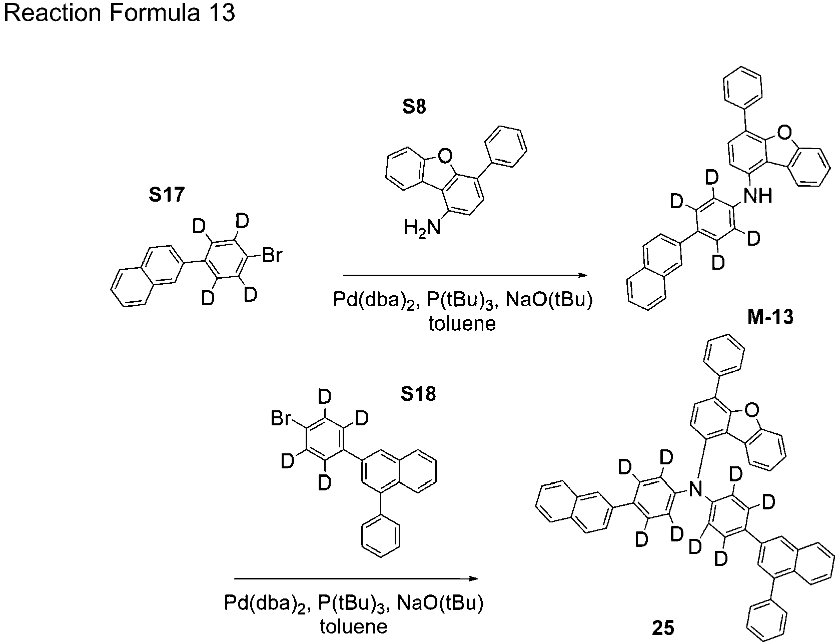

Compound M-13 was synthesized in substantially the same manner as in the synthesis of Compound M-1 described above, except that Compound S17 was used instead of Compound S1 and Compound S8 was used instead of Compound S2. In addition, Compound 25 was synthesized in substantially the same manner as in the synthesis of Compound 1, except that Compound S18 was used instead of Compound S3.

FAB-MS measurement showed a molecular ion peak at m/z (mass number) of 749, thereby identifying Compound 25.

### (14) Synthesis of amine compound 379

Amine compound 379 according to an embodiment may be synthesized by, for example, a process of Reaction Formula 14 below.

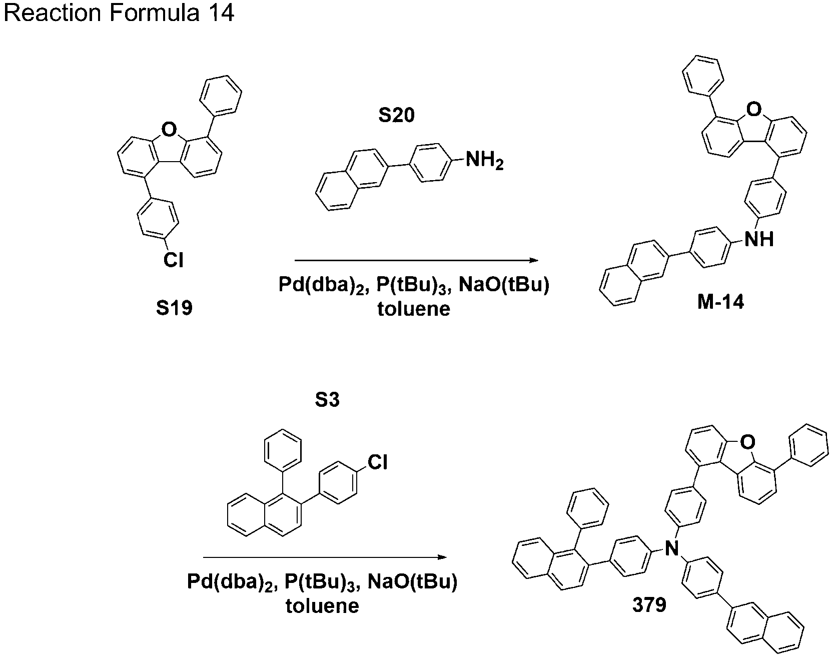

Compound M-14 was synthesized in substantially the same manner as in the synthesis of Compound M-1 described above, except that Compound S19 was used instead of Compound S1 and Compound S20 was used instead of Compound S2. In addition, Compound 379 was synthesized in substantially the same manner as in the synthesis of Compound 1.

FAB-MS measurement showed a molecular ion peak at m/z (mass number) of 815, thereby identifying Compound 379.

### (15) Synthesis of amine compound 383

Amine compound 383 according to an embodiment may be synthesized by, for example, a process of Reaction Formula 15 below.

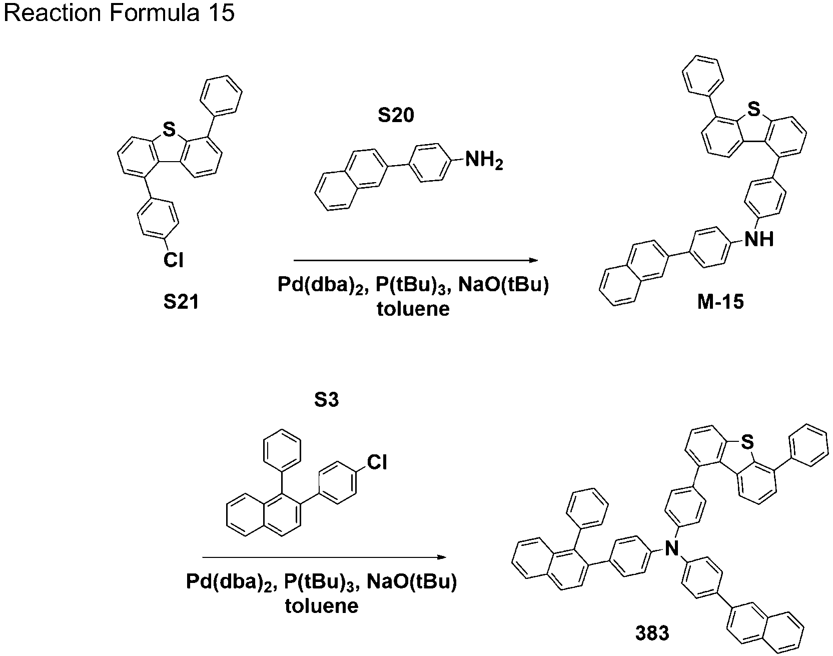

Compound M-15 was synthesized in substantially the same manner as in the synthesis of Compound M-1 described above, except that Compound S21 was used instead of Compound S1 and Compound S20 was used instead of Compound S2. In addition, Compound 383 was synthesized in substantially the same manner as in the synthesis of Compound 1.

FAB-MS measurement showed a molecular ion peak at m/z (mass number) of 831, thereby identifying Compound 379.

### 2. Preparation and Evaluation of Light Emitting Element

### (1) Preparation of light emitting element

Light emitting elements including amine compounds according to an embodiment or Comparative Example Compounds in a hole transport layer were prepared through a method below. The amine compounds according to an embodiment were each used as a hole transport layer material to prepare light emitting elements of Examples 1 to 13. Comparative Example Compounds X-1 to X-8 were each used as a hole transport layer material to prepare light emitting elements of Comparative Examples 1 to 8. Comparative Example Compounds used in Examples 1 to 15 and Comparative Examples 1 to 8 are as follows.

### Example Compound

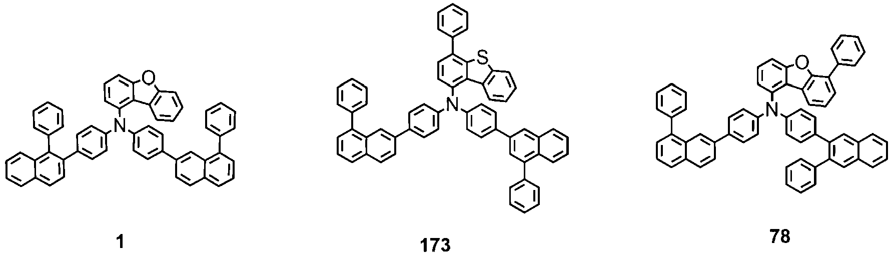

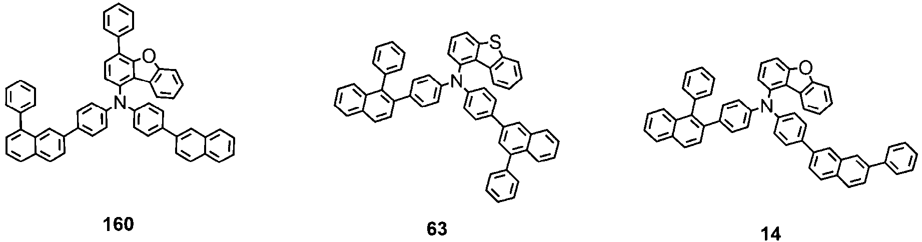

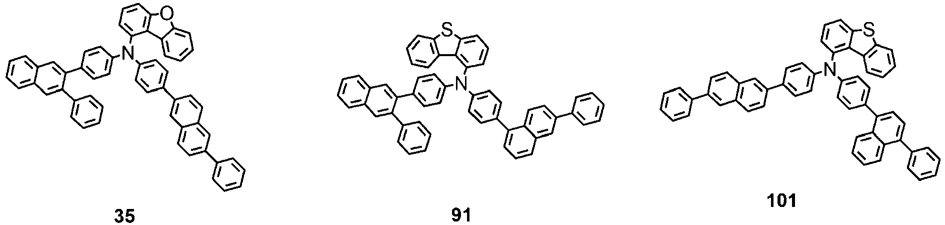

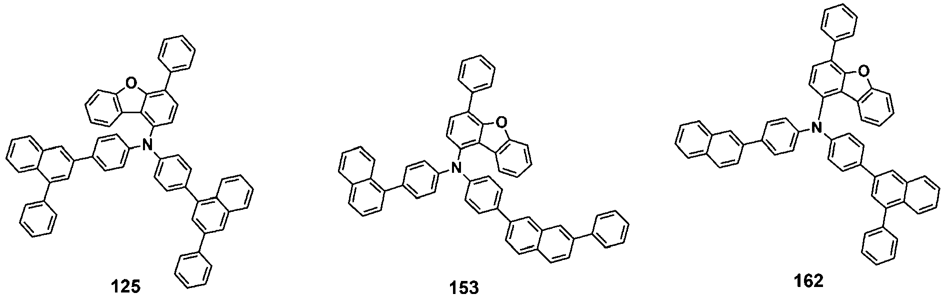

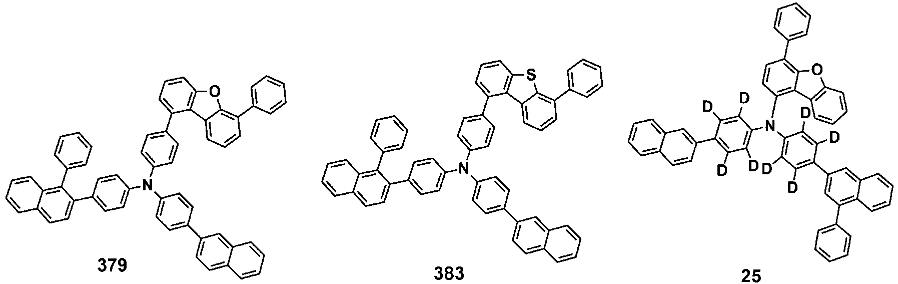

### Comparative Example Compound

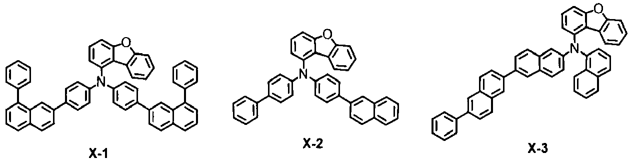

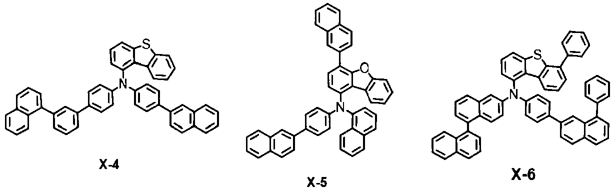

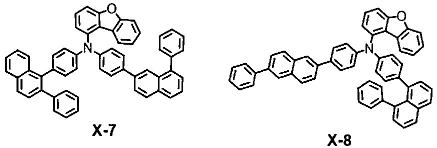

A glass substrate on which an ITO having a thickness of 150 nm was patterned as a first electrode was subjected to ultrasonic cleaning using isopropyl alcohol and pure water each for 5 minutes. The glass substrate was irradiated with UV for 30 minutes, and ozone-treated. Thereafter, a hole injection layer was formed through the deposition of 2-TNATA with a thickness of 60 nm. On the hole injection layer, a hole transport layer was formed through the deposition of Example Compounds or Comparative Example Compounds having a thickness of 30 nm.

On the hole transport layer, an emission layer was formed through the co-deposition of TBP and ADN to a thickness of 25 nm. TBP and ADN were subjected to the co-deposition at a weight ratio of 3:97. Thereafter, an electron transport region was formed through the sequential deposition of Alq₃ to a thickness of 25 nm and LiF to a thickness of 1 nm.

Then, a second electrode was formed through the deposition of Al to a thickness of 100 nm.

In an embodiment, the hole transport region, the emission layer, the electron transport region, and the second electrode were formed using a vacuum deposition apparatus.

The compounds used to manufacture the light emitting elements are as follows.

### Materials used in manufacture of light emitting elements

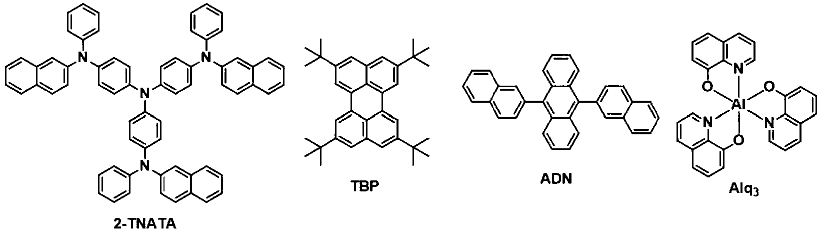

### (2) Evaluation of light emitting element

Table 1 below shows evaluation results of the light emitting elements of Examples and Comparative Examples. Table 1 shows driving voltage, luminous efficiency, and lifespan for the light emitting elements of Examples and Comparative Examples. The driving voltage, luminous efficiency, and lifespan were evaluated in a dark room using a 2400 Series Source Meter from Keithley Instruments, USA, a colorimeter CS-200 from Konica Minolta, Japan, and a measurement PC program LabVIEW 8.2 from National Instruments, Japan. The lifespan is defined as the time required for luminance to decay to 50% from an initial luminance of 100 cd/m² during continuous operation.

**Table 1**

| Preparation example of element | Hole transport layer material | Driving voltage (V) | Luminous efficiency (cd/A) | Element lifespan LT50 (h) |
|---|---|---|---|---|
| Example 1 | Compound 1 | 5.4 | 9.5 | 2800 |
| Example 2 | Compound 173 | 5.5 | 9.4 | 2820 |
| Example 3 | Compound 78 | 5.5 | 9.3 | 2750 |
| Example 4 | Compound 160 | 5.6 | 9.4 | 2780 |
| Example 5 | Compound 63 | 5.7 | 9.6 | 2790 |
| Example 6 | Compound 14 | 5.7 | 9.5 | 2800 |
| Example 7 | Compound 35 | 5.8 | 9.2 | 2760 |
| Example 8 | Compound 91 | 5.8 | 9.3 | 2770 |
| Example 9 | Compound 101 | 6.0 | 9.1 | 2750 |
| Example 10 | Compound 125 | 5.9 | 9.2 | 2760 |
| Example 11 | Compound 153 | 5.8 | 9.4 | 2760 |
| Example 12 | Compound 162 | 5.6 | 9.3 | 2750 |
| Example 13 | Compound 25 | 5.6 | 9.3 | 2780 |
| Example 14 | Compound 379 | 5.4 | 9.3 | 2740 |
| Example 15 | Compound 383 | 5.8 | 9.4 | 2780 |
| Comparative Example 1 | Comparative Example Compound X-1 | 6.8 | 8.0 | 2500 |
| Comparative Example 2 | Comparative Example Compound X-2 | 7.3 | 8.5 | 1800 |
| Comparative Example 3 | Comparative Example Compound X-3 | 6.1 | 8.3 | 2100 |
| Comparative Example 4 | Comparative Example Compound X-4 | 7.5 | 7.2 | 2300 |
| Comparative Example 5 | Comparative Example Compound X-5 | 7.8 | 7.1 | 2400 |
| Comparative Example 6 | Comparative Example Compound X-6 | 8.0 | 7.2 | 2450 |
| Comparative Example 7 | Comparative Example Compound X-7 | 7.7 | 8.4 | 2200 |
| Comparative Example 8 | Comparative Example Compound X-8 | 7.5 | 7.0 | 2300 |

Referring to Table 1, Examples 1 to 15 exhibited low driving voltage/high efficiency/long lifespan element characteristics compared to Comparative Examples 1 to 8.

Example Compounds are amine compounds including a substituted or unsubstituted 1-dibenzoheteroyl group bonded directly to a nitrogen atom or bonded to a nitrogen atom through a linker at a set or specific position, and two different substituted or unsubstituted naphthyl groups bonded to a nitrogen atom through a linker. The Example Compounds are compounds in which at least two of the 1-dibenzoheteroyl group and the two different naphthyl groups include an aromatic compound (aryl, heteroaryl) as a substituent. This allows Example Compounds to have suitable or appropriate steric effects and excellent charge balance. The Example Compounds include different naphthyl groups, and thus possess asymmetry and high amorphousness, which may suppress or reduce crystallization. Consequently, it is determined that elements have improved thin film quality, and thus exhibit low voltage/high efficiency/long lifespan element characteristics. In embodiments, referring to Examples 12 and 13, it is determined that if (e.g., when) some hydrogens are substituted with deuterium, elements exhibit equivalent or slightly improved element characteristics.

Conversely, it is determined that Comparative Example Compound X-1 including the same two naphthyl groups exhibits reduced luminous efficiency and lifespan characteristics.

It is determined that Comparative Example Compound X-2 is a compound including only one naphthyl group, and thus has poor thermal stability and is prone to decomposition, resulting in reduced luminous efficiency and lifespan characteristics.

It is determined that Comparative Examples compounds X-3 and X-5 have one naphthyl group corresponding to Ar₂ of Formula 1 according to embodiments of the present disclosure directly bonded to a nitrogen atom, which excessively increases steric hindrance around the nitrogen atom, resulting in reduced luminous efficiency and lifespan characteristics.

It is determined that in Comparative Example Compound X-4, neither of the two naphthyl groups nor the dibenzothiophene includes an aromatic substituent, causing disrupted charge balance, resulting in reduced luminous efficiency and lifespan characteristics.

It is determined that Comparative Example Compound X-6 is a compound in which Ar₁ of Formula 1 according to embodiments of the present disclosure includes a 1,1'-binaphthyl group, and thus has increased planarity and reduced thermal stability, resulting in reduced luminous efficiency and lifespan characteristics.

It is determined that each of Comparative Example Compounds X-7 and X-8 is a compound in which Ar₁ of Formula 1 according to embodiments of the present disclosure corresponds to a 4-(2-phenylnaphthylen-1-yl)phenyl group and a 4-(8-phenylnaphthylen-1-yl)phenyl group, and has steric hindrance, resulting in reduced luminous efficiency and lifespan characteristics.

A light emitting element according to an embodiment including the amine compound according to an embodiment in a hole transport region may exhibit low voltage operation/high efficiency/long lifespan characteristics. A light emitting element according to an embodiment emitting blue light and a light emitting element according to an embodiment emitting green light include the amine compound according to an embodiment in a hole transport region, and may thus exhibit low voltage operation/high efficiency/long lifespan characteristics.

A light emitting element according to an embodiment includes an amine compound according to an embodiment in a hole transport region, and thus are operable at low voltage and has excellent luminous efficiency and lifespan characteristics.

An amine compound according to an embodiment may be used as a material for a light emitting element, thereby enabling the light emitting element to operate at low voltage and to have excellent luminous efficiency and lifespan characteristics.

An electronic device according to an embodiment may exhibit excellent display quality and improved reliability.

In the above, description has been made with reference to embodiments of the present disclosure, but those skilled or of ordinary skill in the art may understand that various suitable modifications and changes may be made to embodiments of the present disclosure insofar as such modifications and changes do not depart from the technical scope of the present disclosure set forth in the appended claims, and equivalents thereof.

Therefore, the technical scope of the present disclosure is not to be limited to the contents stated in the detailed description of the specification, but should be determined by the claims, and equivalents thereof.

## Claims

1. An amine compound represented by Formula 1 below: 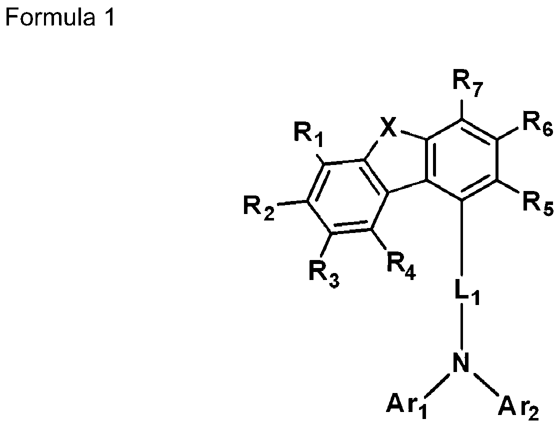 wherein in Formula 1 above,
L₁ is a direct linkage, a substituted or unsubstituted arylene group having 6 to 30 ring-forming carbon atoms, or a substituted or unsubstituted heteroarylene group having 2 to 30 ring-forming carbon atoms,
X is O or S,
R₁ to R₇ are each independently a hydrogen atom, a deuterium atom, a halogen atom, a substituted or unsubstituted alkyl group having 1 to 10 carbon atoms, a substituted or unsubstituted aryl group having 6 to 30 ring-forming carbon atoms, or a substituted or unsubstituted heteroaryl group having 5 to 30 ring-forming carbon atoms,
Ar₁ and Ar₂ are different from each other,
each of Ar₁ and Ar₂ does not include a 4-(8-phenylnaphthylen-1-yl)phenyl group, a 4-(2-phenylnaphthylen-1-yl)phenyl group, and a 1,1'-binaphthyl group, and
Ar₁ and Ar₂ are each independently represented by Formula 2 below: 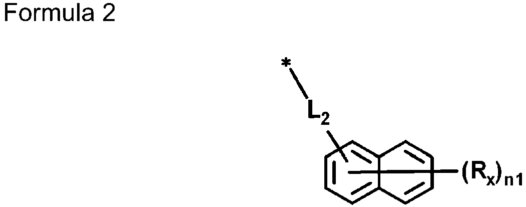
wherein in Formula 2 above,
L₂ is a substituted or unsubstituted arylene group having 6 to 30 ring-forming carbon atoms, or a substituted or unsubstituted heteroarylene group having 5 to 30 ring-forming carbon atoms,
Rₓ is a hydrogen atom, a deuterium atom, a halogen atom, a substituted or unsubstituted alkyl group having 1 to 10 carbon atoms, a substituted or unsubstituted aryl group having 6 to 30 ring-forming carbon atoms, or a substituted or unsubstituted heteroaryl group having 5 to 30 ring-forming carbon atoms wherein a bonding position of the heteroaryl group is not a heteroatom, n1 is an integer of 0 to 7,
if Rₓ is a fused ring in which rings having three or more rings are fused, a case in which the fused ring Rₓ is bonded to a carbon adjacent to a carbon bonded to L₂ is excluded,
at least one selected from R₁ to R₇, and Rₓ of Ar₁ is a substituted or unsubstituted aryl group having 6 to 30 ring-forming carbon atoms, or a substituted or unsubstituted heteroaryl group having 5 to 30 ring-forming carbon atoms wherein a heteroatom is not a bonding position, and
at least one selected from Rₓ's of Ar₂ is a substituted or unsubstituted aryl group having 6 to 30 ring-forming carbon atoms, or a substituted or unsubstituted heteroaryl group having 5 to 30 ring-forming carbon atoms wherein a heteroatom is not a bonding position.

2. The amine compound of claim 1, wherein the amine compound is represented by Formula 1-1 below: 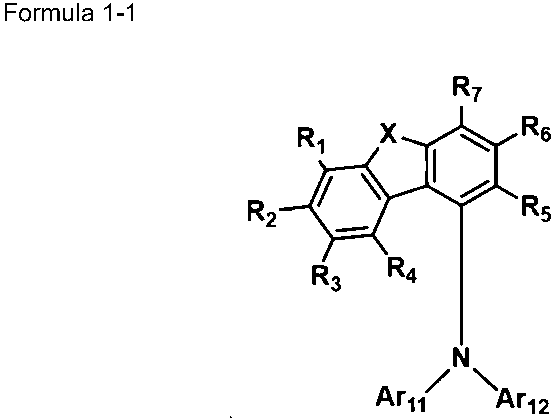
wherein in Formula 1-1 above, R₁ to R₇, and X are the same as defined in Formula 1 above, and
Ar₁₁ and Ar₁₂ are each represented by any one selected from Ar-a to Ar-k below, and Ar₁₁ and Ar₁₂ are different from each other, 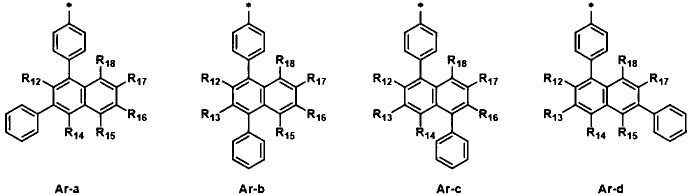 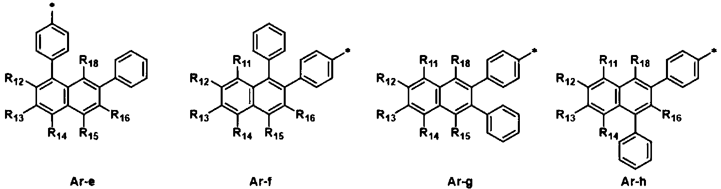 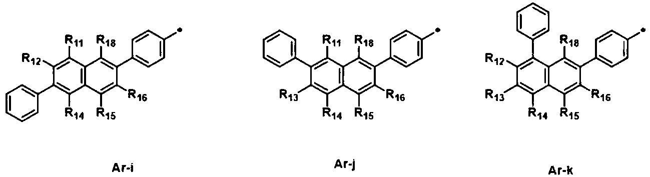
wherein in Ar-a to Ar-k above, R₁₁ to R₁₈ are each independently a hydrogen atom, a deuterium atom, a halogen atom, or a substituted or unsubstituted alkyl group having 1 to 10 carbon atoms.

3. The amine compound of claim 1, wherein the amine compound is represented by any one selected from Formulas 1-a to 1-c below: 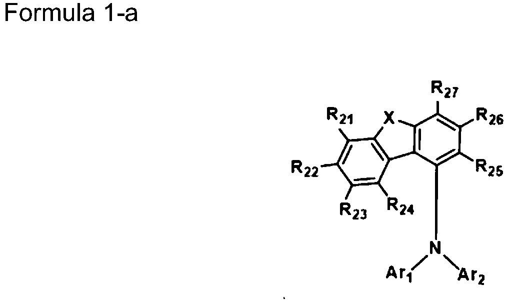 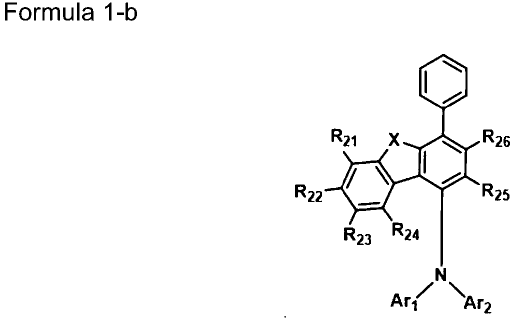 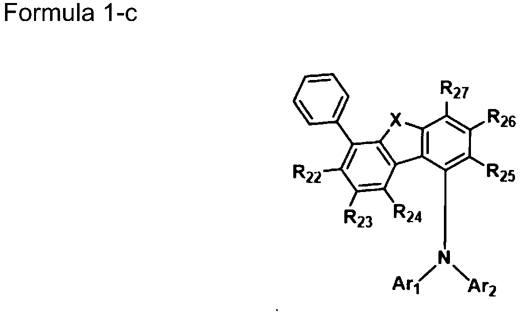
wherein in Formulas 1-a to 1-c above, R₂₁ to R₂₇ are each independently a hydrogen atom, a deuterium atom, a halogen atom, or a substituted or unsubstituted alkyl group having 1 to 10 carbon atoms, and
X, Ar₁, and Ar₂ are the same as defined in Formula 1.

4. The amine compound of any one of claims 1 to 3, wherein at least one hydrogen atom of the amine compound is substituted with a deuterium atom.

5. The amine compound of any one of claims 1 to 4, wherein Formula 1 above is a monoamine compound that does not include an amino group as a substituent.

6. The amine compound of claim 1, wherein the amine compound is any one selected from among compounds in Compound Group 1: 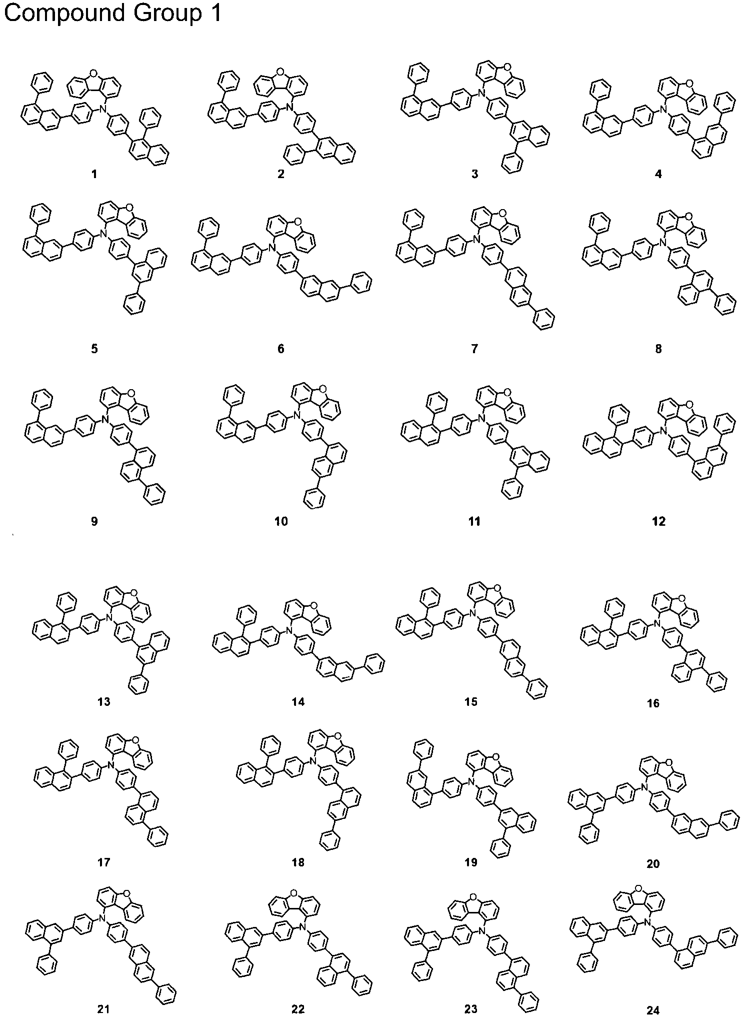 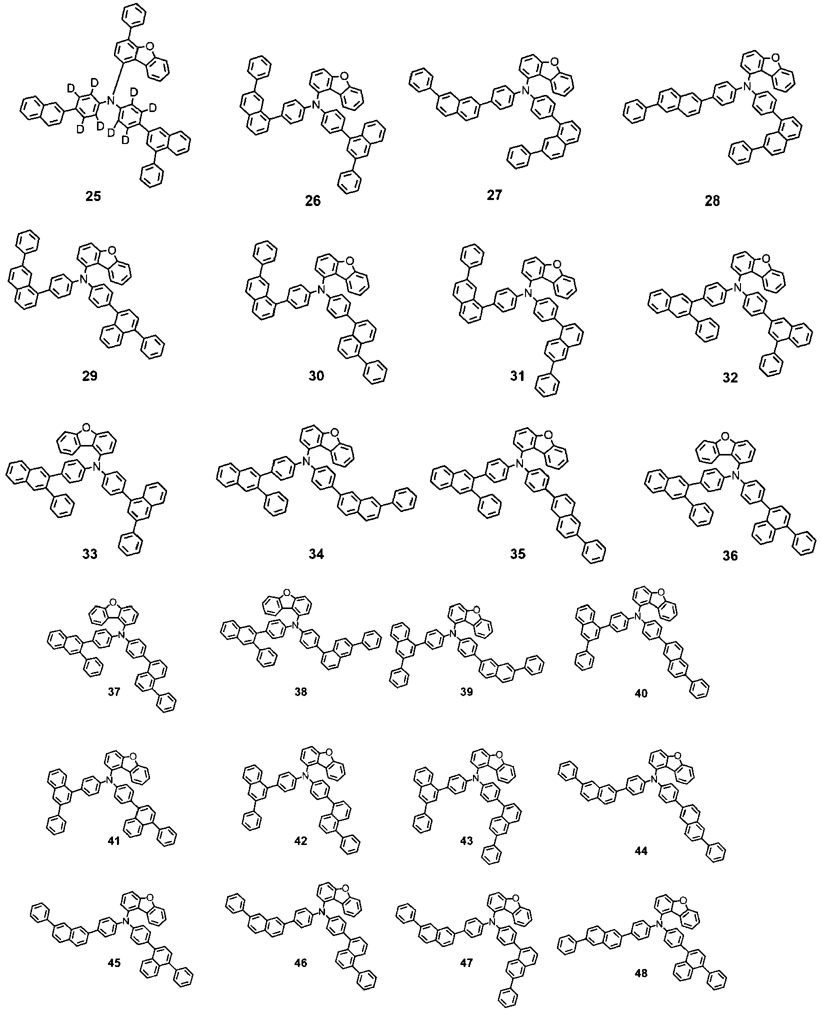 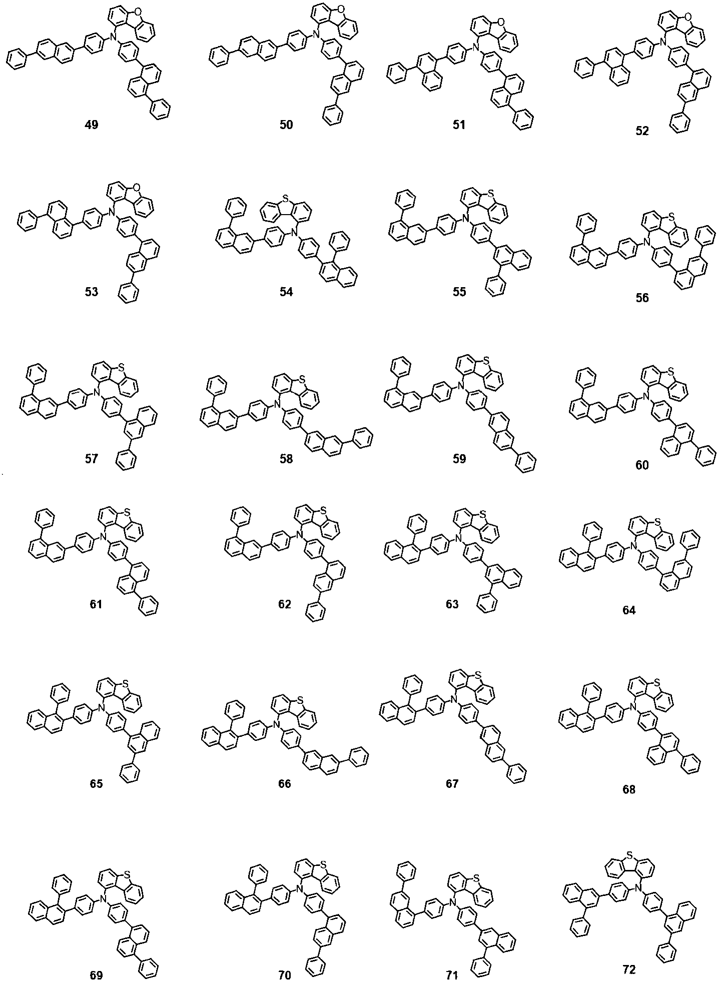 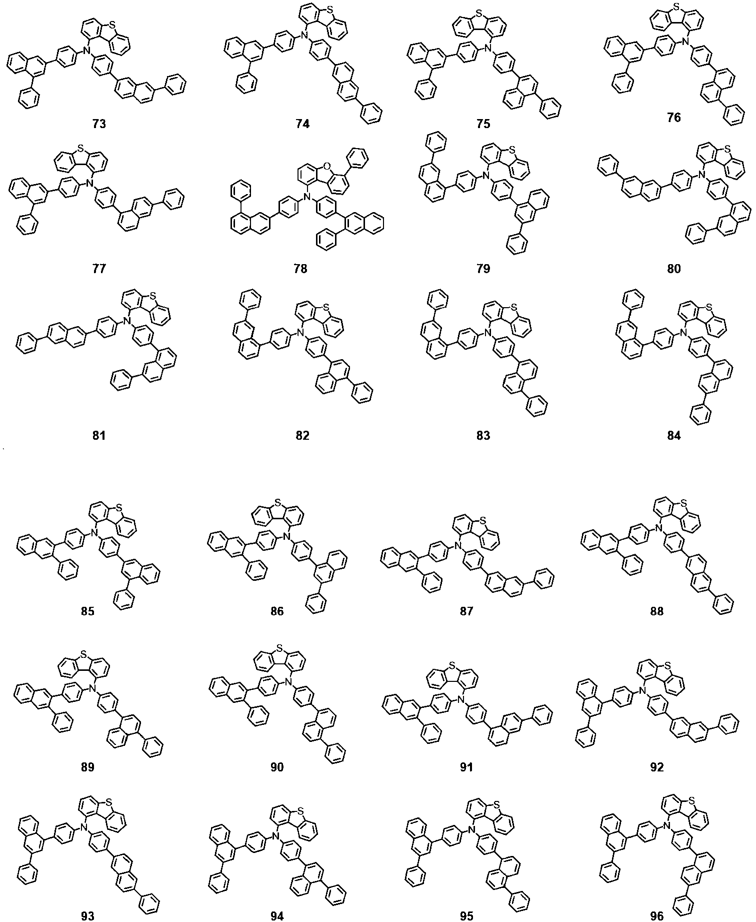 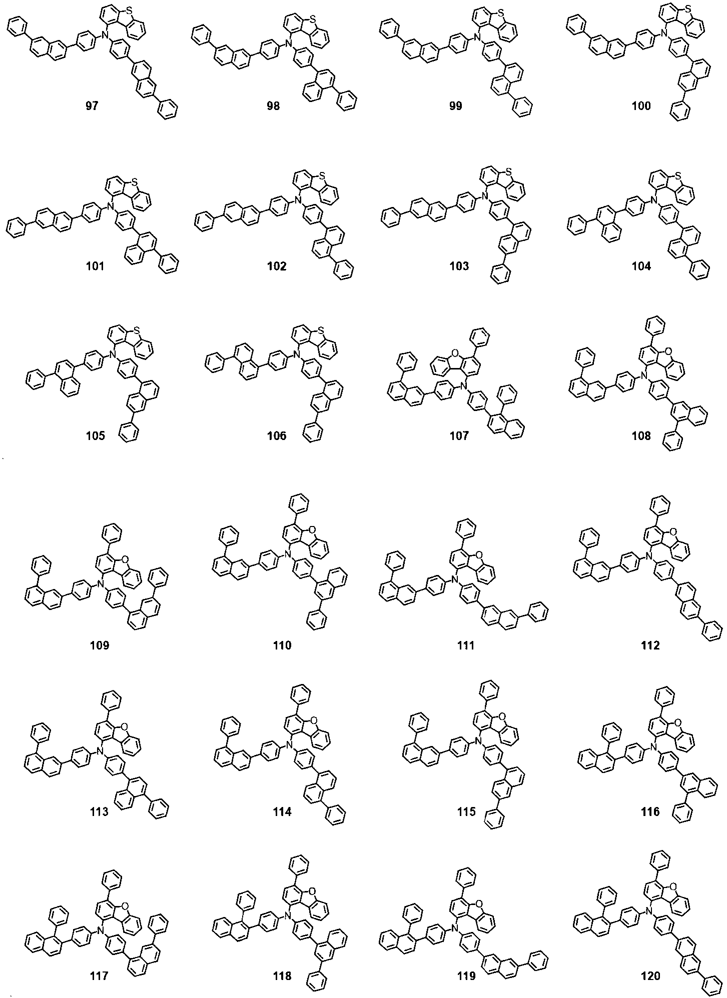 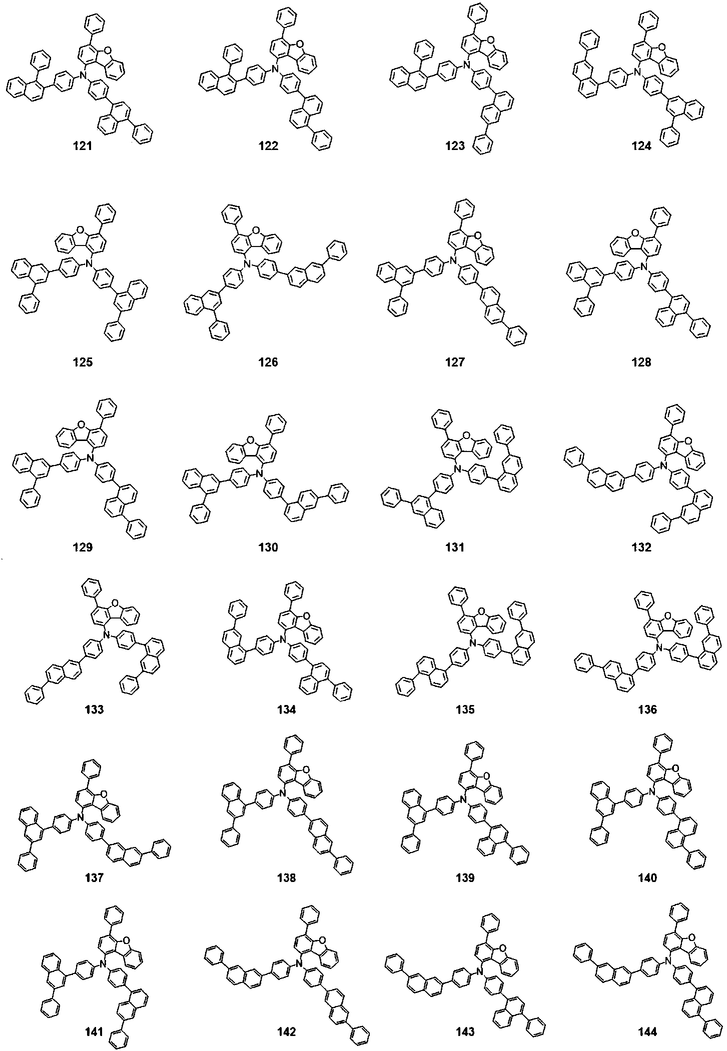 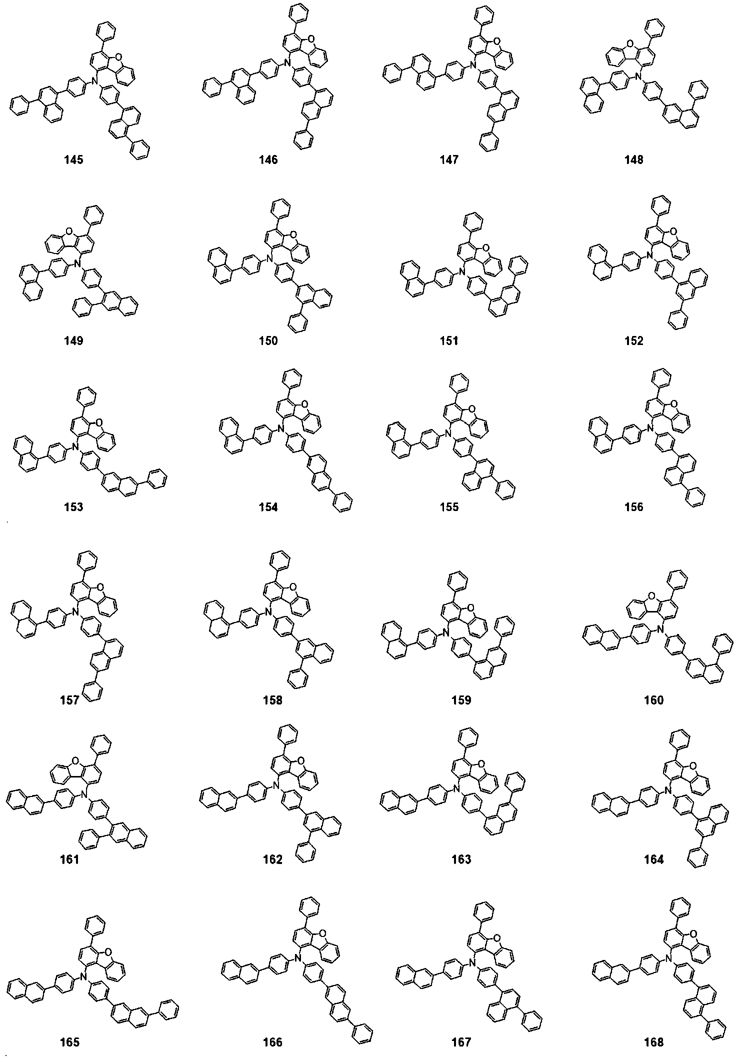 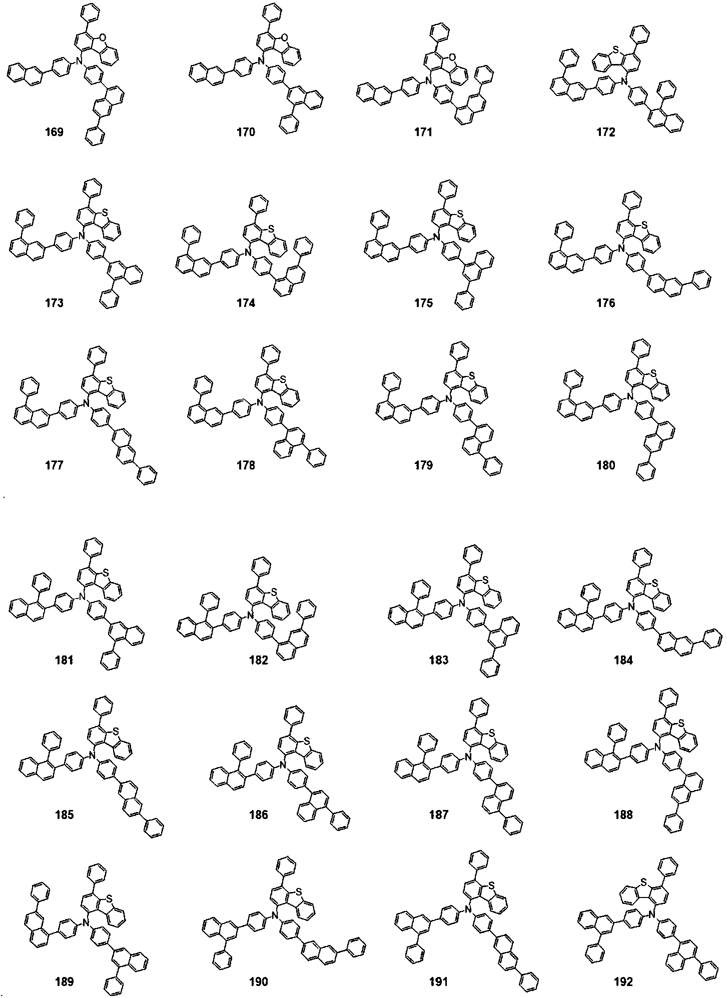 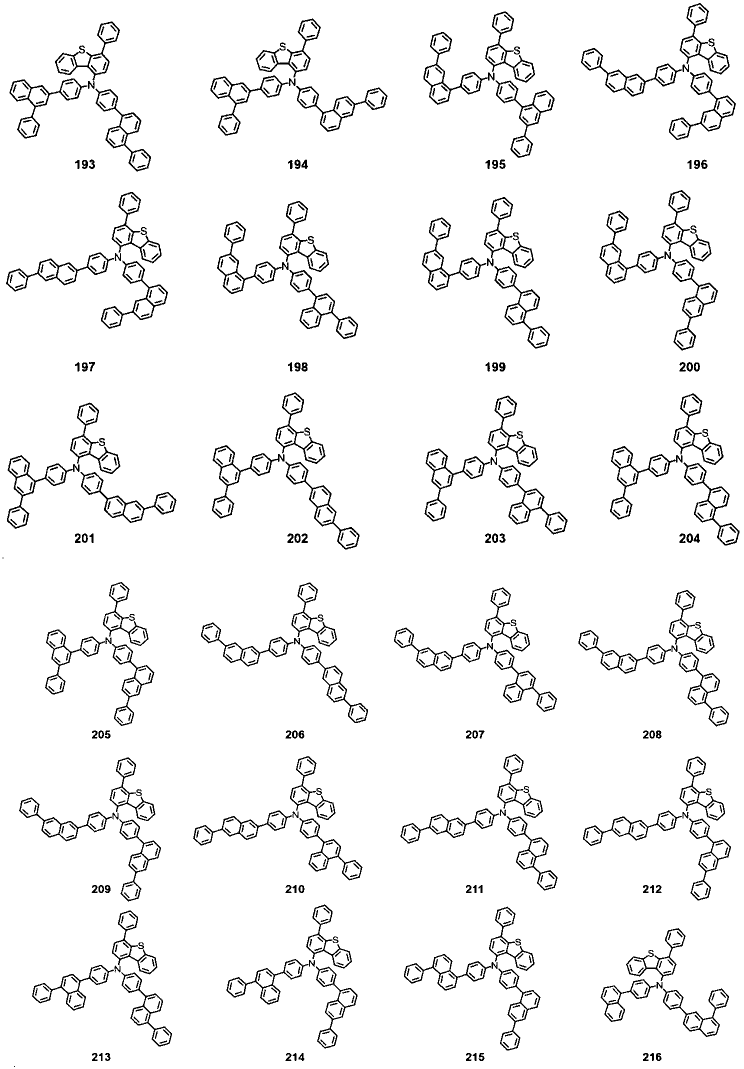 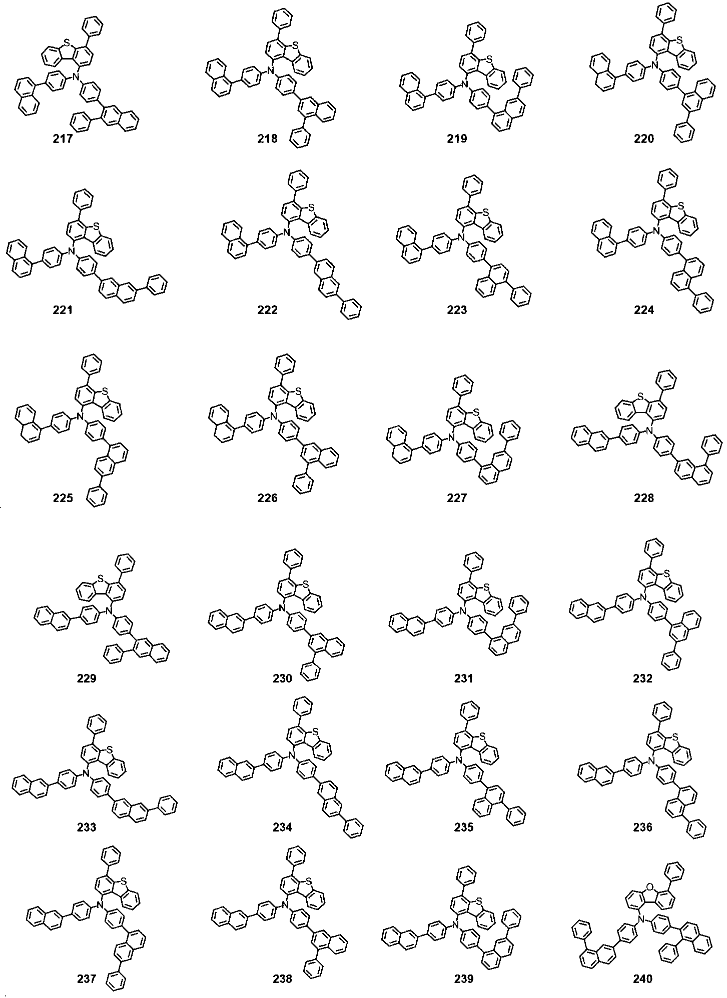 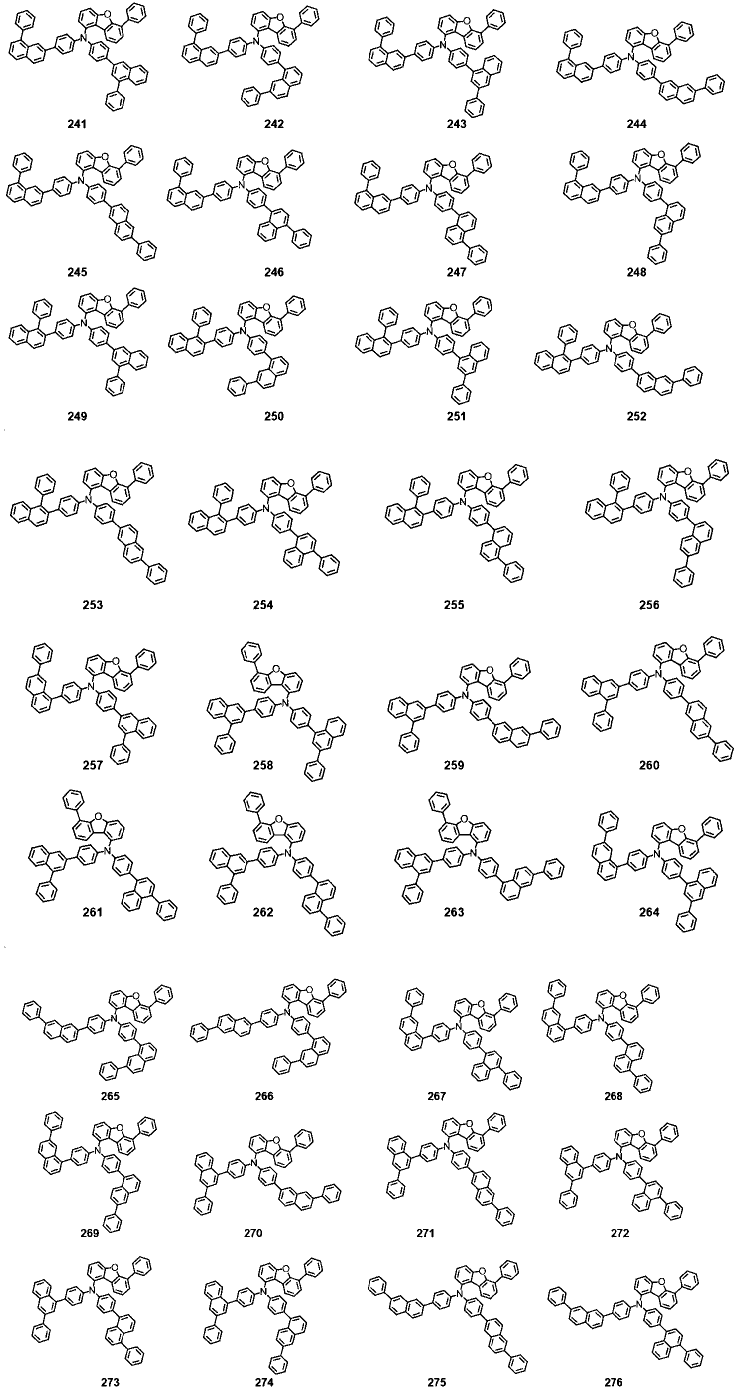 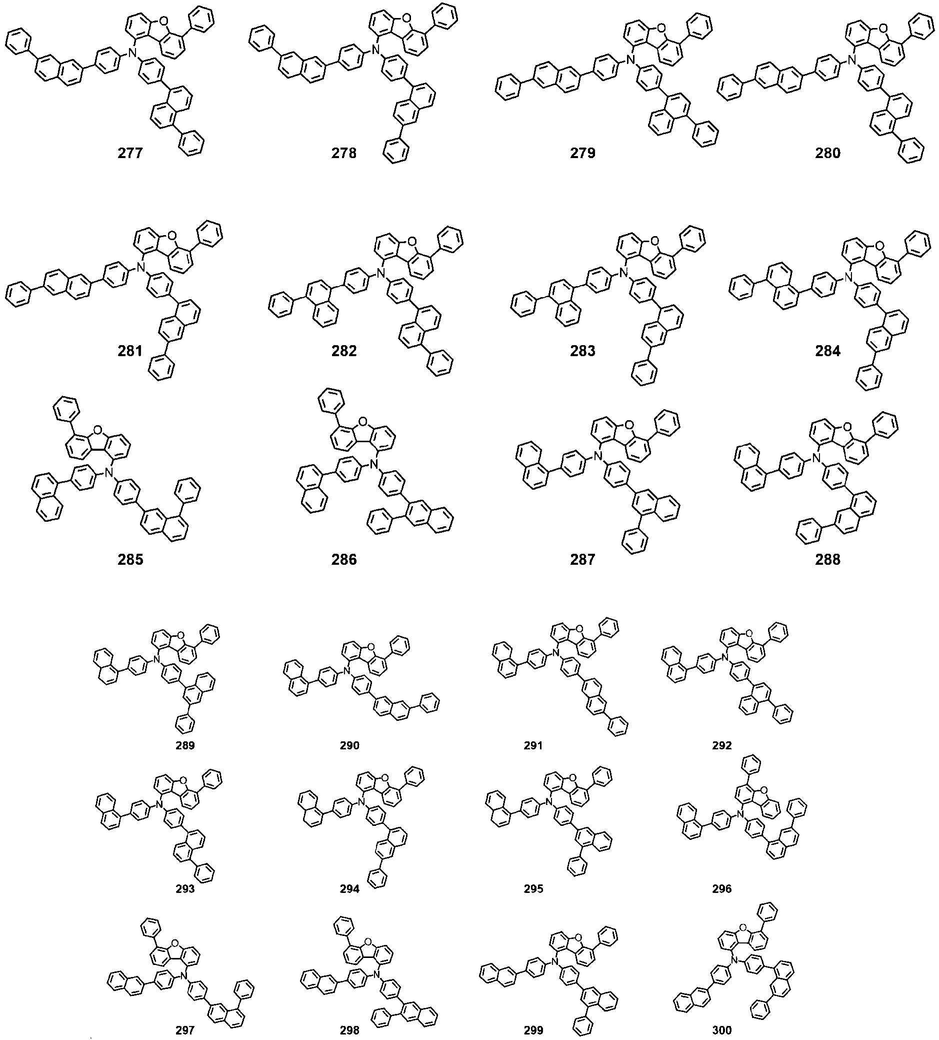 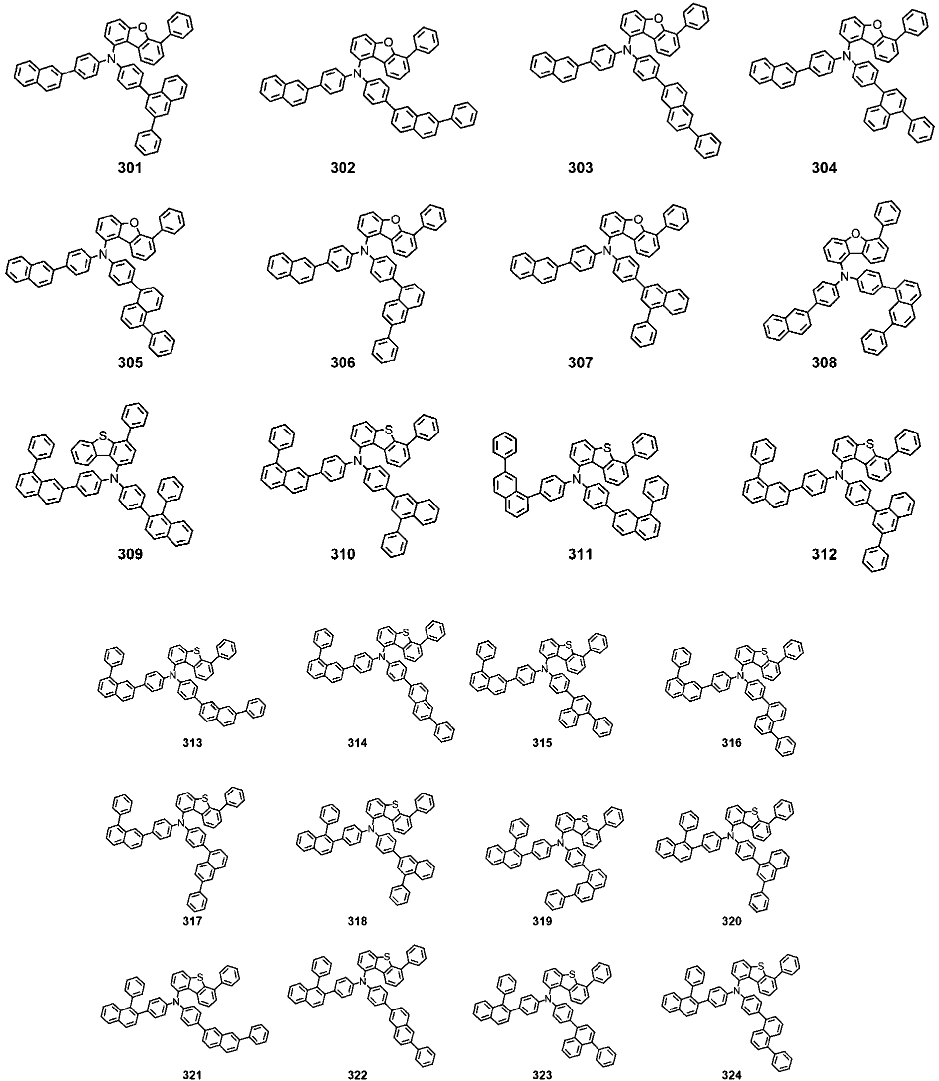 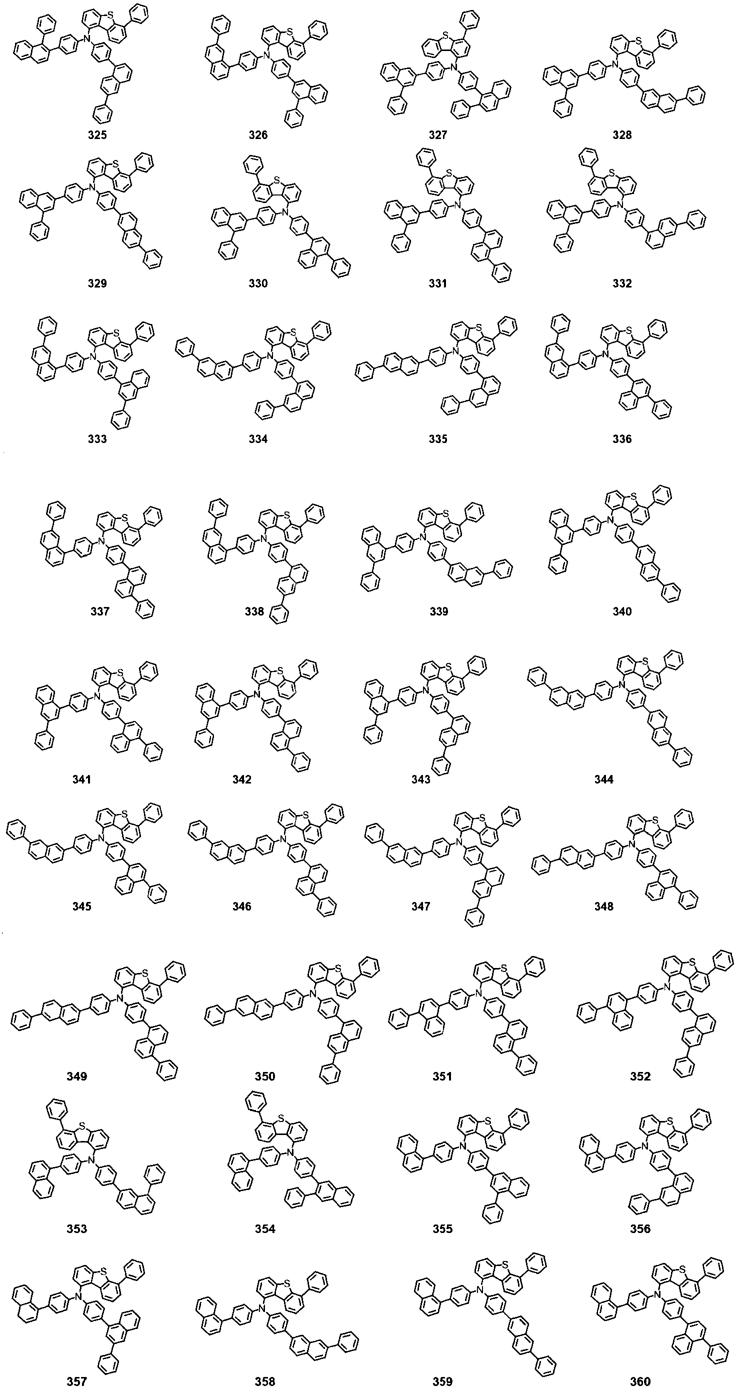 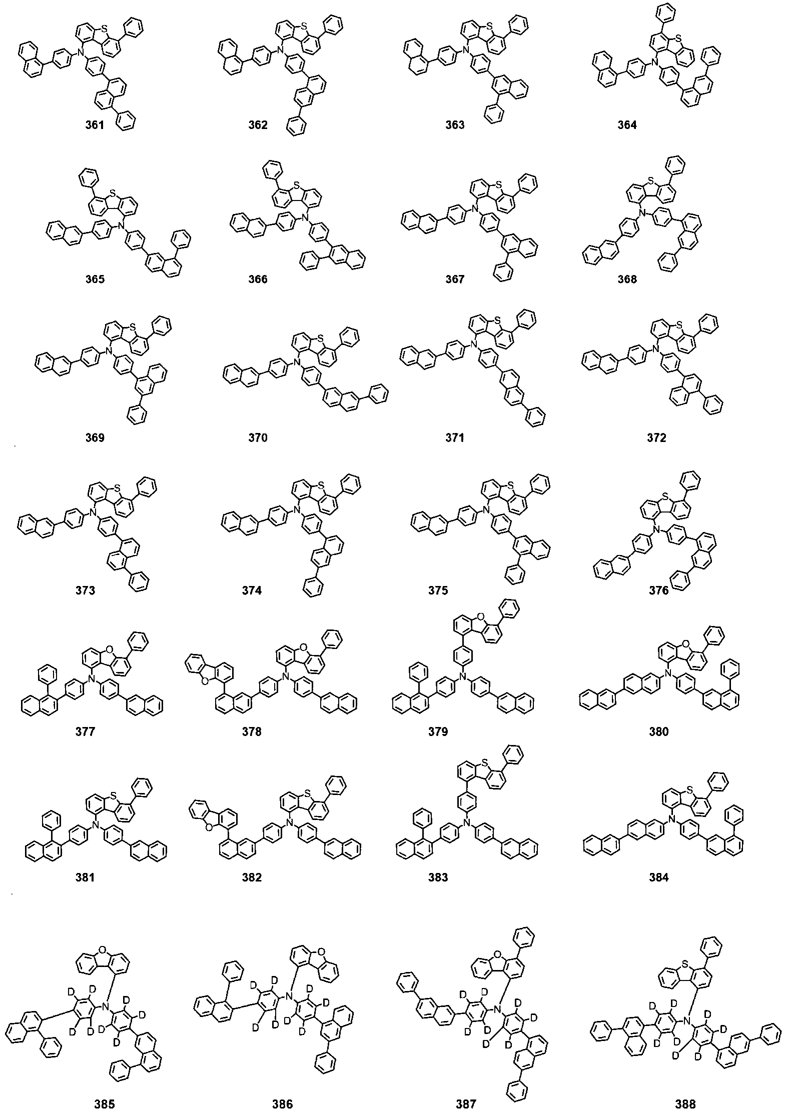 wherein in Compound Group 1 above, D is a deuterium atom.

7. A light emitting element (ED) comprising:
a first electrode (EL1);
a second electrode (EL2) on the first electrode (EL1);
an emission layer (EML) between the first electrode (EL1) and the second electrode (EL2); and
a hole transport region (HTR) between the first electrode (EL1) and the emission layer (EML), and comprising an amine compound according to any one of claims 1 to 6.

8. The light emitting element (ED) of claim 7, wherein the hole transport region (HTR) comprises at least one selected from a hole injection layer (HIL), a hole transport layer (HTL), an electron blocking layer (EBL), and an auxiliary emission layer (EAL), and
at least one selected from the hole injection layer (HIL), the hole transport layer (HTL), the electron blocking layer (EBL), and the auxiliary emission layer (EAL) comprises the amine compound.

9. The light emitting element (ED) of claim 7 or claim 8, wherein the hole transport region (HTR) comprises a hole injection layer (HIL) on the first electrode (EL1), and a hole transport layer (HTL) on the hole injection layer (HIL), and
the hole transport layer (HTL) comprises the amine compound.

10. The light emitting element (ED) of any one of claims 7 to 9, wherein the emission layer (EML) comprises a compound represented by Formula E-1 below: 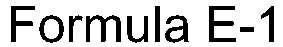 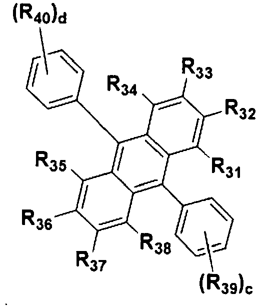 wherein in Formula E-1,
c and d are each independently an integer of 0 to 5, and
R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring.

11. An electronic device (EA) comprising a display module (DM) including a plurality of light emitting elements (ED),
wherein at least one selected from the plurality of light emitting elements (ED) includes a first electrode (EL1), a second electrode (EL2) on the first electrode (EL1), an emission layer (EML) between the first electrode (EL1) and the second electrode (EL2), and a hole transport region (HTR) between the first electrode (EL1) and the emission layer (EML1) and comprising an amine compound according to any one of claims 1 to 6.
